(19) Europäisches Patentamt
European Patent Office
Office européen des brevets

(11) EP 1 220 845 B1

(12) **EUROPÄISCHE PATENTSCHRIFT**

(45) Veröffentlichungstag und Bekanntmachung des Hinweises auf die Patenterteilung:
**13.08.2003 Patentblatt 2003/33**

(21) Anmeldenummer: **00969275.7**

(22) Anmeldetag: **21.09.2000**

(51) Int Cl.[7]: **C07D 235/16**, C07D 235/14, C07D 401/12, A61K 31/4184, A61K 31/4439, A61P 37/08, A61P 29/00

(86) Internationale Anmeldenummer:
**PCT/EP00/09236**

(87) Internationale Veröffentlichungsnummer:
**WO 01/023359 (05.04.2001 Gazette 2001/14)**

(54) **ARYLSULFONAMID-SUBSTITUIERTE BENZIMIDAZOLDERIVATE IHRE VERWENDUNG ALS TRYPTASE-INHIBITOREN**

ARYL-SULFONAMIDE SUBSTITUTED BENZIMIDAZOL DERIVATIVES AND USE OF SAID AS TRYPTASE INHIBITORS

DERIVES DE BENZIMIDAZOLE PORTANT COMME SUBSTITUANT UN ARYLSULFONAMIDE ET LEUR UTILISATION COMME INHIBITEURS DE LA TRYPTASE

(84) Benannte Vertragsstaaten:
**AT BE CH CY DE DK ES FI FR GB GR IE IT LI LU MC NL PT SE**

(30) Priorität: **24.09.1999 DE 19945810**

(43) Veröffentlichungstag der Anmeldung:
**10.07.2002 Patentblatt 2002/28**

(73) Patentinhaber: **Boehringer Ingelheim Pharma GmbH & Co.KG**
**55218 Ingelheim am Rhein (DE)**

(72) Erfinder:
- **ANDERSKEWITZ, Ralf**
  **55411 Bingen (DE)**
- **BRAUN, Christine**
  **CH-6512 Giubiasco (CH)**
- **BRIEM, Hans**
  **28279 Bremen (DE)**
- **DISSE, Bernd**
  **55127 Mainz (DE)**
- **HOENKE, Christoph**
  **55218 Ingelheim/Rhein (DE)**
- **JENNEWEIN, Hans-Michael**
  **65193 Wiesbaden (DE)**
- **SPECK, Georg**
  **55218 Ingelheim/Rhein (DE)**

(74) Vertreter: **Laudien, Dieter, Dr.**
**Boehringer Ingelheim GmbH,**
**CD Patents**
**55216 Ingelheim am Rhein (DE)**

(56) Entgegenhaltungen:
**WO-A-00/08014** **WO-A-94/27958**
**WO-A-98/37075** **WO-A-99/24407**

- **J STÜRZEBECHER ET AL: "Inhibition of human mast cell tryptase by benzamidine derivatives" BIOLOGICAL CHEMISTRY HOPPE-SEYLER, Bd. 373, Nr. 10, Oktober 1992 (1992-10), Seiten 1025-1030, XP002103558 ISSN: 0177-3593**
- **CAUGHEY G H ET AL: "Bis(5-amidino-2-benzimidazolyl)methane and related amidines are potent, reversible inhibitors of mast cell tryptases" JOURNAL OF PHARMACOLOGY AND EXPERIMENTAL THERAPEUTICS, Bd. 264, Nr. 2, 1993, Seiten 676-682, XP002064911 ISSN: 0022-3565**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 220 845 B1

## Beschreibung

Die Erfindung betrifft substituierte Benzimidazolderivate der allgemeinen Formel (I)

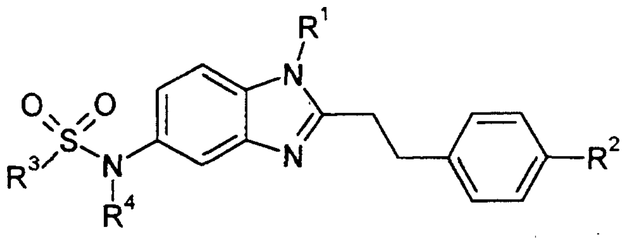

(I),

worin die Reste $R^1$, $R^2$, $R^3$ und $R^4$ die in den Ansprüchen und in der Beschreibung genannten Bedeutungen haben können, Verfahren zu deren Herstellung sowie die Verwendung von Arylsulfonamid-substituierten Benzimidazolderivaten zur Herstellung von Arzneimitteln, insbesondere von Arzneimitteln mit Tryptase-inhibierender Wirkung.

<u>Hintergrund der Erfindung</u>

Benzimidazolderivate sind als Wirkstoffe mit wertvollen pharmazeutischen Eigenschaften aus dem Stand der Technik bekannt. So offenbart die Internationale Patentanmeldung WO 98/37075 neben anderen bicyclischen Heterocyclen auch Benzimidazole, die sich aufgrund einer thrombinhemmenden Wirkung zur Vorbeugung und Behandlung venöser und arterieller thrombotischer Erkrankungen wirksam einsetzen lassen.

Anders als der vorstehend beschriebenen und im Stand der Technik bereits bekannten Verwendung von Benzimidazolderivaten, liegt der vorliegenden Erfindung die Aufgabe zugrunde, neue Tryptase-Inhibitoren bereitzustellen, die aufgrund ihrer Tryptase-inhibierenden Eigenschaften zur Vorbeugung und Behandlung entzündlicher und/oder allergischer Erkrankungen eingesetzt werden können.

<u>Detaillierte Beschreibung der Erfindung</u>

Überraschenderweise wurde gefunden, daß Arylsulfonamid-substituierte Benzimidazolderivate der allgemeinen Formel (I)

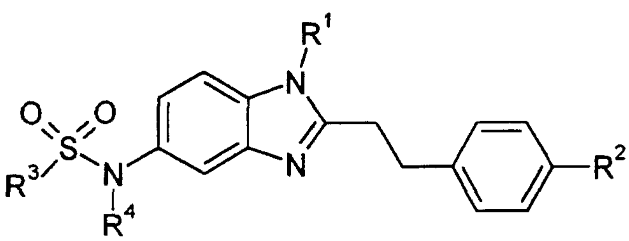

(I),

worin die Reste $R^1$, $R^2$, $R^3$ und $R^4$ die nachstehend genannten Bedeutungen tragen können, eine Tryptase-inhibierende Wirkung aufweisen und erfindungsgemäß zur Vorbeugung und Behandlung von Erkrankungen Verwendung finden können, in denen Tryptase-Inhibitoren einen therapeutischen Nutzen entfalten können.

Die vorliegende Erfindung betrifft folglich die neuen Benzimidazole der allgemeinen Formel (I)

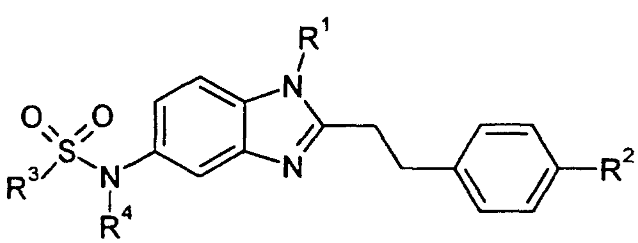

(I),

worin

$R^1$ ein Rest ausgewählt aus der Gruppe $C_1$-$C_6$-Alkyl, $C_2$-$C_6$-Alkenyl und $C_2$-$C_6$-Alkinyl, welcher gegebenenfalls ein-, zwei- oder dreifach durch einen oder mehrere der Reste Hydroxy, $C_1$-$C_4$-Alkoxy, $CF_3$, Phenoxy, COOH, Halogen, -CO($C_1$-$C_4$-alkoxy), -$CONH_2$, -CO-NH($C_1$-$C_4$-alkyl), -CO-N($C_1$-$C_4$-alkyl)$_2$, -$NH_2$, -NH($C_1$-$C_4$-alkyl), -N($C_1$-$C_4$-alkyl)$_2$ oder $C_1$-$C_4$-Alkoxy-phenoxy substituiert sein kann, oder
ein gegebenenfalls über eine $C_1$-$C_4$-Alkylenbrücke verknüpftes $C_3$-$C_8$-Cycloalkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
Phenyl-$C_1$-$C_4$-alkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls durch $C_1$-$C_4$-Alkyl oder Benzyl substituiert sein kann;

$R^2$ -C(=NH)$NH_2$ oder -$CH_2$-$NH_2$;

$R^3$ ein Rest ausgewählt aus der Gruppe Phenyl, Benzyl, Naphthyl, Furan, Chinolyl, Isochinolyl, Benzofuranyl, Thienyl und Benzothienyl der jeweils ein-, zwei- oder dreifach durch einen oder mehrere der Reste ausgewählt aus der Gruppe $C_1$-$C_4$-Alkyl, $C_1$-$C_4$-Alkoxy, Halogen, -$C_1$-$C_4$-alkyl-Halogen, -$NH_2$, -NH($C_1$-$C_4$-alkyl), -N($C_1$-$C_4$-alkyl)$_2$, $NO_2$, Hydroxyl -$CF_3$, -NHCO-$C_1$-$C_4$-alkyl, -COOH, -COO($C_1$-$C_4$-alkyl), -$CONH_2$, -CONH($C_1$-$C_4$-alkyl), -CON($C_1$-$C_4$-alkyl)$_2$,
-CONH($C_1$-$C_4$-alkyl)-COO($C_1$-$C_4$-alkyl) und Phenyl-$C_1$-$C_6$-alkyl substituiert ist, wobei der Substituent Phenyl-$C_1$-$C_6$-alkyl seinerseits substituiert sein kann durch einen Rest ausgewählt aus der Gruppe $C_1$-$C_6$-Alkoxy, Hydroxy, Halogen, $CF_3$ und $C_1$-$C_6$-Alkyl;

$R^4$ Wasserstoff, ein $C_1$-$C_6$-Alkyl-Rest, der ein- oder zweifach durch einen oder zwei der Reste -$NH_2$, -NH($C_1$-$C_4$-alkyl), -N($C_1$-$C_4$-alkyl)$_2$, -C(=NH)$NH_2$ oder -NH-C(=NH)$NH_2$ substituiert sein kann, oder
ein direkt oder über eine $C_1$-$C_4$-Alkylen-Brücke oder über eine $C_1$-$C_4$-Alkylen-CO-Brücke verknüpfter 5-, 6- oder 7-gliedriger, gesättigter oder ungesättigter Heterocyclus, der gegebenenfalls ein, zwei oder drei Heteroatome ausgewählt aus der Gruppe Sauerstoff, Stickstoff oder Schwefel enthalten kann und der gegebenenfalls durch $C_1$-$C_4$-Alkyl, Benzyl oder Pyridyl substituiert sein kann;
$C_3$-$C_8$-Cycloalkyl, das ein- oder zweifach durch einen oder zwei der Reste -$NH_2$, -NH($C_1$-$C_4$-alkyl), -N($C_1$-$C_4$-alkyl)$_2$, -C(=NH)$NH_2$ oder -NH-C(=NH)$NH_2$ substituiert sein kann, oder
Phenyl, Benzyl oder Phenylethyl, die am Phenylring jeweils ein- oder zweifach durch einen oder zwei der Reste -$NH_2$, -NH($C_1$-$C_4$-alkyl), -N($C_1$-$C_4$-alkyl)$_2$, -$C_1$-$C_4$-Alkyl-$NH_2$, -C(=NH)$NH_2$ oder -NH-C(=NH)$NH_2$ substituiert sein können,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Bevorzugt sind Verbindungen der allgemeinen Formel (I), worin
worin

$R^1$ $C_1$-$C_6$-Alkyl, welches gegebenenfalls ein-, zwei- oder dreifach durch einen oder mehrere der Reste Hydroxy, $C_1$-$C_4$-Alkoxy, $CF_3$, Phenoxy, COOH, Halogen, -CO($C_1$-$C_4$-alkoxy), -$CONH_2$, -CO-NH($C_1$-$C_4$-alkyl), -CO-N($C_1$-$C_4$-alkyl)$_2$, -$NH_2$, -NH($C_1$-$C_4$-alkyl), -N($C_1$-$C_4$-alkyl)$_2$ oder $C_1$-$C_4$-Alkoxy-phenoxy substituiert sein kann, oder
ein gegebenenfalls über eine $C_1$-$C_4$-Alkylenbrücke verknüpftes $C_3$-$C_8$-Cycloalkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
Phenyl-$C_1$-$C_4$-alkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls durch $C_1$-$C_4$-Alkyl oder Benzyl substituiert sein kann;

$R^2$ -C(=NH)$NH_2$ oder -$CH_2$-$NH_2$;

$R^3$ ein Rest ausgewählt aus der Gruppe Phenyl, Benzyl, Naphthyl, Chinolyl, Isochinolyl, Thienyl und Benzothienyl der jeweils ein-, zwei- oder dreifach durch einen oder mehrere der Reste ausgewählt aus der Gruppe $C_1$-$C_4$-Alkyl, $C_1$-$C_4$-Alkoxy, Halogen,
-$C_1$-$C_4$-alkyl-Halogen, -$NH_2$, -NH($C_1$-$C_4$-alkyl), -N($C_1$-$C_4$-alkyl)$_2$, $NO_2$, Hydroxy, -$CF_3$, -NHCO-$C_1$-$C_4$-alkyl, -COOH, -COO($C_1$-$C_4$-alkyl), -$CONH_2$, -CONH($C_1$-$C_4$-alkyl), -CON($C_1$-$C_4$-alkyl)$_2$,
-CONH($C_1$-$C_4$-alkyl)-COO($C_1$-$C_4$-alkyl) und Phenyl-$C_1$-$C_4$-alkyl substituiert ist, wobei der Substituent Phenyl-$C_1$-$C_4$-alkyl seinerseits substituiert sein kann durch einen Rest ausgewählt aus der Gruppe $C_1$-$C_4$-Alkoxy, Hydroxy, Halogen, $CF_3$ und $C_1$-$C_4$-Alkyl;

$R^4$ Wasserstoff, ein $C_1$-$C_6$-Alkyl-Rest, der ein- oder zweifach durch einen oder zwei der Reste -$NH_2$, -NH($C_1$-$C_4$-alkyl), -N($C_1$-$C_4$-alkyl)$_2$, -C(=NH)$NH_2$ oder -NH-C(=NH)$NH_2$ substituiert sein kann, oder
ein direkt oder über eine $C_1$-$C_4$-Alkylen-Brücke oder über eine $C_1$-$C_4$-Alkylen-CO-Brücke verknüpfter 5-, 6- oder 7-gliedriger, gesättigter oder ungesättigter Heterocyclus, der gegebenenfalls ein, zwei oder drei Heteroatome ausgewählt aus der Gruppe Sauerstoff, Stickstoff oder Schwefel enthalten kann und der gegebenenfalls durch $C_1$-$C_4$-Alkyl, Benzyl oder Pyridyl substituiert sein kann;
$C_3$-$C_8$-Cycloalkyl, das ein- oder zweifach durch einen oder zwei der Reste -$NH_2$, -NH($C_1$-$C_4$-alkyl), -N($C_1$-$C_4$-alkyl)$_2$, -C(=NH)$NH_2$ oder -NH-C(=NH)$NH_2$ substituiert sein kann, oder
Phenyl, Benzyl oder Phenylethyl, die am Phenylring jeweils ein- oder zweifach durch einen oder zwei der Reste -$NH_2$, -NH($C_1$-$C_4$-alkyl), -N($C_1$-$C_4$-alkyl)$_2$, -$C_1$-$C_4$-Alkyl-$NH_2$, -C(=NH)$NH_2$ oder -NH-C(=NH)$NH_2$ substituiert sein können,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß bevorzugt sind die Verbindungen der allgemeinen Formel (I), worin

$R^1$ $C_1$-$C_5$-Alkyl, welches gegebenenfalls ein-, zwei- oder dreifach durch einen oder mehrere der Reste Hydroxy, $C_1$-$C_4$-Alkoxy, $CF_3$, Phenoxy, COOH, -CO($C_1$-$C_4$-alkoxy), -$CONH_2$, -CO-NH($C_1$-$C_4$-alkyl), -CO-N($C_1$-$C_4$-alkyl)$_2$ oder $C_1$-$C_4$-Alkoxy-phenoxy substituiert sein kann, oder
ein gegebenenfalls über eine $C_1$-$C_4$-Alkylenbrücke verknüpftes $C_3$-$C_8$-Cycloalkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
Phenyl-$C_1$-$C_4$-alkyl, welches gegebenenfalls ein-, zwei- oder dreifach durch einen oder mehrere der Reste Hydroxy, $C_1$-$C_4$-Alkoxy, Carboxy, $C_1$-$C_4$-Alkoxycarbonyl oder $CF_3$ substituiert sein kann, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls durch $C_1$-$C_4$-Alkyl oder Benzyl substituiert sein kann;

$R^2$ -C(=NH)$NH_2$ oder -$CH_2$-$NH_2$;

$R^3$ ein Rest ausgewählt aus der Gruppe Phenyl, Benzyl, Benzothienyl, Chinolyl, Isochinolyl und Naphthyl, der jeweils ein-, zwei- oder dreifach durch einen oder mehrere der Reste ausgewählt aus der Gruppe $C_1$-$C_4$-Alkyl, $C_1$-$C_4$-Alkoxy, Halogen, $CF_3$, -$C_1$-$C_4$-alkyl-Halogen, -$NH_2$, -NH($C_1$-$C_4$-alkyl), -N($C_1$-$C_4$-alkyl)$_2$, $NO_2$, Hydroxy, -$CF_3$, -NHCO-$C_1$-$C_4$-alkyl, -COOH, -COO($C_1$-$C_4$-alkyl), -$CONH_2$, -CONH($C_1$-$C_4$-alkyl), -CON($C_1$-$C_4$-alkyl)$_2$,
-C($CH_3$)$_2$-Phenyl, wobei diese Phenylgruppe durch eine oder mehrere Halogenatome oder Hydroxygruppen substituiert sein kann, und -CONH($C_1$-$C_4$-alkyl)-COO($C_1$-$C_4$-alkyl) substituiert ist;

$R^4$ ein $C_1$-$C_6$-Alkyl-Rest, der ein- oder zweifach durch einen oder zwei der Reste -$NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert ist, oder
ein direkt oder über eine $C_1$-$C_4$-Alkylen-Brücke oder über eine $C_1$-$C_4$-Alkylen-CO-Brücke verknüpfter 5-, 6- oder 7-gliedriger, gesättigter oder ungesättigter Stickstoff-Heterocyclus, der gegebenenfalls ein oder zwei weitere Heteroatome ausgewählt aus der Gruppe Sauerstoff, Stickstoff oder Schwefel enthalten kann und der gegebenenfalls durch $C_1$-$C_4$-Alkyl, Benzyl oder Pyridyl substituiert sein kann;
Cyclopropyl, Cyclopentyl oder Cyclohexyl, die jeweils ein- oder zweifach durch einen oder zwei der Reste -$NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind, oder
unsubstituiertes Benzyl oder Phenyl, Benzyl oder Phenylethyl, die am Phenylring jeweils ein- oder zweifach

durch einen oder zwei der Reste $-NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Bevorzugt sind substituierte Benzimidazol-Derivate der allgemeinen Formel (I), worin

$R^1$ Methyl, Ethyl, Propyl, Butyl oder Pentyl, die jeweils durch einen der Reste Hydroxy, Methoxy, Ethoxy, Propoxy, $CF_3$, Phenoxy, COOH, $CONH_2$, CONHMe oder Methoxy-phenoxy substituiert sein können, oder
Benzyl, Phenylethyl oder Phenylpropyl, die am Phenylring jeweils ein- oder zweifach durch einen oder zwei der Reste Hydroxy, Methoxy, Ethoxy, Carboxy, Methoxycarbonyl, Ethoxycarbonyl oder $CF_3$ substituiert sein können, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff oder Stickstoff enthalten kann und der gegebenenfalls durch Methyl, Ethyl, Propyl oder Benzyl substituiert sein kann;

$R^2$ -C(=NH)$NH_2$ oder $-CH_2$-$NH_2$;

$R^3$ Phenyl, Benzyl oder Naphthyl, die jeweils ein- oder zweifach durch einen oder zwei Reste ausgewählt aus der Gruppe Methyl, Ethyl, Propyl, Butyl, Methoxy, Ethoxy, Propoxy, Butoxy, Fluor, Chlor, Brom, Iod, $-CF_3$, $-NH_2$, -NHMethyl, -NHEthyl, -NMethyl$_2$, -NEthyl$_2$, $NO_2$, -NHCO-Methyl, -NHCO-Ethyl, -COOH, -COOMethyl, -COOEthyl, $-CONH_2$, -CONHMethyl, -CONHEthyl, -CONMethyl$_2$, -CONEthyl$_2$, -CONH-$CH_2$-COOH, -CONH-$CH_2$-COO-Methyl, -CONH-$CH_2$-COOEthyl, -CONH-$CH_2CH_2$-COOH, -CONH-$CH_2CH_2$-COOMethyl, -C($CH_3$)$_2$-Phenyl, wobei diese Phenylgruppe durch eine oder mehrere Halogenatome oder Hydroxygruppen substituiert sein kann, und -CONH-$CH_2CH_2$-COOEthyl substituiert sind;

$R^4$ ein Rest ausgewählt aus der Gruppe Ethyl, Propyl, Butyl, Pentyl und Hexyl, der jeweils ein- oder zweifach durch einen oder zwei der Reste $-NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind, oder
ein direkt oder über eine $C_1$-$C_4$-Alkylen-Brücke verknüpfter 5-, 6- oder 7-gliedriger, gesättigter oder ungesättigter Stickstoff-Heterocyclus, der gegebenenfalls ein oder zwei weitere Heteroatome ausgewählt aus der

Gruppe Sauerstoff oder Stickstoff enthalten kann und der gegebenenfalls durch Methyl, Ethyl, Propyl oder Benzyl substituiert sein kann, oder
Cyclopentyl oder Cyclohexyl, die jeweils ein- oder zweifach durch einen oder zwei der Reste $-NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind, oder
unsubstituiertes Benzyl oder Benzyl oder Phenylethyl, die am Phenylring jeweils ein- oder zweifach durch einen oder zwei der Reste $-NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind substituierte Benzimidazol-Derivate der allgemeinen Formel (I), worin

$R^1$ Methyl, Ethyl, Propyl, Butyl oder Pentyl, bevorzugt Methyl;

$R^2$ -C(=NH)$NH_2$ oder $-CH_2$-$NH_2$, bevorzugt -C(=NH)$NH_2$;

$R^3$ Phenyl oder Naphthyl, die jeweils durch einen oder zwei Reste ausgewählt aus der Gruppe Methyl, Ethyl, *tert*.-Butyl, Methoxy, Ethoxy, Fluor, Chlor, Brom, $CF_3$, $-NH_2$, -NHMethyl, -NHEthyl, -NMethyl$_2$, -NEthyl$_2$, $NO_2$, -NHCO-Methyl, -NHCO-Ethyl, -COOH, -COOMethyl, -COOEthyl, $-CONH_2$, -CONHMethyl, -CONHEthyl, -CONMethyl$_2$, -CONEthyl$_2$, -CONH-$CH_2$-COOH, -CONH-$CH_2$-COOMethyl, -C($CH_3$)$_2$-Phenyl, wobei diese Phenylgruppe durch eine oder mehrere Halogenatome oder Hydroxygruppen substituiert sein kann, und -CONH-$CH_2$-COOEthyl,
substituiert sind;

$R^4$ Ethyl oder Propyl, die jeweils durch einen der Reste $-NH_2$, -N(Methyl)$_2$, -N(Ethyl)$_2$, -N(n-Propyl)$_2$ oder -C(=NH)

$NH_2$ substituiert sind, oder
ein direkt oder über eine Methylen- oder Ethylen-Brücke verknüpfter 5-, 6- oder 7-gliedriger, gesättigter oder ungesättigter Stickstoff-Heterocyclus, der ein Sauerstoff als weiteres Heteroatom enthalten kann und der gegebenenfalls durch Methyl, Ethyl, Propyl oder Benzyl substituiert sein kann, oder
Cyclopentyl oder Cyclohexyl, die jeweils durch einen der Reste $-NH_2$, $-N(Methyl)_2$, $-N(Ethyl)_2$, $-N(n\text{-}Propyl)_2$ oder $-C(=NH)NH_2$ substituiert sind, oder
Benzyl, das am Phenylring durch einen der Reste $-NH_2$, $-N(Methyl)_2$, $-N(Ethyl)_2$, $-N(n\text{-}Propyl)_2$ oder $-C(=NH)NH_2$ substituiert sein kann, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind ferner substituierte Benzimidazol-Derivate der allgemeinen Formel (I), worin

$R^1$ Methyl, Ethyl, Propyl, Butyl oder Pentyl, bevorzugt Methyl;

$R^2$ $-C(=NH)NH_2$ oder $-CH_2\text{-}NH_2$, bevorzugt $-C(=NH)NH_2$;

$R^3$ Phenyl das durch einen oder zwei Reste ausgewählt aus der Gruppe Methyl, Ethyl, *tert*-Butyl, Methoxy, Ethoxy, Fluor, Chlor, Brom, $CF_3$, und/oder einen Rest ausgewählt aus $-NH_2$, -NHMethyl, -NHEthyl, $-NMethyl_2$, $-NEthyl_2$, $NO_2$, -NHCO-Methyl, -NHCO-Ethyl, -COOH, -COOMethyl, -COOEthyl, $-CONH_2$, -CONHMethyl, -CONHEthyl, $-CONMethyl_2$, $-CONEthyl_2$, $-CONH\text{-}CH_2\text{-}COOH$, $-CONH\text{-}CH_2\text{-}COOMethyl$, $-C(CH_3)_2\text{-}Phenyl$, wobei diese Phenylgruppe durch eine oder mehrere Halogenatome oder Hydroxygruppen substituiert sein kann, und $-CONH\text{-}CH_2\text{-}COOEthyl$, substituiert ist, oder
Naphthyl, das durch $-NMethyl_2$ oder Chlor substituiert ist;

$R^4$ Benzyl, Pyridylmethyl, Dimethylaminoethyl, Diethylaminoethyl, Dimethylaminopropyl oder Diethylaminopropyl bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß von besonderer Bedeutung sind substituierte Benzimidazol-Derivate der allgemeinen Formel (I), worin

$R^1$ Methyl, Ethyl oder Propyl, bevorzugt Methyl;

$R^2$ $-C(=NH)NH_2$ oder $-CH_2\text{-}NH_2$, bevorzugt $-C(=NH)NH_2$;

$R^3$ Phenyl das durch einen oder zwei Reste ausgewählt aus der Gruppe Methyl, Ethyl, *tert*-Butyl, Methoxy, Ethoxy, Fluor, Chlor, Brom, $CF_3$, und/oder einen Rest aus der Gruppe $-NH_2$, -NHMethyl, -NHEthyl, $-NMethyl_2$, $-NEthyl_2$, $NO_2$, -NHCO-Methyl, -NHCO-Ethyl, -COOH, -COOMethyl, -COOEthyl, $-CONH_2$, -CONHMethyl, -CONHEthyl, $-CONMethyl_2$, $-CONEthyl_2$, $-CONH\text{-}CH_2\text{-}COOH$, $-CONH\text{-}CH_2\text{-}COOMethyl$, $-C(CH_3)_2\text{-}Phenyl$, wobei diese Phenylgruppe durch eine oder mehrere Halogenatome oder Hydroxygruppen substituiert sein kann, und $-CONH\text{-}CH_2\text{-}COOEthyl$,
substituiert ist, oder
Naphthyl, das durch $-NMethyl_2$ oder Chlor substituiert ist;

$R^4$ Dimethylaminoethyl oder Diethylaminoethyl bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß von besonderer Bedeutung sind ferner substituierte Benzimidazol-Derivate der allgemeinen Formel (I), worin

$R^1$ Methyl;

$R^2$ $-C(=NH)NH_2$ oder $-CH_2\text{-}NH_2$, bevorzugt $-C(=NH)NH_2$;

$R^3$ Phenyl das durch einen oder zwei Reste ausgewählt aus der Gruppe Methyl, *tert*-Butyl, Methoxy, Chlor, Brom,

$CF_3$, und/oder einen Rest aus der Gruppe $-NH_2$, $NO_2$, -NHCO-Methyl, -COOEthyl, -CONH-$CH_2$-COOH, -C$(CH_3)_2$-Phenyl, wobei diese Phenylgruppe durch eine oder mehrere Halogenatome oder Hydroxygruppen substituiert sein kann,
und -CONH-$CH_2$-COOEthyl, substituiert ist, oder
Naphthyl, das durch -$NMethyl_2$ oder Chlor substituiert ist;

$R^4$ Benzyl oder Diethylaminoethyl,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß von besonderem Interesse sind die folgenden Verbindungen:

- N-{2-[2-(4-Amidinophenyl)-ethyl]-1-methyl-benzimidazol-5-yl}-N-diethylaminoethyl-(4-ethoxycarbonyl-benzolsulfonamid)-hydrochlorid
- N-{2-[2-(4-Amidinophenyl)-ethyl]-1-methyl-benzimidazol-5-yl}-N-diethylaminoethyl-(dansylamid)-dihydrochlorid
- N-{2-[2-(4-Amidinophenyl)-ethyl]-1-methyl-benzimidazol-5-yl}-N-diethylaminoethyl-(3-brom-benzolsulfonamid)-hydrochlorid
- N-{2-[2-(4-Amidinophenyl)-ethyl]-1-methyl-benzimidazol-5-yl}-N-diethylaminoethyl-(4-nitro-benzolsulfonamid)-hydrochlorid
- N-{2-[2-(4-Amidinophenyl)-ethyl]-1-methyl-benzimidazol-5-yl}-N-diethylaminoethyl-(3-methoxy-benzolsulfonamid)-hydrochlorid
- N-{2-[2-(4-Amidinophenyl)-ethyl]-1-methyl-benzimidazol-5-yl}-N-diethylaminoethyl-(3-methyl-benzolsulfonamid)-hydrochlorid
- N-{2-[2-(4-Amidinophenyl)-ethyl]-1-methyl-benzimidazol-5-yl}-N-diethylaminoethyl-(3-chlor-benzolsulfonamid)-hydrochlorid
- N-{2-[2-(4-Amidinophenyl)-ethyl]-1-methyl-benzimidazol-5-yl}-N-diethylaminoethyl-(4-amino-benzolsulfonamid)-tetrahydrochlorid
- N-{2-[2-(4-Amidinophenyl)-ethyl]-1-methyl-benzimidazol-5-yl}-N-diethylaminoethyl-(4-acetylamino-benzolsulfonamid)
- N-{2-[2-(4-Amidinophenyl)-ethyl]-1-methyl-benzimidazol-5-yl}-N-diethylaminoethyl-(4-chlor-benzolsulfonamid)
- N-{2-[2-(4-Amidinophenyl)-ethyl]-1-methyl-benzimidazol-5-yl}-N-diethylaminoethyl-[3,5-di-(trilluormethyl)-benzolsulfonamid] dihydrochlorid
- N-{2-[2-(4-Amidinophenyl)-ethyl]-1-methyl-benzimidazol-5-yl}-N-diethylaminoethyl-(6-hydroxy-naphth-2-ylsulfonamid) dihydrochlorid
- N-{2-[2-(4-Amidinophenyl)-ethyl]-1-methyl-benzimidazol-5-yl}-N-diethylaminoethyl-(3-trifluormethyl-benzolsulfonamid) dihydrochlorid
- N-{2-[2-(4-Amidinophenyl)-ethyl]-1-methyl-benzimidazol-5-yl}-N-diethylaminoethyl-[3-(1-methyl-1-phenyl-ethyl)-benzolsulfonamid] dihydrochlorid
- N-{2-[2-(4-Amidinophenyl)-ethyl]-1-methyl-benzimidazol-5-yl}-N-aminoethyl(dansylamid) dihydrochlorid
- N-{2-[2-{4-Amidinophenyl)-ethyl]-1-methyl-benzimidazol-5-yl}-N-diethylaminoethyl-(4-*tert*-butyl-benzolsulfonamid) dihydrochlorid
- N-{2-[2-(4-Amidinophenyl)-ethyl]-1-methyl-benzimidazol-5-yl}-N-pyrid-3-ylmethyl-(dansylamid) dihydrochlorid
- N-{2-[2-(4-Amidinophenyl)-ethyl]-1-methyl-benzimidazol-5-yl}-N-diethylaminoethyl-(8-chlor-naphth-1-ylsulfonamid) dihydrochlorid
- N-{2-[2-(4-Amidinophenyl)-ethyl]-1-methyl-benzimidazol-5-yl}-N-pyrid-4-ylmethyl-(dansylamid) dihydrochlorid
- N-{2-[2-(4-Amidinophenyl)-ethyl]-1-methyl-benzimidazol-5-yl}-N-benzyl-(4-*tert*butyl-benzolsulfonamid) dihydrochlorid
- N-{2-[2-(4-Amidinophenyl)-ethyl]-1-methyl-benzimidazol-5-yl}-N-diethylaminoethyl-(3-nitro-benzolsulfonamid) dihydrochlorid
- N-{2-[2-(4-Amidinophenyl)-ethyl]-1-methyl-benzimidazol-5-yl}-N-diethylaminoethyl-(3-amino-benzolsulfonamid) dihydrochlorid

Neben den vorstehend genannten Verbindungen der allgemeinen Formel (I) zielt die vorliegende Erfindung ferner auf Verbindungen, die aufgrund einer in vivo abspaltbaren Funktionalität erst nach ihrer Einnahme durch den Patienten vom Organismus in die therapeutisch wirksamen Verbindungen der allgemeinen Formel (I) überführt werden. Solche Verbindungen werden als Prodrugs bezeichnet. Ein weiterer Aspekt der vorliegenden Erfindung zielt dementsprechend auf Prodrugs der allgemeinen Formel (II)

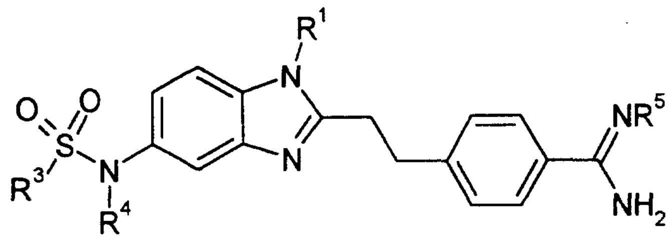

(II),

worin

R[1] und R[3] die vorstehend genannten Bedeutungen aufweisen können und

$R^4$ die vorstehend genannten Bedeutungen aufweisen kann oder $C_1$-$C_4$-Alkyl bedeutet, welches durch einen Rest ausgewählt aus der Gruppe -C(=NOH)$NH_2$, -C(=NCOO-$C_1$-$C_{12}$-alkyl)$NH_2$ oder -C(=NCOO-$C_1$-$C_8$-alkyl-Phenyl)$NH_2$ substituiert ist;

$R^5$ Hydroxy, -COO-$C_1$-$C_{12}$-Alkyl, -CO-Phenyl, -CO-Pyridyl oder -COO-$C_1$-$C_8$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Bevorzugt sind Prodrugs der allgemeinen Formel (II), worin

$R^1$ und $R^3$ die vorstehend genannten Bedeutungen aufweisen können und

$R^4$ die vorstehend genannten Bedeutungen aufweisen kann oder $C_1$-$C_4$-Alkyl bedeutet, welches durch einen Rest ausgewählt aus der Gruppe -C(=NOH)$NH_2$, -C(=NCOO-$C_1$-$C_6$-alkyl)$NH_2$ oder -C(=NCOO-$C_1$-$C_6$-alkyl-Phenyl)$NH_2$ substituiert ist;

$R^5$ Hydroxy, -COO-$C_1$-$C_6$-Alkyl, -CO-Phenyl, -CO-Pyridyl oder -COO-$C_1$-$C_6$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann, bedeuten kann, gegebenenfalls in Form ihrer Tautomeren, ihrer Racemate, ihrer Enantiomere, ihrer Diastereomere und ihre Gemische, sowie gegebenenfalls ihrer pharmakologisch unbedenklichen Säureadditionssalze.

Besonders bevorzugt sind Prodrugs der allgemeinen Formel (II), worin

$R^1$, $R^3$ und $R^4$ die vorstehend genannten Bedeutungen aufweisen können und
$R^5$ Hydroxy, Methoxycarbonyl, Ethoxycarbonyl, Propyloxycarbonyl, Butyloxycarbonyl, Benzoyl, Benzyloxycarbonyl oder Nicotinoyl bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Als Alkylgruppen (auch soweit sie Bestandteil anderer Reste sind) werden, falls nicht anders definiert, verzweigte und unverzweigte Alkylgruppen mit 1 bis 12 Kohlenstoffatomen, bevorzugt 1 bis 8 Kohelnstoffatomen, besonders bevorzugt mit 1 bis 6 Kohelnstoffatomen betrachtet. Beispielsweise werden genannt: Methyl, Ethyl, Propyl, Butyl, Pentyl, Hexyl etc. Sofern nicht anders genannt, sind von den vorstehend genannten Bezeichnungen Propyl, Butyl, Pentyl oder Hexyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc. Gegebenfalls werden zur Bezeichnung der vorstehend genannten Alkylreste auch gängige Abkürzungen wie Me für Methyl, Et für Ethyl etc. verwendet.

Als Alkenylgruppen (auch soweit sie Bestandteil anderer Reste sind) werden verzweigte und unverzweigte Alkenylgruppen mit 2 bis 6 Kohlenstoffatomen, bevorzugt 2 bis 4 Kohlenstoffatomen genannt, soweit sie mindestens eine Doppelbindung aufweisen, beispielsweise auch oben genannte Alkylgruppen bezeichnet, soweit sie mindestens eine Doppelbindung aufweisen, wie zum Beispiel Vinyl (soweit keine unbeständigen Enamine oder Enolether gebildet

werden), Propenyl, iso-Propenyl, Butenyl, Pentenyl, Hexenyl.

Als Alkinylgruppen (auch soweit sie Bestandteil anderer Reste sind) werden Alkinylgrupppen mit 2 bis 6 Kohlenstoffatomen bezeichnet, soweit sie mindestens eine Dreifachbindung aufweisen, beispielsweise Ethinyl, Propargyl, Butinyl, Pentinyl, Hexinyl.

Als Halogen wird im allgemeinen Fluor, Chlor, Brom oder Jod bezeichnet.

Als $C_3$-$C_8$-Cycloalkylgruppen werden erfindungsgemäß verstanden Reste ausgewählt aus der Gruppe Cyclopropyl, Cyclobutyl, Cyclopentyl, Cyclohxyl, Cycloheptyl und Cyclooctyl.

Als 5- ,6- oder 7-gliedrige, gesättigte oder ungesättigte Heterocyclen, die als Heteroatome Stickstoff, Sauerstoff oder Schwefel enthalten können, werden, soweit in den Definitionen nicht anders beschrieben beispielsweise Furan, Tetrahydrofuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Pyrazolidin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Diazepan, Oxazol, Isoxazol, Oxazin, Thiazol, Isothiazol, Thiadiazol, Oxadiazol, Pyrazolidin genannt, wobei der Heterocyclus wie in den Definitionen angegeben substituiert sein kann.

Sofern nicht anders genannt können in den Dialkylaminosubstituenten $-N(C_1-C_4-alkyl)_2$ die beiden Alkylgruppen gleich oder verschieden sein.

"=O" bedeutet ein über eine Doppelbindung verknüpftes Sauerstoffatom.

Wie eingangs erwähnt, weisen die erfindungsgemäßen Verbindungen der allgemeinen Formel (I) eine Wirksamkeit als Tryptase-Inhibitoren auf. Ein weiterer Aspekt der Erfindung zielt folglich auf die Verwendung der vorstehend definierten Verbindungen der allgemeinen Formel (I) zur Herstellung eines Arzneimittels zur Vorbeugung und/oder Behandlung von Krankheiten, in denen Tryptase-Inhibitoren einen therapeutischen Nutzen entfalten können,

Erfindungsgemäß bevorzugt ist die vorstehend genannte Verwendung von Verbindungen der allgemeinen Formel (I) zur Herstellung eines Arzneimittels zur Vorbeugung und/oder Behandlung entzündlicher und/oder allergischer Erkrankungen. Besonders bevorzugt ist die eingangs genannte Verwendung der Verbindung der allgemeinen Formel (I) zur Herstellung eines Arzneimittels zur Vorbeugung und/oder Behandlung von Asthma bronchiale, allergischer Rhinitis, allergischer Conjunctivitis, atopischer Dermatitis, Urticaria, allergischer Otitis, allergischer Magen-Darmerkrankungen, Morbus Crohn, Colitis ulcerosa, anaphylaktischer Schock, septischer Schock, Schocklunge (ARDS) und Arthritis. Ferner ist von Interesse die eingangs genannte Verwendung der Verbindungen der allgemeinen Formel (I) zur Herstellung eines Arzneimittels zur Vorbeugung und/oder Behandlung von Fibrosen wie Lungenfibrose, fibrosierende Alveolitis und Narbenbildung, von Kollagenosen wie Lupus erythematodes und Sklerodermie sowie von Arteriosklerose, Psoriasis und Neoplasien.

Die Synthese der substituierten Benzimidazol-Derivate der Formel (I) sowie die der Prodrugs der allgemeinen Formel (II) kann über unterschiedliche synthetische Zugänge erfolgen. Mögliche Zugänge in Anlehnung an und unter Verwendung von konventionellen chemischen Synthesemethoden werden im Folgenden exemplarisch dargestellt. Aus Schema 1 ist eine mögliche Vorgehensweise zum Aufbau des Benzimidazolgrundkörpers der erfindungesgemäßen Verbindungen ersichtlich.

In einem ersten Schritt (Schema, Stufe i) erfolgt die Umsetzung der 3-Nitro-4-halogen-Aniline (1) zu den Sulfonamiden (2).

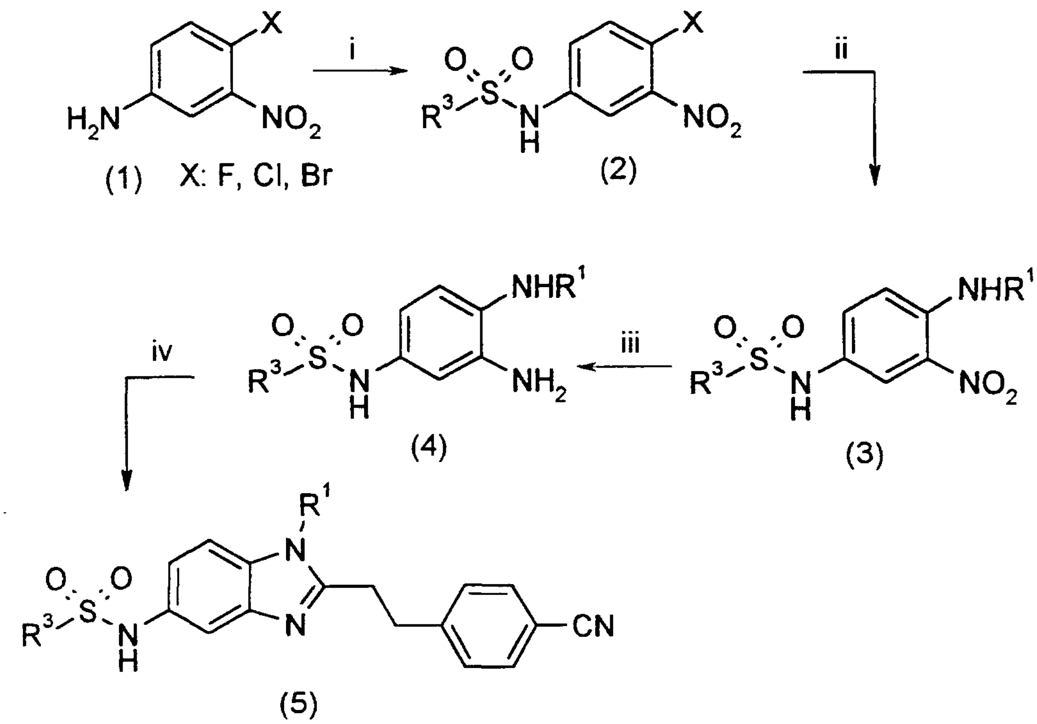


Schema 1:

Hierzu werden die Verbindungen (1) in einem geeigneten organischen, wasserfreien Lösemittel aufgenommen und gegebenenfalls bei Gegenwart einer organischen Base unter Kühlung mit dem gewünschten Arylsulfonsäurechlorid versetzt. Als organische Lösemittel kommen halogenierte Kohlenwasserstoffe wie Dichlormethan oder Chloroform sowie Lösemittel ausgewählt aus der Gruppe Dioxan, Tetrahydrofuran, Dimethylformamid, Acetonitril, Pyridin in Betracht. Erfindungsgemäß bevorzugt ist die Verwendung von Pyridin oder Pyridin im Gemisch mit Dichlormethan oder Chloroform als Lösemittel.
Erfolgt der Zusatz einer organischen Base, gelangen in erster Linie Amine wie Triethylamin, Diisopropylethylamin, N-Methylmorpholin oder auch Pyridin zur Anwendung. Die Zugabe des Arylsulfonsäurechlorids erfolgt vorzugsweise bei Temperaturen unterhalb der Raumtemperatur, besonders bevorzugt zwischen -40°C und 20°C, besonders bevorzugt bei -20°C bis 10°C. Nach beendeter Reaktion (ca. 0.5 - 2h) wird nach gängigen Verfahren aufgearbeitet. Eine Reinigung der Verbindungen (2) kann gegebenenfalls über Kristallisation aus unpolaren organischen Lösemitteln wie beispielsweise Diethylether, Petrolether, gegebenenfalls im Gemisch mit Ethylacetat erfolgen.

In einer zweiten Stufe (Schema 1, Stufe ii) erfolgt die Umsetzung der Sulfonamide (2) zu den Verbindungen (3). Die Aminolyse der Verbindungen (2) mit den primären Aminen $R^1$-$NH_2$ erfolgt in geeigneten organischen Lösemitteln wie beispielsweise Dimethylsulfoxid, N,N-Dimethylformamid, N-Methylpyrrolidon, Aceton oder gegebenenfalls auch in Wasser oder Alkoholen bei Raumtemperetur oder in einem Temperaturbereich von 30-80°C, bevorzugt 40-50°C.

Die Reduktion der Nitro-gruppe zu den Verbindungen (4, Schema 1, Stufe iii) gelingt beispielsweise durch katalytische Hydrierungen in organischen Lösemitteln wie beispielsweise Methanol, Ethanol, Isopropanol, Tetrahydrofuran, gegebenenfalls auch im Gemisch mit Dimethylformamid, Ethylacetat, Dioxan oder Essigsäure, bei erhöhtem Wasserstoffdruck oder bei Normaldruck bei Temperaturen zwischen 0-50°C, vorzugsweise bei 20-40'°C. Als Katalysatoren kommen gängoge Hydrierkatalysatoren in Betracht. Bevorzugt sind Palladium und Raney-Nickel. Erfindungsgemäß kommt vorzugsweise Palladium in Betracht. Besonders bevorzugt ist als Katalysator Palladium auf Kohle (5%). Eine alternative Vorgehensweise zur Reduktion der Nitroverbindungen (3) sieht die Verwendung von Reduktionsmitteln wie $Na_2S_2O_4$ oder $SnCl_2$ vor. Diese Umsetzung erfolgt in protischen, mit Wasser mischbaren organischen Lösemitteln wie kurzkettigen Alkoholen (Methanol, Ethanol, Isopropanol) oder in einer Mischung vorstehend genannter Lösemittel mit Wasser, gegebenenfalls mit Essigsäure, Dimethylformamid oder Ethylacetat. Die Reaktion wird üblicherweise bei erhöhter Temperatur, vorzugsweise unter Rückfluß des jeweiligen Lösemittels oder Lösemittelgemischs geführt. Nach vollständigem Umsatz der Ausgangsverbindungen (3) wird auf üblichem Wege aufgearbeitet. Eine Reinigung der Verbindungen (4) kann beispielsweise durch Kristallisation aus unpolareren organischen Lösemitteln wei Diethylether, Petrolether, gegebenenfalls im Gemisch mit Ethylacetat erfolgen.

In einem vierten Schritt (Schema 1, Stufe iv) gelingt der Ringschluß zu den Benzimidazolen (5) durch Umsetzung der Sulfonamid-Derivate (4) mit p-Cyanophenylpropionsäure in Gegenwart dehydratisierender Reagentien. Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan durchgeführt. Als dehydratisierende Mittel kommen beispielsweise in Betracht Chlorameisensäureisobutylester, Orthokohlensäuretetraethylester, Orthoessigsäuretrimethylester, 2,2-Dimethoxypropan, Tetramethoxysilan, Phosphoroxychlorid, Thionylchlorid, Trimethylchlorsilan, Phosphortrichlorid, Phosphorpentoxid, 1,2-Dihydro-2-ethoxy-chinolin-1-carbonsäureethylester (EEDQ), 1,2-Dihydro-2-*i*-propyloxy-chinolin-1 -carbonsäure-*i*-propylester (IIDQ), N,N'-Dicyclohexylcarbodiimid, N, N'-Dicyclohexylcarbodiimid/N-Hydroxysuccinimid, N,N'-Dicyclohexylcarbodiimid/1-Hydroxy-benztriazol, 2-(1H-Benzotriazol-1 -yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff. Gegebenenfalls kann sich der Zusatz einer Base wie Pyridin, 4-Dimethylaminopyridin, N-Methyl-morpholin oder Triethylamin als zweckmäßig erweisen. Die Umsetzung erfolgt üblicherweise bei Temperaturen zwischen 0 und 150°C, vorzugsweise bei Temperaturen zwischen 20 und 120°C.

Ein alternativer Zugang zu den Verbindungen (5) ist exemplarisch in Schema 2 dargestellt.

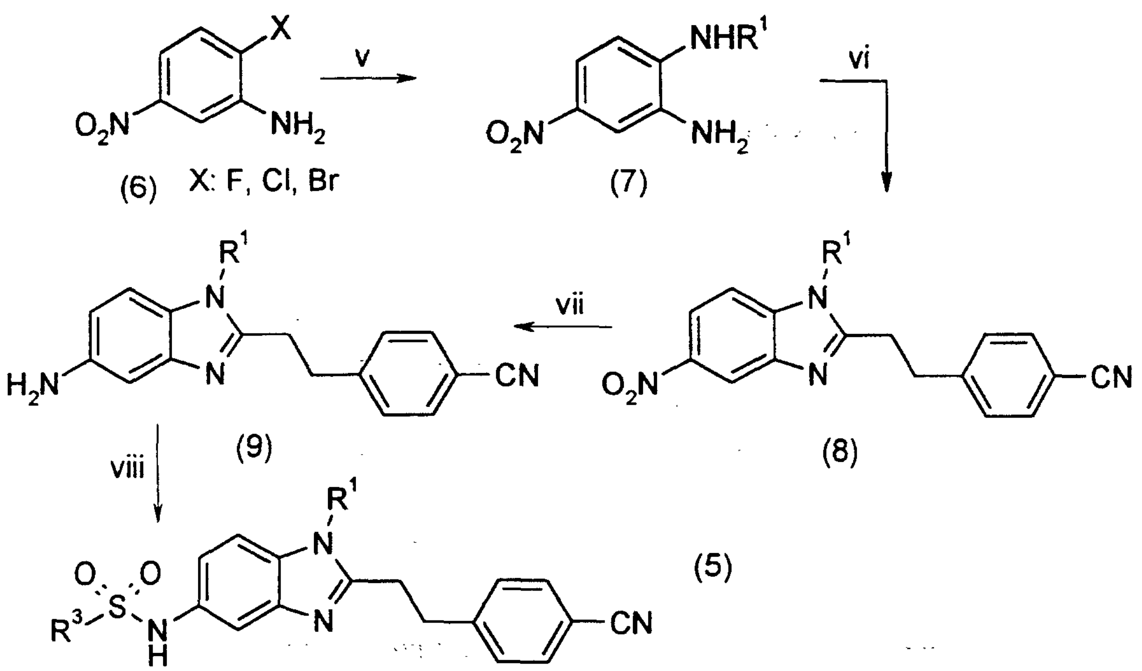


Schema 2:

Ausgehend von den 2-Halogen-5-nitro-anilinen (6) kann zunächst eine Aminolyse zu den Diaminonitrobenzolen (7) gemäß Schema 2 (Stufe v) erfolgen. Die Umsetzung der Verbindungen (7) mit p-Cyanophenylpropionsäure führt zu den Nitrobenzimidazolen (8, Stufe vi), welche reduktiv in die Amino-benzimidazole (9) überführt werden können (Stufe vii, Schema 2). Die Verbindungen (5) sind aus den Amino-benzimidazolen (9) durch Umsetzung mit den entsprechenden Arylsulfonsäurechloriden erhältlich (Schema 2, Stufe viii). Die experimentelle Durchführung der gemäß Schema 2 skizzierten Synthese gelingt in Analogie zu der für die Stufen i-iv (Schema 1) beschriebenen Vorgehensweise. Stufe v wird durchgeführt in Analogie zur Vorgehensweise nach Stufe ii, Stufe vi in Analogie zu der gemäß Stufe iv, Stufe vii in Analogie zu der für Stufe ii beschriebenen Vorgehensweise und abschließend Stufe viii entsprechend der experimentellen Durchführung nach Stufe i.

Ausgehend von den gemäß der Schemata 1 und 2 erhältlichen Benzimidazole (5) erfolgt die Bildung der Intermediate der Formel (III, siehe Schema 3) durch Umsetzung mit den Verbindungen $R^4$-Nu, wobei Nu für eine nukleofuge Austrittsgruppe wie beispielsweise Chlor, Brom, Iod, Methansulfonat, Methyltriflat, p-Toluolsulfonat etc. steht.

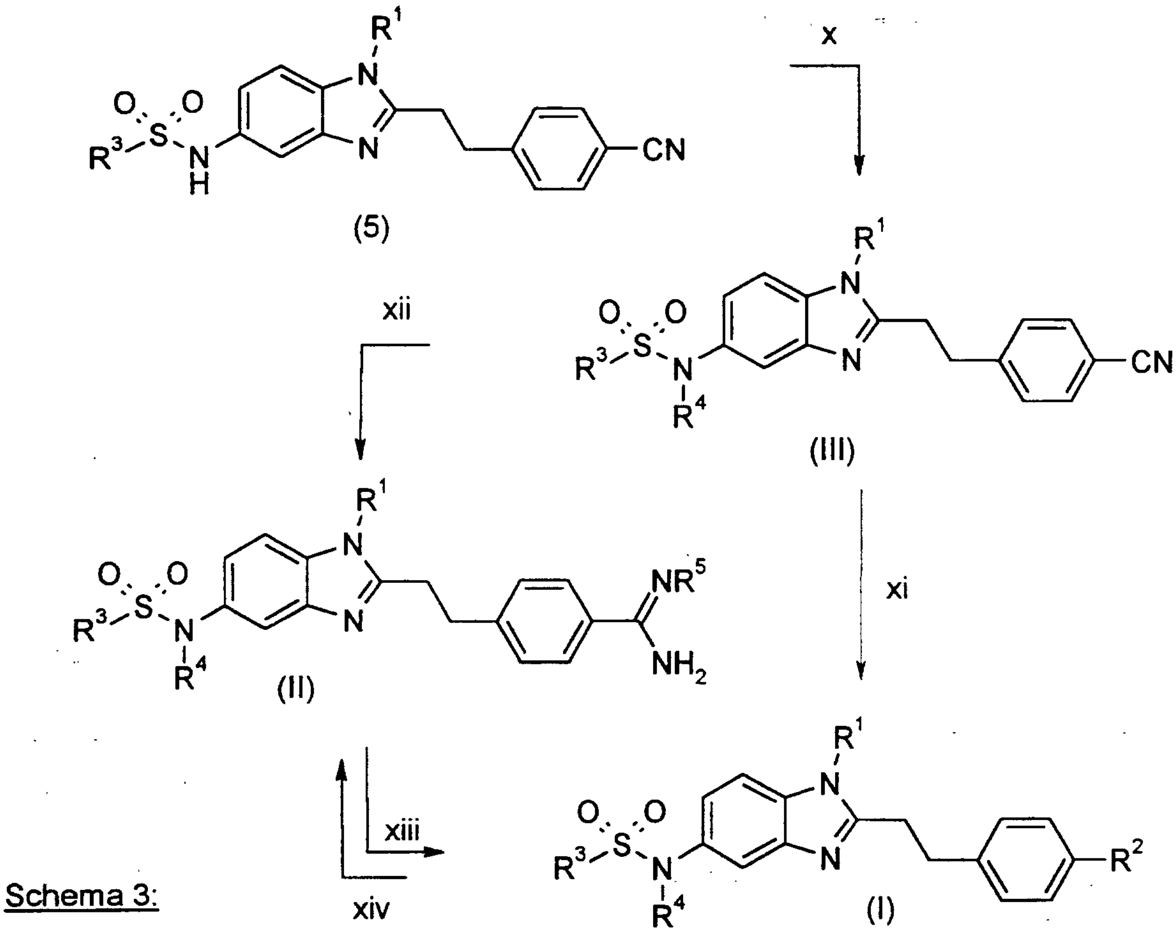


(5) (III) (II) (I)

Schema 3:

Alternativ dazu können die Intermediate (III) ausgehend von den Verbindungen (5) auch im Sinne einer reduktiven Aminierung durch Umsetzung mit entsprechend substituierten Ketonen oder Aldehyden unter reduktiven Bedingungen erhalten werden.

Zur Umsetzung der Verbindungen (5) mit $R^4$-Nu gemäß Stufe x wird wie folgt vorgegangen. Eine Verbindung (5) wird in einem polaren Lösungsmittels, wie Dimethylformamid, Dimethylactamid, Methylenchlorid, Tetrahydrofuran, bevorzugt Dimethylformamid und besonders bevorzugt wasserfreies, gegebenenfalls absolutes Dimethylformamid gelöst. Die so erhaltene Lösung wird mit einer Base und dem entsprechenden Alkylierungsmittel R4-Nu versetzt. Als Base kommen die Alkali- oder die Erdalkalicarbonate des Lithiums, Natriums, Kaliums, Calziums wie Natriumcarbonat, Lithiumcarbonat, Kaliumcarbonat, Calziumcarbonat und bevorzugt Kaliumcarbonat in Betracht. Ferner sind die Alkali- oder Erdalkalihydroxide des Lithiums, Natriums, Kaliums, Magnesiums, Calziums, bevorzugt jedoch Natriumhydroxid, Kaliumhydroxid, Lithiumhydroxid und Calziumhydroxid in Alkoholen oder Wasser einsetzbar. Das Reaktionsgemisch wird 0.5-8h, bevorzugt 1-4h bei erhöhter Temperatur, vorzugsweise bei 50-120°C, besonders unter Rückfluß des verwendeten Lösemittels gerührt. Nach vollständigem Umsatz wird auf üblichem Wege aufgearbeitet und das erhaltene Rohprodukt durch Kristallisation oder Chromatographie an Kieselgel gereinigt.

Werden die Intermediate (III) aus den Verbindungen (5) durch reduktive Aminierung erhalten, wird wie folgt vorgegangen. In einem geeigneten Lösemittel wie beispielsweise Dichlormethan, Dichlorethan, Methanol, Ethanol, Tetrahydrofuran oder Toluol wird die Verbindung (5) gelöst und zwischen 0-60°C, vorzugsweise bei 20-40°C mit der entsprechenden Carbonylverbindung in Gegenwart einer Säure, vorzugsweise einer Carbonsäure, besonders bevorzugt einer kurzkettigen Carbonsäure, höchst bevorzugt Essigsäure versetzt. anschließend erfolgt die Zugabe eines geeigneten Reduktionsmittels. Als Reduktionsmittel kommen erfindungsgemäß in Betracht $Na[HB(OAc)_3]$, $Na[BH_3CN]$, $NaBH_4$, Pd/C-$H_2$, bevorzugt ist $Na[HB(OAc)_3]$. Nach üblicher Aufarbeitung erfolgt die Reinigung des Produkts durch Kristallisation oder Chromatographie an Kieselgel.

Gemäß Stufe xi sind aus den Intermediaten (III) die erfindungsgemäßen Verbindungen der allgmeinen Formel (I) zugänglich. Zur Herstellung der erfindungsgemäßen Verbindungen der allgemeinen Formel (I) in denen $R^2$ -C(=NH)$NH_2$ bedeutet kann auf unterscheidliche Art und Weise vorgegangen werden.

Eine Verbindung der allgemeinen Formel (I) erhält man beispielsweise durch Behandlung einer Verbindung der allgemeinen Formel (III) mit einem entsprechenden Alkohol wie Methanol, Ethanol, n-Propanol, Isopropanol oder Benzylalkohol gegebenenfalls im Gemisch mit einem anderen organischen Lösungsmittel wie beispielsweise Chloroform, Nitrobenzol oder Toluol in Gegenwart einer Säure wie Salzsäure oder durch Umsetzung eines entsprechenden Amids mit einem Trialkyloxoniumsalz wie Triethyloxonium-tetrafluorborat in einem Lösemittel wie Methylenchlorid, Tetrahydrofuran oder Dioxan bei Temperaturen zwischen -10 und 50°C, vorzugsweise jedoch bei 0-20°C und nachfolgender Aminolyse mit beispielsweise alkoholischer Ammoniaklösung. Alternativ dazu lassen sich die Verbindungen der allgemeinen Formel (I) erhalten durch Umsetzung einer Verbindung der allgemeinen Formel (III) mit Schwefelnukleophilen wie z.B. Schwefelwasserstoff, Ammonium- bzw. Natriumsulfid, Natriumhydrogensulfid, Kohlenstoffdisulfid, Thioacetamid oder Bistrimethylsilylthioether gegebenenfalls in Gegenwart von Basen wie Triethylamin, Ammoniak, Natriumhydrid oder Natriumalkoholat in Lösungsmitteln wie Methanol, Ethanol, Wasser, Tetrahydrofuran, Pyridin , Dimethylformamid oder 1,3-Dimethyl-imidazolidin-2-on bei 20-100 °C und anschließende Behandlung mit einem geeigneten Methylierungsmittel wie z.B. Methyliodid oder Dimethylsulfat in einem Lösungsmittel wie Acetonitril oder Aceton bei Temperaturen zwischen -10 und 50°C, vorzugsweise jedoch bei 0-20°C und anschließende Behandlung mit Ammoniak, Ammoniumcarbonat oder Ammoniumchlorid in einem geeigneten Alkohol, wie beispielsweise Methanol, Ethanol, Isopropanol etc. bei Temperaturen zwischen
-10 und 50°C, vorzugsweise jedoch bei 0-20°C.
Ferner sind die erfindungsgemäßen Verbindungen der allgemeinen Formel (I) zugänglich durch Behandlung einer Verbindung der allgemeinen Formel (III) mit Lithiumhexamethyldisilazid in einem geeigneten organischen Lösungsmittel wie z.B. Tetrahydrofuran bei Temperaturen zwischen -20 und 50 °C, vorzugsweise jedoch bei 0-20 °C und anschließende Hydrolyse mit verdünnter Salzsäure bei 0-5 °C.
Ein weiterer alternativer Zugang zu Verbindungen der allgemeinen Formel (I) gelingt durch Behandlung einer Verbindung der allgemeinen Formel (III) mit Ammoniumchlorid und Trimethylaluminium in einem geeigneten organischen Lösungsmittel wie z.B. Toluol bei Temperaturen zwischen 20 und 150 °C, vorzugsweise jedoch bei 110 °C.

Verbindungen der allgemeinen Formel (I) in denen $R^2$ $-CH_2-NH_2$ bedeutet lassen sich aus den Intermediaten (III) beispielsweise durch katalytische Hydrierung an Raney-Nickel erhalten. Diese Umsetzungen werden vorzugsweise in protischen organischen Lösemitteln wie kurzkettigen Alkoholen (Methanol, Ethynol oder Isopropanol) bei Temperaturen zwischen 10-40°C, vorzugsweise bei 20-30°C unter Normaldruck durchgeführt.

Eine Verbindung der allgemeinen Formel (II) erhält man beispielsweise durch Behandlung einer Verbindung der allgemeinen Formel (III, Schema 3, Stufe xii) mit Hydroxylamin in Gegenwart von Carbonaten oder Alkoholaten der Alkali- oder Erdalkalimetalle in Lösemitteln wie Methanol, Ethanol, n-Propanol oder Isopropanol gegebenenfalls im Gemisch mit Dioxan oder Tetrahydrofuran. Die Alkoholate können dargestellt werden aus den jeweiligen Alkalimetallen oder Metallhydriden und dem entsprechenden Alkohol. Die Reaktion wird vorzugsweise bei 20-100°C, besonders bevorzugt bei der Siedetemperatur des verwendeten Lösemittels durchgeführt. Verbindungen der allgemeinen Formel (II) sind alternativ zugänglich durch Behandlung einer Verbindung der allgemeinen Formel (III) mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösemittel wie Methylenchlorid, Tetrahydrofuran oder Dioxan bei Temperaturen zwischen -10 und 50°C, vorzugsweise jedoch bei 0-20°C und anschließende Behandlung mit Hydroxylamin in Gegenwart von Basen in einem geeigneten Alkohol, wie Methanol, Ethanol, Isopropanol etc. bei Temperaturen zwischen -10 und 50°C, vorzugsweise jedoch bei 0-20°C.

Eine Verbindung der allgemeinen Formel (I) erhält man beispielsweise durch Behandlung einer Verbindung der allgemeinen Formel (II, Schema 3, Stufe xiii) mit Wasserstoff in Gegenwart von Hydrierkatalysatoren wie Raney-Nikkel oder Rhodium/Aluminiumoxid in Wasser oder Methanol gegebenenfalls unter Zusatz von Säuren wie Salzsäure oder Methansulfonsäure oder durch Behandlung mit Wasserstoff in Gegenwart von Palladium/Kohle in Essigsäure/Essigsäureanhydrid bei 20-50 °C und 1-5 bar Wasserstoffdruck, bevorzugt bei Raumtemperatur und Normaldruck,

Die Acyl- oder Alkoxycarbonyl-Prodrugs (II) der Verbindung mit der allgemeinen Formel (I) erhält man durch Umsetzung der Verbindungen der allgemeinen Formel (I) mit den entsprechenden Säurechloriden in Gegenwart von Basen wie z.B. Triethylamin, N-Methylmorpholin, Diethylisopropylamin oder DBU in einem geeigneten Lösungsmittel wie Methylenchlorid, Chloroform, Tetrahydrofuran, Acetonitril, Dimethylformamid oder Dimethylsulfoxid.

Ihrer zentralen Bedeutung für die Synthese der erfindungsgemäßen Verbindungen der allgemeinen Formel (I) sowie für die Synthese der Prodrugs der allgemeinen Formel (II) entsprechend, zielt ein weiterer Aspekt der vorliegenden Erfindung auf die Intermediate der allgemeinen Formel (III)

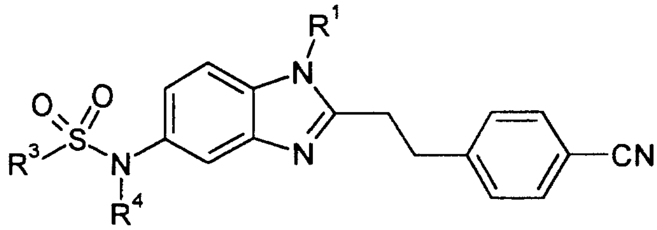

(III)

in der die Reste $R^1$, $R^3$ und $R^4$ die vorstehend genannte Bedeutung aufweisen können. Die Verbindungen der allgemeinen Formel (III) stellen, wertvolle Zwischenprodukte zur Herstellung der erfindungsgemäßen Benzimidazol-Derivate der allgemeinen Formel (I) sowie der der erfindungsgemäßen Prodrugs der allgemeinen Formel (II) dar.

Im Folgenden werden exemplarische Vorgehensweisen zur Herstellung der erfindungsgemäßen Verbindungen detaillierter beschrieben. Die nachfolgenden Synthesebeispiele dienen ausschließlich der detaillierteren Erläuterung, ohne den Gegenstand der Erfindung auf selbige zu beschränken.

**Beispiel 1:** N-{2-[2-(4-Amidinophenyl)-ethyl]-1-methyl-benzimidazol-5-yl}-N-diethylaminoethyl-(4-ethoxycarbonyl-benzolsulfonamid)-hydrochlorid

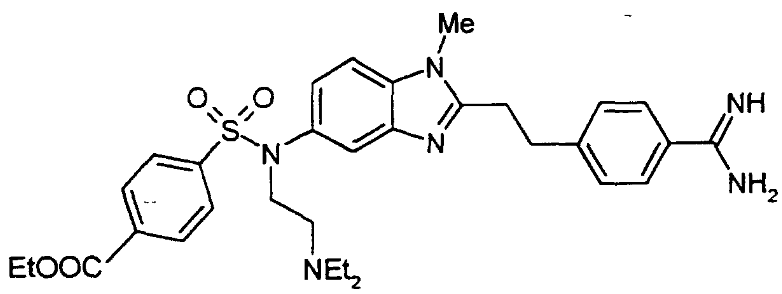

a) $N^1$-Methyl-1,2-diamino-4-nitrobenzol

2-Fluor-5-nitro-anilin (15.0 g, 160 mmol) wird in 480 mL 40%iger wäßriger Methylaminlösung aufgenommen, 2.5 Tage bei Raumtemperatur und 2 h bei 40-50 °C gerührt. Es wird mit Wasser verdünnt, der Feststoff abfiltriert, mit Wasser gewaschen und getrocknet.
Ausbeute: 26 g (97%); Schmp.: 171-173 °C;

b) 2-[2-(4-Cyanophenyl)-ethyl]-1-methyl-5-nitro-benzimidazol

$N^1$-Methyl-1,2-diamino-4-nitrobenzol (8,3 g, 49,6 mmol) und *p*-Cyanophenylpropionsäure (9,6 g, 55 mmol) werden in 90 mL $POCl_3$ aufgenommen, und 1,5 h unter Rückfluß erhitzt. Nach dem Abkühlen wird das überschüssige $POCl_3$ mit Eiswasser zersetzt. Es wird mit $NH_3$ unter Rühren / Kühlung alkalisch gestellt und 1 h bei Raumtemperatur gerührt. Der Feststoff wird abfiltriert, mit Wasser gewaschen und aus DMF umkristallisiert.
Ausbeute: 11,7 g (76%); Schmp.: 202-204 °C;

c) 5-Amino-2-[2-(4-cyanophenyl)-ethyl]-1-methyl-benzimidazol

2-[2-(4-Cyanophenyl)-ethyl]-1-methyl-5-nitro-benzimidazol (5,5 g, 18 mmol) in 150 mL THF/75 mL Methanol wird in Gegenwart von 1,0 g 5% Pd/C bei Raumtemperatur und Normaldruck hydriert. Der Katalysator wird abfiltriert, das Filtrat nicht komplett zur Trockene eingeengt, mit 100 mL Acetonitril verdünnt und bis zu einem Restvolumen von 30 mL eingeengt. Der Kristallbrei wird abgekühlt und filtriert. Die Kristalle werden mit kaltem Acetonitril und Ether gewaschen.
Ausbeute: 4,7 g (94%); Schmp.: 187-192 °C;

d) N-{2-[2-(4-Cyanophenyl)-ethyl]-1-methyl-benzimidazol-5-yl}-{4-carboxybenzolsulfonamid)

10 mmol 5-Amino-2-[2-(4-cyanophenyl)-ethyl]-1-methyl-benzimidazol werden in 10 mL Pyridin aufgenommen und auf 10 °C gekühlt. Das Benzolsulfonsäurechlorid (ca. 11 mmol) wird unter Rühren innerhalb von ca. 20 min bei 10-20 °C zugetropft. Es wird 30 min. bei RT gerührt und auf Eiswasser gegossen. Nach dem Ansäuern mit konz. HCl wird mit 50 mL Ethylacetat extrahiert, mit Wasser gewaschen, getrocknet und eingeengt. Der verbleibende Rückstand wird durch Chromatographie an Kieselgelgereinigt.
Ausbeute: 48 %; Schmp.: 158-160 °C;

e) N-{2-[2-(4-Cyanophenyl)-ethyl]-1-methyl-benzimidazol-5-yl}-(4-ethoxycarbonylbenzolsulfonamid)

N-{2-[2-(4-Cyanophenyl)-ethyl]-1-methyl-benzimidazol-5-yl}-(4-carboxybenzolsulfonamid) (2.0 g, 4.3 mmol) wird in 10 mL Thionylchlorid 1.5 h unter Rückfluß erhitzt. Es wird mit 50 mL Ethylacetat verdünnt, das kristalline Hydrochlorid des Säurechlorides abfiltriert und mit Ethylacetat/Diethylether gewaschen. Der Feststoff wird in 40 mL Ethanol aufgeschlämmt und langsam bis zum Rückfluß erhitzt. Das Ethanol wird zur Hälfte abdestilliert, nach dem Abkühlen mit Eiswasser versetzt und mit wässriger Kaliumcarbonatlösung unter Rühren alkalisch gestellt. Es wird mit Wasser verdünnt, der Feststoff abfiltriert und mit Wasser gewaschen.
Ausbeute: 1.8 g (85 %); Schmp: 160-163 °C;

f) N-{2-[2-(4-Cyanophenyl)-ethyl]-1-methyl-benzimidazol-5-yl}-N-diethylaminoethyl-(4-ethoxycarbonyl-benzolsulfonamid)

6.1 mmol N-{2-[2-(4-Cyanophenyl)-ethyl]-1-methyl-benzimidazol-5-yl}-(4-ethoxycarbonyl-benzolsulfonamid), Diethylaminoethylchlorid (1.2 g, 7.0 mmol), 4.0 g Kaliumcarbonat sowie Katalytische Mengen Kaliumiodid werden in 50 mL Dimethylfomamid etwa 1.5 h unter Rückfluß erhitzt. Nach dem Abkühlen wird auf Eiswasser gegossen, mit 100 mL Ethylacetat extrahiert, getrocknet und eingeengt. Der Rückstand wird mit Dichlormethan/Methanol über eine Kieselgelsäule filtriert. Nach dem Einengen wird aus Aceton/Diethylether kristallisiert.
Ausbeute: 80 %; Schmp.: 110-112 °C;

g) N-{2-[2-(4-Amidinophenyl)-ethyl]-1-methyl-benzimidazol-5-yl}-N-diethylaminoethyl-(4-ethoxycarbonyl-benzolsulfonamid)-hydrochlorid

5,7 mmol N-{2-[2-(4-Cyanophenyl)-ethyl]-1-methyl-benzimidazol-5-yl}-N-diethylaminoethyl-(4-ethoxycarbonyl-benzolsulfonamid) werden in etwa 120 mL einer gekühlten bei 0 °C gesättigten ethanolischen HCl-Lösung aufgenommen. Es wird bis zur vollständigen Auflösung des Eduktes gerührt und anschließend über Nacht bei 0-5 °C gehalten. Das Ethanol wird bei maximal 40 °C abdestilliert und der Rückstand in 100 mL einer bei 0 °C gesättigten, ethanolischen Ammoniak-Lösung aufgenommen. Es wird 2 h bei Raumtemperatur, 3 h bei 50-60 °C gerührt, mit weiteren 30 mL gesättigter Ammoniaklösung versetzt und 2 h unter Rückfluß gekocht. Es wird mit 50 mL Wasser und 60 mL Ethylacetat versetzt, 30 min. bei Raumtemperatur gerührt. Nach Abdestillieren des Lösemittels im Vakuum wird der verbleibende Rückstand durch Chromatographie an Kieselgel gereinigt.
Ausbeute: 76% (amorpher Feststoff);
Masse: ber.: [604], gef.: $[M+H]^+$ 605;
$^1$H-NMR (250 MHz, DMSO-d6): δ=9.25; 9.02 (4H, 2s, $-C(=NH_2^+)NH_2$); 8.13-6.80 (11H, m, aryl-H); 4.35 (2H, qu, J=7.0 Hz, $OCH_2CH_3$); 3.71 (3H, s, $N-CH_3$); 3.36 (4H, s, $-CH_2-CH_2-$); 2.48 (4H, m, $N-(CH_2-CH_3)_2$); 1.34 (3H, t, J=7.0 Hz, $OCH_2CH_3$); 0.94 (6H, m, $N-(CH_2-CH_3)_2$).

**Beispiel 2:** N-{2-[2-(4-Amidinophenyl)ethyl]-1-methyl-benzimidazol-5-yl}-N-diethylaminoethyl-(dansylamid)-dihydrochlorid

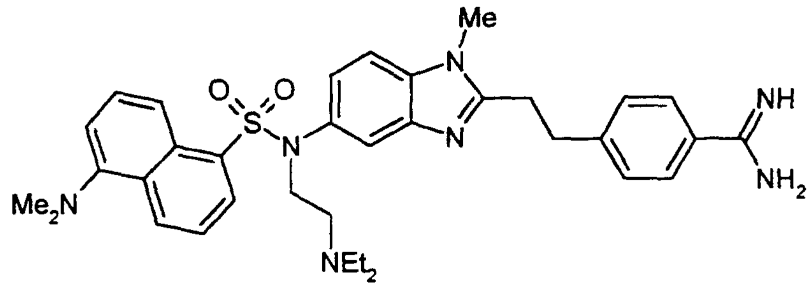

a) N-{2-[2-(4-Cyanophenyl)-ethyl]-1-methyl-benzimidazol-5-yl}-(dansylamid)

Die Umsetzung erfolgt ausgehend von 8 mmol 5-Amino-2-[2-(4-cyanophenyl)-ethyl]-1-methyl-benzimidazol (erhältlich gemäß Beispiel 1, Stufe c) in Analogie zu der unter Beispiel 1, Stufe d beschriebenen Vorgehensweise. Das Produkt wird durch Kristallisation aus Ethylacetat erhalten.
Ausbeute: 83%; Schmp.: 216-218 °C;

b) N-{2-[2-(4-Cyanophenyl)-ethyl]-1-methyl-benzimidazol-5-yl}-N-diethylaminoethyl-(dansylamid)

Die Umsetzung erfolgt ausgehend von 3.9 mmol N-{2-[2-(4-Cyanophenyl)-ethyl]-1-methyl-benzimidazol-5-yl}-(dansylamid) in Analogie zu der unter Beispiel 1, Stufe f beschriebenen Vorgehensweise in Dimethylformamid als Lösemittel. Das Produkt wird durch Kristallisation aus Ethylacetat/Diethylether erhalten.
Ausbeute: 80%; Schmp.: 143-145 °C;

c) N-{2-[2-(4-Amidinophenyl)-ethyl]-1-methyl-benzimidazol-5-yl}-N-diethylaminoethyl-(dansylamid)-dihydrochlorid

Die Umsetzung erfolgt ausgehend von 3.0 mmol N-{2-[2-(4-Cyanophenyl)-ethyl]-1-methyl-benzimidazol-5-yl}-N-diethylaminoethyl-(dansylamid) in Analogie zu der unter Beispiel 1, Stufe g beschriebenen Vorgehensweise. Das Produkt wird durch Chromatographie an Kieselgel erhalten.
Ausbeute: 70%(amorpher Feststoff);
Masse:.: [625], gef.: $[M+H]^+$ 626;
$^1$H-NMR (250 MHz, DMSO-d6): δ=9.43; 9.25 (4H, 2d, $-C(=NH_2^+)NH_2$); 8.60-6.91 (13H, m, aryl-H); 4.00; 2.78 (4H, 2m, $N-CH_2CH_2N$); 3.73 (3H, s, $N-CH_3$); 3.22 (4H, s, $-CH_2-CH_2-$); 2.84 (6H, s, $N-(CH_3)_2$); 2.76 (4H, m, $N-(CH_3CH_3)_2$; 0.94 (6H, m, $N-(CH_2CH_3)_2$).

Sofern nicht anders beschrieben, wurden in Analogie zu den vorstehend beschriebenenen Verfahren oder nach gemeinhin bekannten Standardverfahren u.a. die nachfolgend beschriebenen Verbindungen erhalten.

**Beispiel 3:** N-{2-[2-(4-Amidinophenyl)-ethyl]-1-methyl-benzimidazol-5-yl}-N-diethylaminoethyl-(3-brom-benzolsulfonamid)-hydrochlorid

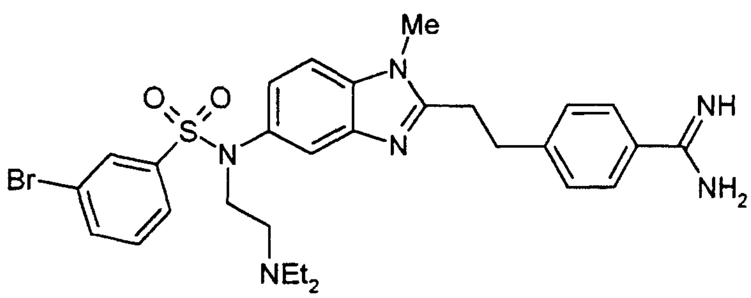

Masse: ber.: [611], gef.: $[M+H]^+$ 611/613;

**Beispiel 4:** N-{2-[2-(4-Amidinophenyl)-ethyl]-1-methyl-benzimidazol-5-yl}-N-diethylaminoethyl-(4-nitro-benzolsulfonamid)-hydrochlorid

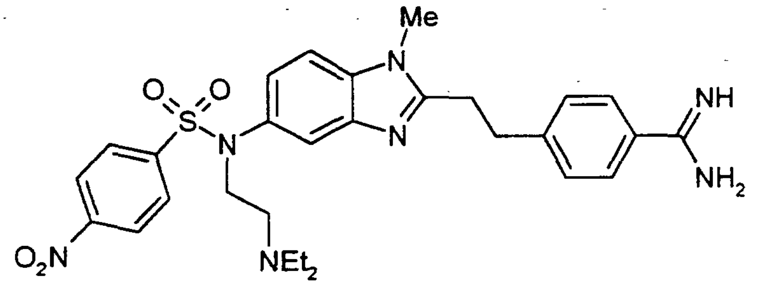

Ausbeute: 83% (amorpher Feststoff);
Masse: : ber.: [577], gef.: $[M+H]^+$ 578
$^1$H-NMR (250 MHz, DMSO-d6): δ=9.65; 9.47 (4H, 2s, $-C(=NH_2^+)NH_2$); 8.64-7.04 (11H, m, aryl-H); 4.00; 2.80 (4H, 2m, $N-CH_2CH_2-N$); 3.84 (3H, s, $N-CH_3$); 3.52 (4H, s, $-CH_2-CH_2-$); 3.24 (4H, m, $N-(CH_2CH_3)_2$); 1.02 (6H, m, $N-(CH_2CH_3)_2$).

**Beispiel 5:** N-{2-[2-(4-Amidinophenyl)-ethyl]-1-methyl-benzimidazol-5-yl}-N-diethylaminoethyl-(3-methoxy-benzolsulfonamid)-hydrochlorid

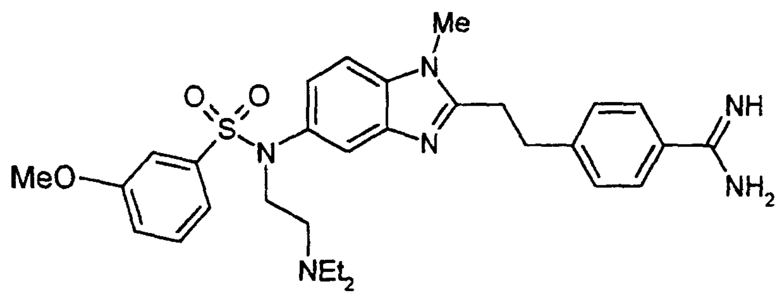

Masse: ber.: [562], gef.: $[M+H]^+$ 563, $[M+2H]^{2+}$ 282.
$^1$H-NMR (250 MHz; DMSO-d6): δ= 10.80; 9.45; 9.28 (5H, 3s, $H^+$; $-C(=NH_2^+)NH_2$); 7.49-6.93 (11H, m, aryl-H); 4.10; 3.14 (4H, 2m, $N-CH_2CH_2-N$); 3.78 (3H, s, $N-CH_3$); 3.74 (3H, s, $OCH_3$); 3.27 (4H, s, $-CH_2-CH_2-$); 3.12 (4H, m, $N-(CH_2CH_3)_2$; 1.14 (6H, t, J=7.0 Hz, $N-(CH_2-CH_3)_2$).

**Beispiel 6:** N-{2-[2-(4-Amidinophenyl)-ethyl]-1-methyl-benzimidazol-5-yl}-N-diethylaminoethyl-(3-methyl-benzolsulfonamid)-hydrochlorid

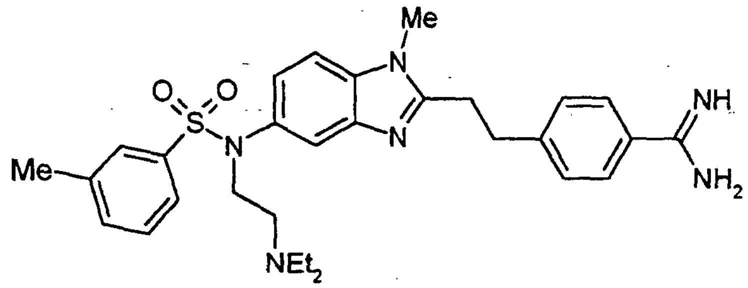

Masse: ber.: [546], gef.: $[M+H]^+$ 547, $[M+2H]^{2+}$ 274.
$^1$H-NMR (250 MHz, DMSO-d6): δ=7.75-6.92 (11H, m, aryl-H); 4.03; 3.28 (4H, 2m, N-$CH_2CH_2$-N); 3.72 (3H, s, N-$CH_3$); 3.25 (4H, s, -$CH_2$-$CH_2$); 3.21 (4H, m, N-$(CH_2CH_3)_2$); 2.35 (3H, s, 0-$CH_3$); 1.27 (6H, t, J=7.0 Hz, N-$(CH_2CH_3)_2$).

**Beispiel 7:** N-{2-[2-(4-Amidinophenyl)-ethyl]-1-methyl-benzimidazol-5-yl}-N-diethylaminoethyl-(3-chlor-benzolsulfonamid)-hydrochlorid

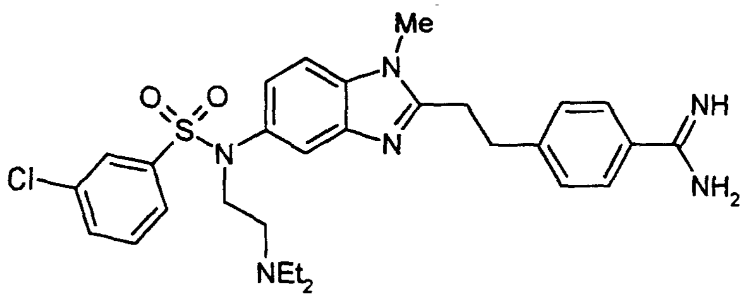

Masse: ber.: [567], gef.: $[M+H]^+$ 567, 569
$^1$H-NMR (250 MHz, DMSO-d6): δ=10.76; 9.42; 9.24 (5H, 3s, $H^+$; -C(=$NH_2^+$)$NH_2$); 7.90-6.90 (11H, m, aryl-H); 4.13; 3.14 (4H, 2m, N-$CH_2CH_2$-N); 3.76 (3H, s, N-$CH_3$); 3.25 (4H, s, -$CH_2CH_2$-); 3.14 (4H, m, N-$(CH_2CH_3)_2$; 1.76 (6H, m, N-$(CH_2$-$CH_3)_2$).

**Beispiel 8:** N-{2-[2-(4-Amidinophenyl)-ethyl]-1-methyl-benzimidazol-5-yl}-N-diethylaminoethyl-(4-amino-benzolsulfonamid)-tetrahydrochlorid

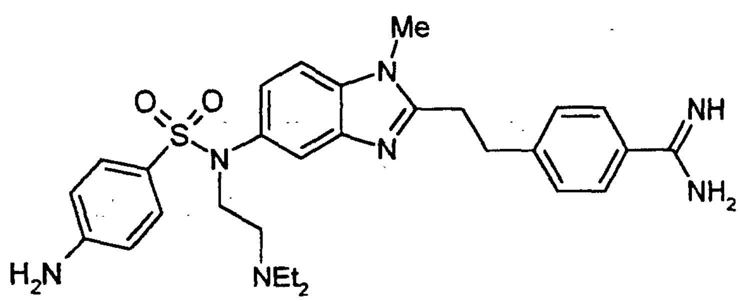

3.9 mmol N-{2-[2-(4-Amidinophenyl)-ethyl]-1-methyl-benzimidazol-5-yl}-N-diethylaminoethyl-(4-nitro-benzolsulfonamid) (Beispiel 4) werden in reinem MeOH bei Normaldruck und Raumtemperatur an PD/C (5%) hydriert. Die Titelverbindung wird aus Methanol/Isopropanol mit konzentrierter Salzsäure kristallisiert.
Ausbeute: 74%; Schmp.: 190-205 °C;
Masse: ber.: [547], gef.: $[M+H]^+$ 548
$^1$H-NMR (250 MHz, DMSO-d6): δ=9.49; 9.32 (4H, 2s, $-C(=NH_2^+)NH_2$); 7.25-6.57 (11H, m, aryl-H); 6.19 (2H, s, $NH_2$); 3.76 (3H, s, $N-CH_3$); 3.27 (4H, s, $-CH_2-CH_2-$); 2.77 (4H, m, $N-(CH_2CH_3)_2$; 0.99 (6H, m, $N-(CH_2-CH_3)_2$).

**Beispiel 9:** N-{2-[2-(4-Amidinophenyl)-ethyl]-1-methyl-benzimidazol-5-yl}-N-diethylaminoethyl-(4-acetylamino-benzolsulfonamid)

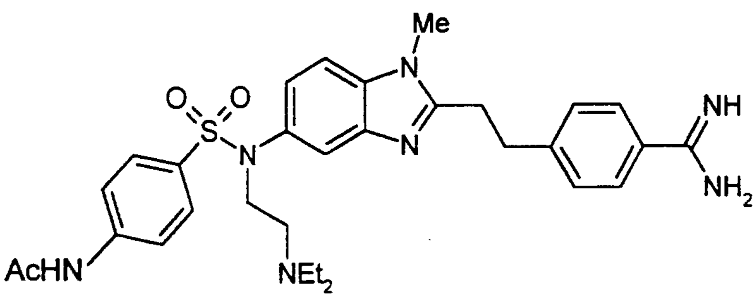

1.4 g (2.4 mmol) N-{2-[2-(4-Amidinophenyl)-ethyl]-1-methyl-benzimidazol-5-yl}-N-diethylaminoethyl-(4-amino-benzolsulfonamid)-tetrahydrochlorid (Beispiel 8) in 5 mL Ethylacetat und 0.5 mL Acetanhydrid 1 h bei Raumtemperatur gehalten, kurz auf 30-40 °C erwärmt und weitere 16 h bei Raumtemperatur belassen. Es wird mit 10 mL Wasser versetzt und eingeengt. Der Rückstand wird in Wasser aufgenommen und mit wässriger Kaliumcarbonatlösung alkalisch gestellt. Nach Zugabe von 5 mL Ethylacetat wird gut durchgerührt und nach 1 h der ausgefallene Feststoff abfiltriert, mit Wasser, Ethylacetat und Diethylether gewaschen. Ausbeute: 88%;
Masse: ber.: [589], gef.: $[M+H]^+$ 590
$^1$H-NMR (250 MHz, DMSO-d6): δ=7.97-6.93 (11H, m, aryl-H); 3.75 (3H, s, $N-CH_3$); 3.21 (4H, s, $-CH_2CH_2-$); 2.43 (4H, qu, J=7.0 Hz, $N-(CH_2CH_3)_2$); 2.13 (3H, s, $CH_3-C=O$); 0.85 (6H, t, J=7.0 Hz, $N-(CH_2CH_3)_2$).

**Beispiel 10:** N-{2-[2-(4-Amidinophenyl)-ethyl]-1-methyl-benzimidazol-5-yl}-N-diethylaminoethyl-(4-chlor-benzolsulfonamid)

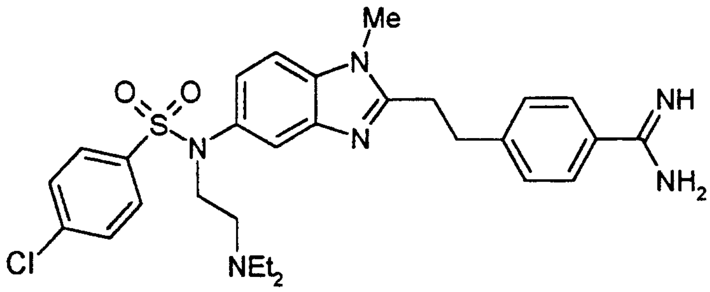

Ausbeute: 79%; Schmp.: 88-90 °C;
Masse: ber.: [567], gef.: $[M+H]^+$ 567/569 (1 Cl)
$^1$H-NMR (250 MHz, $CD_3OD$): δ=7.67-6.96 (11H, m, aryl-H); 3.76; 2.59 (4H, 2m, $N-CH_2-CH_2-N$); 3.63 (3H, s, $N-CH_3$); 3.21 (4H, m, $-CH_2CH_2-$); 2.51 (4H, qu, J=7.0 Hz, $N-(CH_2CH_3)_2$; 0.95 (6H, t, J=7.0 Hz, $N-(CH_2-CH_3)_2$).

**Beispiel11:** N-{2-[2-(4-Amidinophenyl)-ethyl]-1-methyl-benzimidazol-5-yl}-N-diethylaminoethyl-[4-ethoxycarbonylmethylaminocarbonyl-benzolsulfonamid]-hydrochlorid

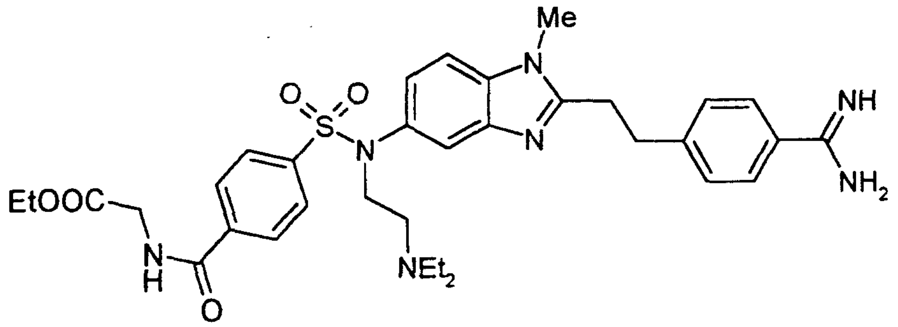

Ausbeute: 42%;
Masse: ber.: [661], gef.: $[M+H]^+$ 662
$^1$H-NMR (250 MHz, DMso-d6): δ=9.30; 9.11 (4H, 2s, $-C(=NH_2^+)NH_2$); 9.27 (1H, t, J=5.8 Hz, $NH-CH_2$); 8.09-6.79(11H, m, aryl-H); 4.12 (2H, qu, J=7.0 Hz; $O-CH_2CH_3$); 4.02 (2H, d, J=5.8 Hz, $NH-CH_2-$); 3.72 (3H, s, $N-CH_3$); 2.88 (4H, 2m, $N-CH_2CH_2-N$); 3.22 (4H, s, $-CH_2CH_2-$); 1.23 (3H, t, J=7.0 Hz, $OCH_2\ CH_3$); 1.02 (6H, m, $N-(CH_2\ CH_3)_2$; 2.88 (4H, m, $N-(CH_2CH_3)_2$).

**Beispiel 12:** N-{2-[2-(4-Amidinophenyl)-ethyl]-1-methyl-benzimidazol-5-yl}-N-diethylaminoethyl-(4-methoxy-benzolsulfonamid)-trihydrochlorid

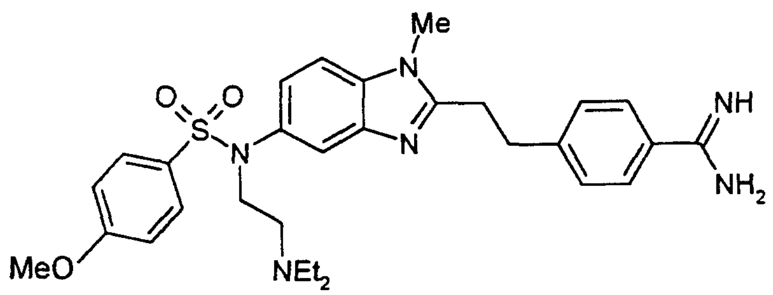

1.0 g (1.7 mmol) N-{2-[2-(4-(Amino-hydroximinomethyl)-phenyl)-ethyl]-1-methylbenzimidazol-5-yl}-N-diethylaminoethyl-(4-methoxy-benzolsulfonamid) (erhältlich in Analogie zu der unter Beispiel 16 beschriebenen Vorgehensweise) werden in 30mL Essigsäure und 0.5 mL Acetanhydrid aufgenommen und in Gegenwart von 5 %igem Pd/C (0.3 g) bei Normaldruck hydriert. Der Katalysator wird abfiltriert, das Filtrat nach Zugabe von 5 mL Wasser eingeengt und der Rückstand über Kieselgel chromatographiert. Das Trihydrochlorid wird aus Ethanol/Aceton mit konzentrierter Salzsäure ausgefällt und mit Ethanol/Aceton gewaschen.
Ausbeute: 0.6 g (52%); Schmp: 190-200 °C;
Masse: ber.: [562], gef.: $[M+H]^+$ 563
$^1$H-NMR (250 MHz, $CD_3OD$): δ=7.17 (11H, m, aryl-H); 4.19; 3.36 (4H, 2m, $N-CH_2-CH_2-N$); 4.05 (3H, s, $OCH_3$); 3.95 (3H, s, $N-CH_3$); 3.38 (4H, $N-(CH_2CH_3)_2$); 3.68; 3.36 (4H, 2m, $-CH_2CH_2-$); 1.34 (6H, t, J=7.0 Hz, $N-(CH_2CH_3)_2$).

**Beispiel 13:** N-{2-[2-(4-Amidinophenyl)-ethyl]-1-methyl-benzimidazol-5-yl}-N-methyl-(dansylamid)-hydrochlorid

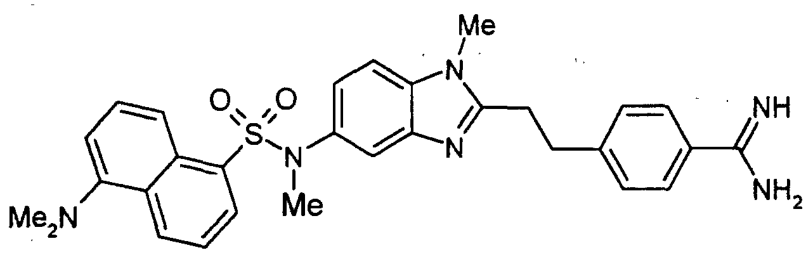

Ausbeute: 73%; Schmp.: 222-225 °C;
Masse: ber.: [540], gef.: $[M+H]^+$ 541.

**Beispiel 14:** N-{2-[2-(4-Amidinophenyl)-ethyl]-1-methyl-benzimidazol-5-yl}-N-diethylaminoethyl-[4-carboxy-benzolsulfonamid]-fumarat

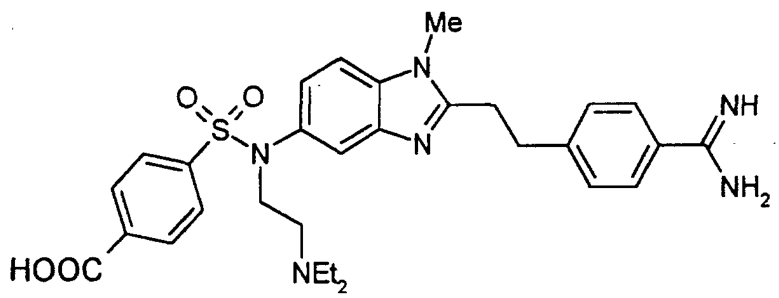

Schmp.: 200-204 °C;
Masse: ber.: [576], gef.: $[M+H]^+$ 577;
$^1$H-NMR (250 MHz, DMSO-d6): $\delta$=11.32; 9.36 (6H, 2s, $H^+$; -C(=$NH_2^+$)$NH_2$); 8.36-7.09 (11H, m, aryl-H); 6.77 (2H, s, Fumarsäure); 3.92; 2.74 (4H, 2m, N-$CH_2CH_2$N); 3.89 (3H, s, N-$CH_3$); 3.44 (4H, s, -$CH_2$-$CH_2$-); 2.76 (4H, m, N-$(CH_2CH_3)_2$; 1.07 (6H, t, J=7.0 Hz, N-$(CH_2CH_3)_2$).

**Beispiel 15:** N-{2-[2-(4-Amidinophenyl)-ethyl]-1-methyl-benzimidazol-5-yl}-N-diethylaminoethyl-[4-carboxymethylaminocarbonyl-benzolsulfonamid]

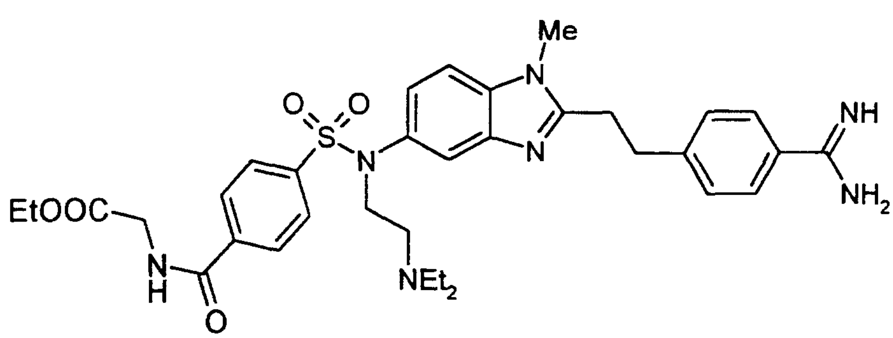

Schmp.: 205-210 °C;
Masse: ber.: [633], gef.: $[M+H]^+$ 634

$^1$H-NMR (250 MHz, $CD_3OD$): δ=8.04-6.94 (11H, m, aryl-H); 2H, s, $N-CH_2$; 3.88; 2.62 (4H, 2s, $N-CH_2CH_3-N$); 3.70 (3H, s, $N-CH_3$); 3.30 (4H, s, $-CH_2CH_2-$); 2.53 (4H, qu, J=7.0 Hz, $N-(CH_2CH_3)_2$); 0.95 (6H, t, J=7.0 Hz, $N-(CH_2CH_3)_2$).

- Die nachfolgende Tabelle faßt in Analogie zu den vorstehend beschriebenen Beispielen weitere, erfindungsgemäß synthetisierte Verbindungen der allgemeinen Formel (I) zusammen.

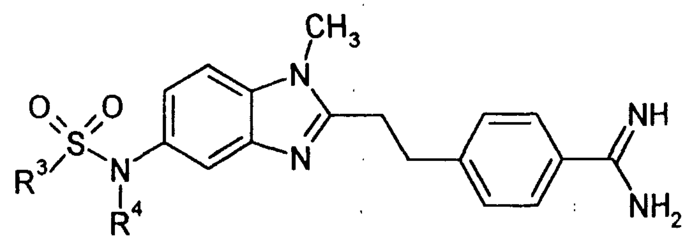 (I),

Tabelle 1:

| Nr | -$R^3$ | -$R^4$ | Chemische Bezeichnung |
|---|---|---|---|
| 16 | 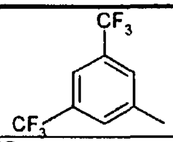 | 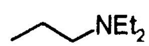 | N-{2-[2-(4-Amidinophenyl)-ethyl]-1-methyl-benzimidazol-5-yl}-N-2-diethylaminoethyl-[3,5-di-(trifluormethyl)-benzolsulfonamid] dihydrochlorid |
| 17 | 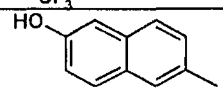 | 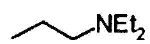 | N-{2-[2-(4-Amidinophenyl)-ethyl]-1-methyl-benzimidazol-5-yl}-N-2-diethylaminoethyl-(6-hydroxy-naphth-2-ylsulfonamid) dihydrochlorid |
| 18 | 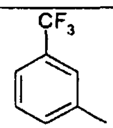 | 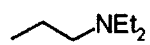 | N-{2-[2-(4-Amidinophenyl)-ethyl]-1-methyl-benzimidazol-5-yl}-N-2-diethylaminoethyl-(3-trifluormethyl-benzolsulfonamid) dihydrochlorid |
| 19 | 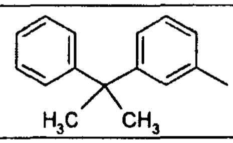 | 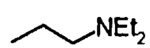 | N-{2-[2-(4-Amidinophenyl)-ethyl]-1-methyl-benzimidazol-5-yl}-N-2-diethylaminoethyl-[3-(1-methyl-1-phenyl-ethyl)-benzolsulfonamid] dihydrochlorid |

| Nr | -R3 | -R4 | Chemische Bezeichnung |
|---|---|---|---|
| 20 | 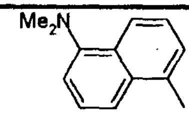 | 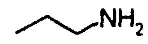 | N-{2-[2-(4-Amidinophenyl)-ethyl]-1-methyl-benzimidazol-5-yl}-N-aminoethyl-(dansylamid) dihydrochlorid |
| 21 | 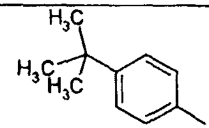 | 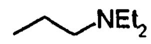 | N-{2-[2-(4-Amidinophenyl)-ethyl]-1-methyl-benzimidazol-5-yl}-N-diethylaminoethyl-(4-tert-butyl-benzolsulfonamid) dihydrochlorid |
| 22 | 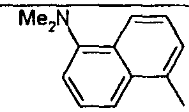 | 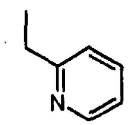 | N-{2-[2-(4-Amidinophenyl)-ethyl]-1-methyl-benzimidazol-5-yl}-N-pyrid-2-ylmethyl-(dansylamid) dihydrochlorid |
| 23 | 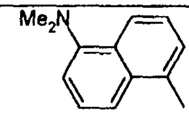 | 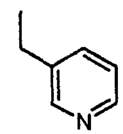 | N-{2-[2-(4-Amidinophenyl)-ethyl]-1-methyl-benzimidazol-5-yl}-N-pyrid-3-ylmethyl-(dansylamid) dihydrochlorid |
| 24 | 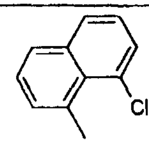 | 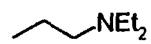 | N-{2-[2-(4-Amidinophenyl)-ethyl]-1-methyl-benzimidazol-5-yl}-N-diethylaminoethyl-(8-chlor-naphth-1-ylsulfonamid) dihydrochlorid |
| 25 | 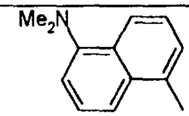 | 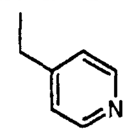 | N-{2-[2-(4-Amidinophenyl)-ethyl]-1-methyl-benzimidazol-5-yl}-N-pyrid-4-ylmethyl-(dansylamid) dihydrochlorid |
| 26 | 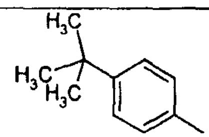 | 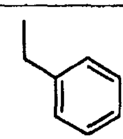 | N-{2-[2-(4-Amidinophenyl)-ethyl]-1-methyl-benzimidazol-5-yl}-N-benzyl-(4-tert-butyl-benzolsulfonamid) dihydrochlorid |
| 27 | 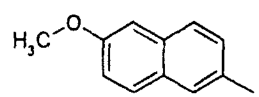 | 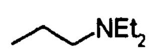 | N-{2-[2-(4-Amidinophenyl)-ethyl]-1-methyl-benzimidazol-5-yl}-N-diethylaminoethyl-(6-methoxy-naphth-2-ylsulfonamid) diacetat |

| Nr | -R3 | -R4 | Chemische Bezeichnung |
|---|---|---|---|
| 28 | 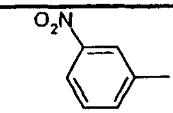 | 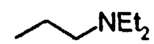 | N-{2-[2-(4-Amidinophenyl)-ethyl]-1-methyl-benzimidazol-5-yl}-N-2-diethylaminoethyl-(3-nitro-benzolsulfonamid) dihydrochlorid |
| 29 | 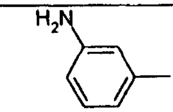 | 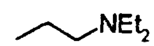 | N-{2-[2-(4-Amidinophenyl)-ethyl]-1-methyl-benzimidazol-5-yl}-N-2-diethylaminoethyl-(3-amino-benzolsulfonamid) dihydrochlorid |
| 30 | 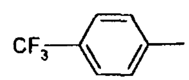 | 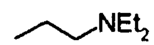 | N-{2-[2-(4-Amidinophenyl)-ethyl]-1-methyl-benzimidazol-5-yl}-N-2-diethylaminoethyl-(4-trifluoromethyl-benzolsulfonamid) dihydrochlorid |
| 31 | 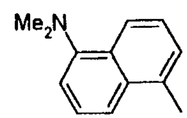 | 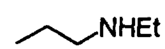 | N-{2-[2-(4-Amidinophenyl)-ethyl]-1-methyl-benzimidazol-5-yl}-N-2-ethylaminoethyl-(dansylamid) dihydrochlorid |
| 32 | 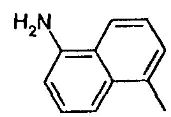 | 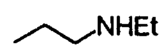 | N-{2-[2-(4-Amidinophenyl)-ethyl]-1-methyl-benzimidazol-5-yl}-N-2-ethylaminoethyl-(5-amino-naphth-1-ylsulfonamid) dihydrochlorid |
| 33 | 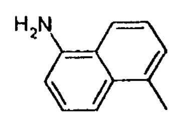 | 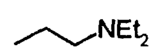 | N-{2-[2-(4-Amidinophenyl)-ethyl]-1-methyl-benzimidazol-5-yl}-N-2-diethylaminoethyl-(5-amino-naphth-1-ylsulfonamid) dihydrochlorid |
| 34 | 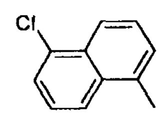 | 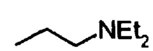 | N-{2-[2-(4-Amidinophenyl)-ethyl]-1-methyl-benzimidazol-5-yl}-N-2-diethylaminoethyl-(5-chlor-naphth-1-ylsulfonamid) dihydrochlorid |

**Beispiel 35:** N-{2-[2-(4-Amino-hydroximinomethyl)phenyl)-ethyl]-1-methylbenzimidazol-5-yl}-N-diethylaminoethyl-(dansylamid)

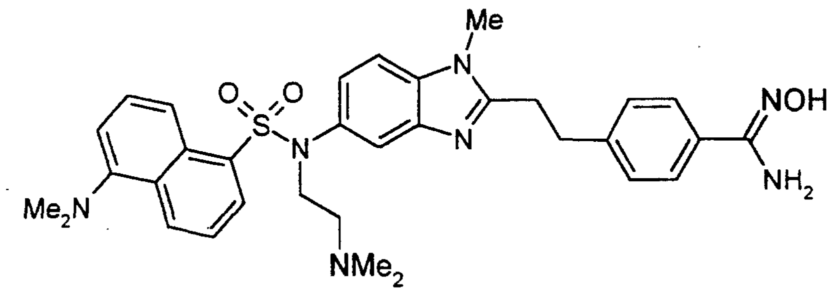

2,3 mmol N-{2-[2-(4-Cyanophenyl)-ethyl]-1-methyl-benzimidazol-5-yl}-N-diethylaminoethyl-(dansylamid), welches in Analogie zu Beispiel 1 (Stufen a-f) erhalten werden kann, $NH_2OH$*HCl (1.0 g) und $Na_2CO_3$ (1.7 g) in 40 mL Methanol/Tetrahydrofuran werden 2 h unter Rückfluß erhitzt. Das Lösemittel wird abdestilliert, der Rückstand in Wasser aufgenommen und mit Ethylacetat extrahiert. Die Reinigung erfolgt durch Chromatographie an Kieselgel.
Ausbeute: 47%;
Masse: ber.: [641], gef.: $[M+H]^+$ 642, $[M+2H]^{2+}$ 322
$^1$H-NMR (250 MHz,DMSO-d6):δ=9.66 (1H,s,OH); 8.62-6.96 (13H,m,aryl-H); 5.83 (2H,s,$NH_2$); 3.77; 2.40 (4H,2m, N-$CH_2CH_2$-N); 3.69 (3H,s,N-$CH_3$); 3.15 (4H,s,-$CH_2$-$CH_2$-); 2.87 (6H,s,N-$(CH_3)_2$); 2.36 (4H,m,N-($\underline{CH_2}CH_3)_2$); 0.78 (6H, m,N-($CH_2$-$\underline{CH_2})_2$).

**Beispiel 36:** N-{2-[2-(4-Amino-hydroximinomethyl)phenyl-ethyl]-1-methylbenzimidazol-5-yl}-N-diethylaminoethyl-(3-brom-benzolsulfonamid)

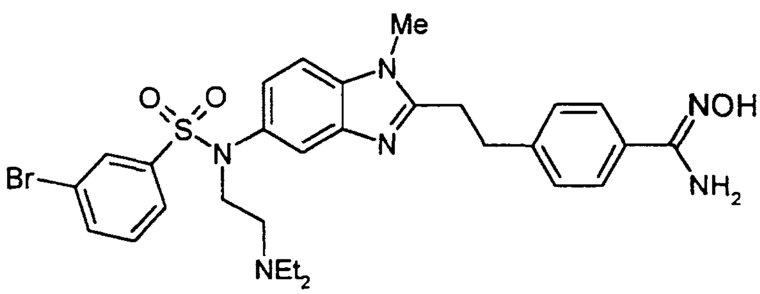

Die Herstellung der Titelverbindung gelingt ausgehend von N-{2-[2-(4-Cyanophenyl)-ethyl]-1-methyl-benzimidazol-5-yl}-N-diethylaminoethyl-(3-brom-benzol-sulfonamid) in Analogie zu der unter Beispiel 16 beschriebenen Vorgehensweise.
Schmp: 156 °C;
Masse ber.: [627], gef.: $[M+H]^+$ 627/629, $[M+2H]^{2+}$ 315.

**Beispiel 37:** N-{2-[2-(4-(Amino-benzoylimino-methyl)-phenyl)-ethyl]-1-methylbenzimidazol-5-yl}-N-diethylaminoethyl-(dansylamid)

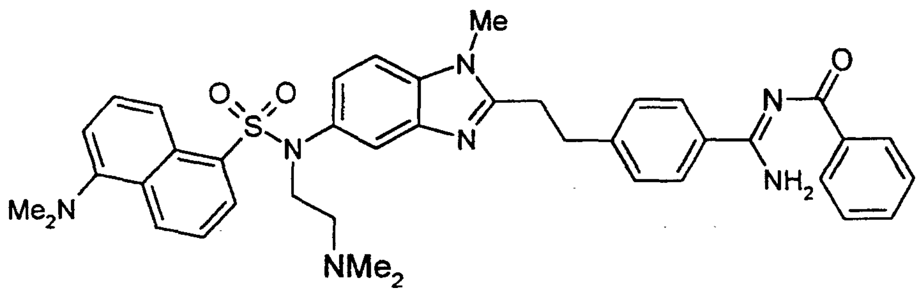

N-{2-[2-(4-Amidinophenyl)-ethyl]-1-methyl-benzimidazol-5-yl}-N-diethylaminoethyl-(dansylamid)-dihydrochlorid (Beispiel 2, 3.0 mmol) und TEA (48 mmol) in 100 mL $CH_2Cl_2$ werden bei Raumtemperatur unter Rühren mit Benzoylchlorid (3.3 mmol) versetzt. Es wird bis zum vollständigen Umsatz bei Raumtemperatur gerührt, mit 50 mL $CH_2Cl_2$ verdünnt, mit Wasser gewaschen, getrocknet und eingeengt. Das Produkt wird durch Kristallisation aus *t*-Butyl-methyl-ether gereinigt.
Masse: ber.: [729], gef.: $[M+H]^+$ 730, $[M+2H]^{2+}$ 365

**Beispiel 38:** N-{2-[2-(4-(Amino-nicotinoylimino-methyl)-phenyl)-ethyl]-1-methylbenzimidazol-5-yl}-N-diethylaminoethyl-(dansylamid)

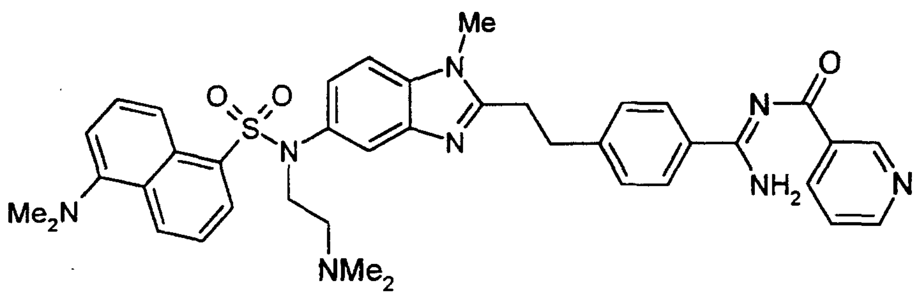

Ausgehend von N-{2-[2-(4-Amidinophenyl)-ethyl]-1-methyl-benzimidazol-5-yl}-N-diethylaminoethyl-(dansylamid)-dihydrochlorid (Beispiel 2) gelingt die Synthese der Zielverbindung in Analogie zu der für Beispiel 18 beschriebenen Vorgehensweise unter Verwendung von Pyridin-3-carbonsäurechlorid.
Masse: ber.: [730], gef.: $[M+H]^+$ 731, $[M+2H]^{2+}$ 366.

**Beispiel 39:** N-{2-[2-(4-(Amino-methyloxycarbonylimino-methyl)-phenyl)-ethyl]-1-methyl-benzimidazol-5-yl}-N-diethylaminoethyl-(dansylamid)

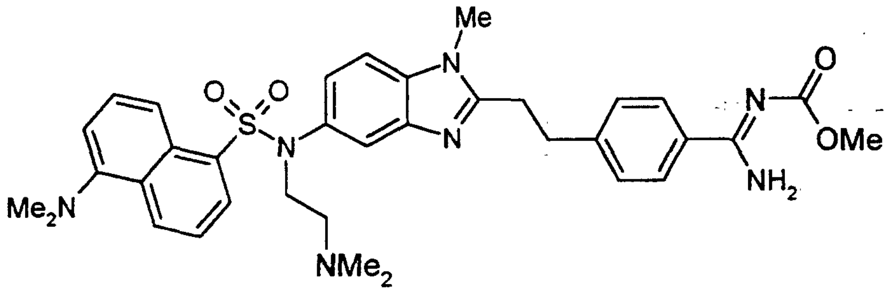

Ausgehend von N-{2-[2-(4-Amidinophenyl)-ethyl]-1-methyl-benzimidazol-5-yl}-N-diethylaminoethyl-(dansylamid)-dihydrochlorid (Beispiel 2) gelingt die Synthese der Zielverbindung in Analogie zu der für Beispiel 18 beschriebenen Vorgehensweise unter Verwendung von Chlorameisensäure-methyl-ester.
Masse: ber.: [683], gef.: $[M+H]^+$ 684, $[M+2H]^{2+}$ 342.

**Beispiel 40:** N-{2-[2-(4-(Amino-ethyloxycarbonylimino-methyl)-phenyl)-ethyl]-1-methyl-benzimidazol-5-yl}-N-diethylaminoethyl-(dansylamid)

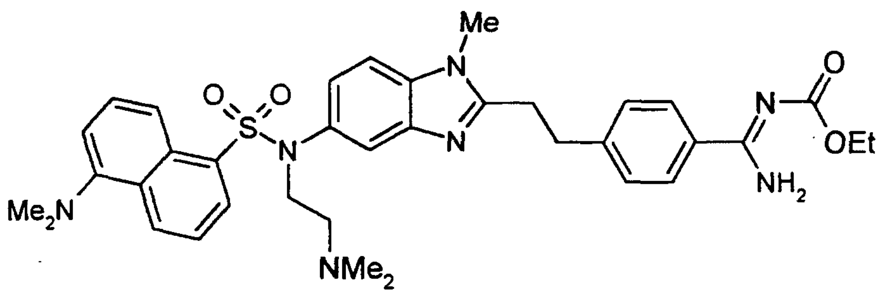

1.9 mmol N-{2-[2-(4-Amidinophenyl)-ethyl]-1-methyl-benzimidazol-5-yl}-N-diethylaminoethyl-(dansylamid) (Beispiel 2) in 20 mL Dimethylformamid werden bei Raumtemperatur unter Rühren mit Chlorameisensäureethylester (0.9 mmol) versetzt. Es wird 1 h bei Raumtemperatur gerührt, mit 75 mL Ethylacetat verdünnt, mit verdünnter NaOH-Lösung und mit Wasser gewaschen, getrocknet und eingeengt. Das Produkt wird durch Chromatographie über Kieselgel gereinigt und gegebenenfalls aus Ethylacetat/Diethyether kristallisiert.
Ausbeute. 0,6 g (93%); Schmp.: 136-139 °C;
Masse: ber.: [697], gef.: $[M+H]^+$ 698, $[M+2H]^{2+}$ 349.

**Beispiel 41:** N-{2-[2-(4-(Amino-propyloxycarbonylimino-methyl)-phenyl)-ethyl]-1-methyl-benzimidazol-5-yl}-N-diethylaminoethyl-(dansylamid)

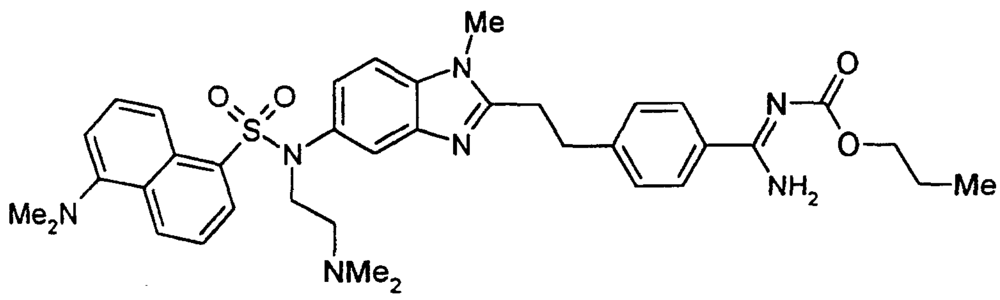

Masse:ber.: [711], gef.: $[M+H]^+$ 712, $[M+2H]^{2+}$ 357.

**Beispiel 42:** N-{2-[2-(4-(Amino-iso-butyloxycarbonylimino-methyl)-phenyl)-ethyl]-1-methyl-benzimidazol-5-yl}-N-diethylaminoethyl-(dansylamid)

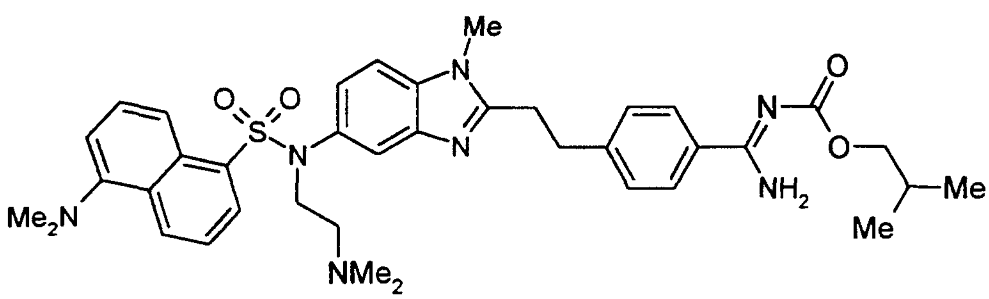

Masse: ber.: [725], gef.: $[M+H]^+$ 726, $[M+2H]^{2+}$ 364.

**Beispiel 43:** N-{2-[2-(4-Amino-hydroximinomethyl)-phenyl)-ethyl]-1-methylbenzimidazol-5-yl}-N-diethylaminoethyl-(6-hydroxy-naphth-2-ylsulfonamid)

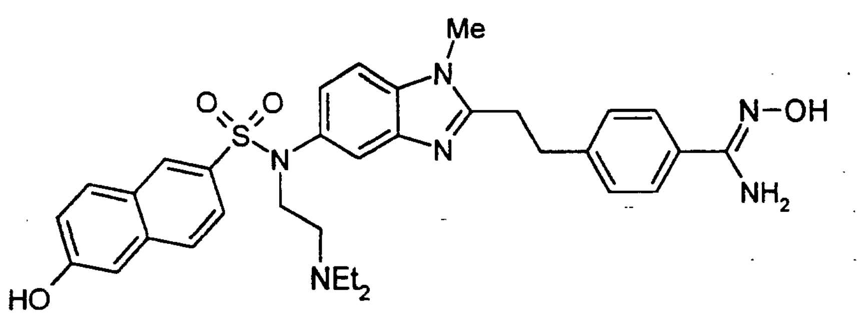

**Beispiel 44:** N-{2-[2-(4-(Amino-iso-butyloxycarbonylimino-methyl)-phenyl)-ethyl]-1-methyl-benzimidazol-5-yl}-N-diethylaminoethyl-(8-chlornaphth-1-ylsulfonamid)

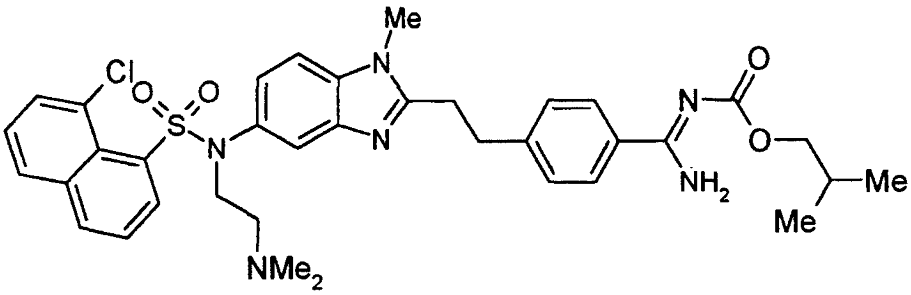

**Beispiel 45:** N-{2-[2-(4-(Amino-iso-butyloxycarbonylimino-methyl)-phenyl)-ethyl]-1-methyl-benzimidazol-5-yl}-N-diethylaminoethyl-(3,5-di-(trifluormethyl)-benzolsulfonamid)

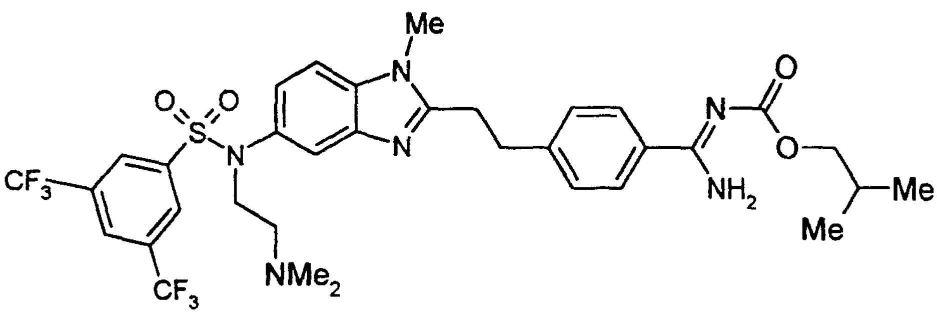

Die erfindungsgemäßen Verbindungen zeichnen sich durch ihre Tryptase-inhibierende Wirksamkeit aus. Besagte Fähigkeit, die Tryptase zu inhibieren, wurde gemäß der nachfolgenden Testbeschreibung untersucht.
Die Bestimmung wird in Tris HCl Puffer (100 mM), der zusätzlich Calcium (5 mM) und Heparin (100 mg/ml) enthält, bei pH 7.4 durchgeführt. Als Standard wird rh beta Tryptase eingesetzt, die beispielsweise von Promega käuflich zu erwerben ist. Als Substrat dient N-p-Tosyl-Gly-Pro-Lys-para-nitroanilin in einer Konzentration von 0.6 mM. Das Substrat wird durch Tryptase verdaut wobei p-Nitroanilin entsteht, das bei 405 nm gemessen werden kann. Üblicherweise wird eine Inkubationszeit von 5 Minuten und eine Inkubationstemperatur von 37°C gewählt. Als Enzymaktivität werden 0.91 U/ml eingesetzt. Die Bestimmung erfolgt in einem Autoanalyser (Cobas Bio) der Firma Hofmann LaRoche. Die potentiellen Hemmsubstanzen werden in Konzentrationen von 10 µM im Screening eingesetzt, wobei die Hemmung der Tryptase in Prozent angegeben wird. Bei über 70 % Hemmung wird die $IC_{50}$ bestimmt (Konzentration bei der 50% der Enzymaktivität gehemmt ist). Nach 5-minütiger Vorinkubation der potentiellen Hemmsubstanzen, wird das Substrat zum Starten der Reaktion zugegeben, wobei die Bildung von p-Nitroanilin nach 5 Minuten, nach Testung der Linearität, als Maß für die Enzymaktivität genommen wird.

Die nach Durchführung des vorstehend beschriebenen Tests erhaltenen Daten (IC50-Werte) sind Tabelle 2 zu entnehmen.

Tabelle 2:

| Beispiel | $IC_{50}$ [µM] |
|---|---|
| 1 | 0,0097 |
| 2 | 0,011 |
| 3 | 0,017 |
| 4 | 0,023 |

Tabelle 2: (fortgesetzt)

| Beispiel | $IC_{50}$ [µM] |
|---|---|
| 5 | 0,024 |
| 6 | 0,03 |
| 7 | 0,03 |
| 8 | 0,034 |
| 9 | 0,044 |
| 10 | 0,049 |
| 16 | 0,025 |
| 17 | 0,280 |
| 18 | 0,043 |
| 19 | 0,0088 |
| 20 | 0,022 |
| 21 | 0,0041 |
| 22 | 0,014 |
| 23 | 0,013 |
| 24 | 0,013 |
| 25 | 0,036 |
| 26 | 0,0076 |
| 27 | 0,0173 |
| 28 | 0,044 |
| 29 | 0,052 |

Die erfindungsgemäßen Tryptase-Inhibitoren können oral, transdermal, inhalativ oder parenteral verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung

eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Maisstärke | 190 mg |
| | Milchzucker | 55 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragees | pro Dragée |
|---|---|---|
| | Wirkstoff | 5 mg |

(fortgesetzt)

| C) | Dragees | pro Dragée |
|---|---|---|
| | Maisstärke | 41,5 mg |
| | Milchzucker | 30 mg |
| | Polyvinylpyrrolidon | 3 mg |
| | Magnesiumstearat | 0,5 mg |
| | | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.

| D) | Kapseln | pro Kapsel |
|---|---|---|
| | Wirkstoff | 50 mg |
| | Maisstärke | 268,5 mg |
| | Magnesiumstearat | 1.5 mg |
| | | 320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| F) Suppositorien | |
|---|---|
| Wirkstoff | 50 mg |
| Adeps solidus | 1650 mg |
| | 1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

## Patentansprüche

1. Benzimidazolderivate der allgemeinen Formel (I)

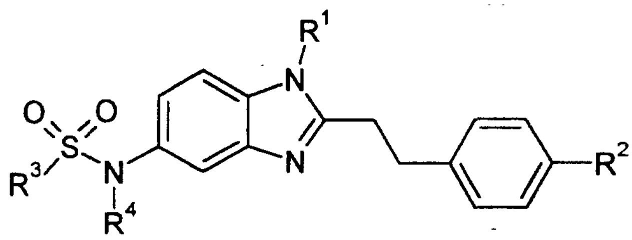

(I),

worin

$R^1$ ein Rest ausgewählt aus der Gruppe $C_1$-$C_6$-Alkyl, $C_2$-$C_6$-Alkenyl und $C_2$-$C_6$-Alkinyl, welcher gegebenenfalls ein-, zwei- oder dreifach durch einen oder mehrere der Reste Hydroxy, $C_1$-$C_4$-Alkoxy, $CF_3$, Phenoxy, COOH, Halogen, -CO($C_1$-$C_4$-alkoxy), -$CONH_2$, -CO-NH($C_1$-$C_4$-alkyl), -CO-N($C_1$-$C_4$-alkyl)$_2$, -$NH_2$, -NH($C_1$-$C_4$-alkyl), -N($C_1$-$C_4$-alkyl)$_2$ oder $C_1$-$C_4$-Alkoxy-phenoxy substituiert sein kann, oder
ein gegebenenfalls über eine $C_1$-$C_4$-Alkylenbrücke verknüpftes $C_3$-$C_8$-Cycloalkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
Phenyl-$C_1$-$C_4$-alkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls durch $C_1$-$C_4$-Alkyl oder Benzyl substituiert sein kann;

$R^2$ -C(=NH)$NH_2$ oder -$CH_2$-$NH_2$;

$R^3$ ein Rest ausgewählt aus der Gruppe Phenyl, Benzyl, Naphthyl, Chinolyl, Isochinolyl, Furan, Benzofuran, Thiophen und Benzothiophen der jeweils ein-, zwei- oder dreifach durch einen oder mehrere der Reste ausgewählt aus der Gruppe $C_1$-$C_4$-Alkyl, $C_1$-$C_4$-Alkoxy, Halogen, -$C_1$-$C_4$-alkyl-Halogen, -$NH_2$, -NH($C_1$-$C_4$-alkyl), -N($C_1$-$C_4$-alkyl)$_2$, $NO_2$, Hydroxy, -$CF_3$, -NHCO-$C_1$-$C_4$-alkyl, -COOH, -COO($C_1$-$C_4$-alkyl), -$CONH_2$, -CONH($C_1$-$C_4$-alkyl), -CON($C_1$-$C_4$-alkyl)$_2$,
-CONH($C_1$-$C_4$-alkyl)-COO($C_1$-$C_4$-alkyl) und Phenyl-$C_1$-$C_6$-alkyl substituiert ist, wobei der Substiuent Phenyl-$C_1$-$C_6$-alkyl seinerseits substituiert sein kann durech einen Rest ausgewählt aus der Gruppe $C_1$-$C_6$-Alkoxy, Hydroxy, Halogen, $CF_3$ und $C_1$-$C_6$-Alkyl;

$R^4$ Wasserstoff, ein $C_1$-$C_6$-Alkyl-Rest, der ein- oder zweifach durch einen oder zwei der Reste -$NH_2$, -NH($C_1$-$C_4$-alkyl), -N($C_1$-$C_4$-alkyl)$_2$, -C(=NH)$NH_2$ oder -NH-C(=NH)$NH_2$ substituiert sein kann, oder
ein direkt oder über eine $C_1$-$C_4$-Alkylen-Brücke oder über eine $C_1$-$C_4$-Alkylen-CO-Brücke verknüpfter 5-, 6- oder 7-gliedriger, gesättigter oder ungesättigter Heterocyclus, der gegebenenfalls ein, zwei oder drei Heteroatome ausgewählt aus der Gruppe Sauerstoff, Stickstoff oder Schwefel enthalten kann und der gegebenenfalls durch $C_1$-$C_4$-Alkyl, Benzyl oder Pyridyl substituiert sein kann;
$C_3$-$C_8$-Cycloalkyl, das ein- oder zweifach durch einen oder zwei der Reste -$NH_2$, -NH($C_1$-$C_4$-alkyl), -N($C_1$-$C_4$-alkyl)$_2$, -C(=NH)$NH_2$ oder -NH-C(=NH)$NH_2$ substituiert sein kann, oder
Phenyl, Benzyl oder Phenylethyl, die am Phenylring jeweils ein- oder zweifach durch einen oder zwei der Reste -$NH_2$, -NH($C_1$-$C_4$-alkyl), -N($C_1$-$C_4$-alkyl)$_2$, -$C_1$-$C_4$-Alkyl-$NH_2$, -C(=NH)$NH_2$ oder -NH-C(=NH)$NH_2$ substituiert sein können,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**2.** Verbindungen der allgemeinen Formel (I) gemäß Anspruch 1, worin

$R^1$ $C_1$-$C_6$-Alkyl, welches gegebenenfalls ein-, zwei- oder dreifach durch einen oder mehrere der Reste Hydroxy, $C_1$-$C_4$-Alkoxy, $CF_3$, Phenoxy, COOH, Halogen, -CO($C_1$-$C_4$-alkoxy), -$CONH_2$, -CO-NH($C_1$-$C_4$-alkyl), -CO-N($C_1$-$C_4$-alkyl)$_2$, -$NH_2$, -NH($C_1$-$C_4$-alkyl), -N($C_1$-$C_4$-alkyl)$_2$ oder $C_1$-$C_4$-Alkoxy-phenoxy substituiert sein kann, oder
ein gegebenenfalls über eine $C_1$-$C_4$-Alkylenbrücke verknüpftes $C_3$-$C_8$-Cycloalkyl, welches gegebenenfalls

ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
Phenyl-$C_1$-$C_4$-alkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls durch $C_1$-$C_4$-Alkyl oder Benzyl substituiert sein kann;

$R^2$ -C(=NH)$NH_2$ oder -$CH_2$-$NH_2$;

$R^3$ ein Rest ausgewählt aus der Gruppe Phenyl, Benzyl, Naphthyl, Chinolyl, Isochinolyl, Thienyl und Benzothienyl der jeweils ein-, zwei- oder dreifach durch einen oder mehrere der Reste ausgewählt aus der Gruppe $C_1$-$C_4$-Alkyl, $C_1$-$C_4$-Alkoxy, Halogen,
-$C_1$-$C_4$-alkyl-Halogen, -$NH_2$, -NH($C_1$-$C_4$-alkyl), -N($C_1$-$C_4$-alkyl)$_2$, $NO_2$, Hydroxy, -$CF_3$, -NHCO-$C_1$-$C_4$-alkyl, -COOH, -COO($C_1$-$C_4$-alkyl), -$CONH_2$, -CONH($C_1$-$C_4$-alkyl), -CON($C_1$-$C_4$-alkyl)$_2$,
-CONH($C_1$-$C_4$-alkyl)-COO($C_1$-$C_4$-alkyl) und Phenyl-$C_1$-$C_4$-alkyl substituiert ist, wobei der Substiuent Phenyl-$C_1$-$C_4$-alkyl seinerseits substituiert sein kann durech einen Rest ausgewählt aus der Gruppe $C_1$-$C_4$-Alkoxy, Hydroxy, Halogen, $CF_3$ und $C_1$-$C_4$-Alkyl;

$R^4$ Wasserstoff, ein $C_1$-$C_6$-Alkyl-Rest, der ein- oder zweifach durch einen oder zwei der Reste-$NH_2$, -NH($C_1$-$C_4$-alkyl), -N($C_1$-$C_4$-alkyl)$_2$, -C(=NH)$NH_2$ oder -NH-C(=NH)$NH_2$ substituiert sein kann, oder
ein direkt oder über eine $C_1$-$C_4$-Alkylen-Brücke oder über eine $C_1$-$C_4$-Alkylen-CO-Brücke verknüpfter 5-, 6- oder 7-gliedriger, gesättigter oder ungesättigter Heterocyclus, der gegebenenfalls ein, zwei oder drei Heteroatome ausgewählt aus der Gruppe Sauerstoff, Stickstoff oder Schwefel enthalten kann und der gegebenenfalls durch $C_1$-$C_4$-Alkyl, Benzyl oder Pyridyl substituiert sein kann; $C_3$-$C_8$-Cycloalkyl, das ein- oder zweifach durch einen oder zwei der Reste -$NH_2$, -NH($C_1$-$C_4$-alkyl), -N($C_1$-$C_4$-alkyl)$_2$, -C(=NH)$NH_2$ oder -NH-C (=NH)$NH_2$ substituiert sein kann, oder
Phenyl, Benzyl oder Phenylethyl, die am Phenylring jeweils ein- oder zweifach durch einen oder zwei der Reste -$NH_2$, -NH($C_1$-$C_4$-alkyl), -N($C_1$-$C_4$-alkyl)$_2$, -$C_1$-$C_4$-Alkyl-$NH_2$, -C(=NH)$NH_2$ oder -NH-C(=NH)$NH_2$ substituiert sein können,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**3.** Verbindungen der allgemeinen Formel (I) gemäß Anspruch 1 oder 2, worin

$R^1$ $C_1$-$C_5$-Alkyl, welches gegebenenfalls ein-, zwei- oder dreifach durch einen oder mehrere der Reste Hydroxy, $C_1$-$C_4$-Alkoxy, $CF_3$, Phenoxy, COOH, -CO($C_1$-$C_4$-alkoxy), -$CONH_2$, -CO-NH($C_1$-$C_4$-alkyl), -CO-N($C_1$-$C_4$-alkyl)$_2$ oder $C_1$-$C_4$-Alkoxy-phenoxy substituiert sein kann, oder
ein gegebenenfalls über eine $C_1$-$C_4$-Alkylenbrücke verknüpftes $C_3$-$C_8$-Cycloalkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder
Phenyl-$C_1$-$C_4$-alkyl, welches gegebenenfalls ein-, zwei- oder dreifach durch einen oder mehrere der Reste Hydroxy, $C_1$-$C_4$-Alkoxy, Carboxy, $C_1$-$C_4$-Alkoxycarbonyl oder $CF_3$ substituiert sein kann, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls durch $C_1$-$C_4$-Alkyl oder Benzyl substituiert sein kann;

$R^2$ -C(=NH)$NH_2$ oder -$CH_2$-$NH_2$;

$R^3$ ein Rest ausgewählt aus der Gruppe Phenyl, Benzyl, Benzothienyl, Chinolyl, Isochinolyl und Naphthyl, der jeweils ein-, zwei- oder dreifach durch einen oder mehrere der Reste ausgewählt aus der Gruppe $C_1$-$C_4$-Alkyl, $C_1$-$C_4$-Alkoxy, Halogen, $CF_3$, -$C_1$-$C_4$-alkyl-Halogen, -$NH_2$, -NH($C_1$-$C_4$-alkyl), -N($C_1$-$C_4$-alkyl)$_2$, $NO_2$, Hydroxy, -$CF_3$, -NHCO-$C_1$-$C_4$-alkyl, -COOH, -COO($C_1$-$C_4$-alkyl), -$CONH_2$, -CONH($C_1$-$C_4$-alkyl), -CON ($C_1$-$C_4$-alkyl)$_2$, -C($CH_3$)$_2$-Phenyl, wobei diese Phenylgruppe durch eine oder mehrere Halogenatome oder Hydroxygruppen substituiert sein kann, und -CONH($C_1$-$C_4$-alkyl)-COO($C_1$-$C_4$-alkyl) substituiert ist;

$R^4$ ein $C_1$-$C_6$-Alkyl-Rest, der ein- oder zweifach durch einen oder zwei der Reste -$NH_2$, -NHMethyl, -N(Methyl)$_2$,

-NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert ist, oder
ein direkt oder über eine $C_1$-$C_4$-Alkylen-Brücke oder über eine $C_1$-$C_4$-Alkylen-CO-Brücke verknüpfter 5-, 6- oder 7-gliedriger, gesättigter oder ungesättigter Stickstoff-Heterocyclus, der gegebenenfalls ein oder zwei weitere Heteroatome ausgewählt aus der Gruppe Sauerstoff, Stickstoff oder Schwefel enthalten kann und der gegebenenfalls durch $C_1$-$C_4$-Alkyl, Benzyl oder Pyridyl substituiert sein kann;
Cyclopropyl, Cyclopentyl oder Cyclohexyl, die jeweils ein- oder zweifach durch einen oder zwei der Reste -$NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind, oder
Phenyl, Benzyl oder Phenylethyl, die am Phenylring jeweils ein- oder zweifach durch einen oder zwei der Reste -$NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**4.** Verbindungen der allgemeinen Formel (I) gemäß einem der Ansprüche 1, 2 oder 3, worin

$R^1$ Methyl, Ethyl, Propyl, Butyl oder Pentyl, die jeweils durch einen der Reste Hydroxy, Methoxy, Ethoxy, Propoxy, $CF_3$, Phenoxy, COOH, $CONH_2$, CONHMe oder Methoxy-phenoxy substituiert sein können, oder
Benzyl, Phenylethyl oder Phenylpropyl, die am Phenylring jeweils ein- oder zweifach durch einen oder zwei der Reste Hydroxy, Methoxy, Ethoxy, Carboxy, Methoxycarbonyl, Ethoxycarbonyl oder $CF_3$ substituiert sein können, oder
ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff oder Stickstoff enthalten kann und der gegebenenfalls durch Methyl, Ethyl, Propyl oder Benzyl substituiert sein kann;

$R^2$ -C(=NH)$NH_2$ oder -$CH_2$-$NH_2$;

$R^3$ Phenyl, Benzyl oder Naphthyl, die jeweils ein- oder zweifach durch einen oder zwei Reste ausgewählt aus der Gruppe Methyl, Ethyl, Propyl, Butyl, Methoxy, Ethoxy, Propoxy, Butoxy, Fluor, Chlor, Brom, Iod, -$CF_3$, -$NH_2$, -NHMethyl, -NHEthyl, -NMethyl$_2$, -NEthyl$_2$, $NO_2$, -NHCO-Methyl, -NHCO-Ethyl, -COOH, -COOMethyl, -COOEthyl, -$CONH_2$, -CONHMethyl, -CONHEthyl, -CONMethyl$_2$, -CONEthyl$_2$, -CONH-$CH_2$-COOH, -CONH-$CH_2$-COOMethyl, -CONH-$CH_2$-COOEthyl, -CONH-$CH_2CH_2$-COOH, -CONH-$CH_2CH_2$-COOMethyl, -C$(CH_3)_2$-Phenyl, wobei diese Phenylgruppe durch eine oder mehrere Halogenatome oder Hydroxygruppen substituiert sein kann, und -CONH-$CH_2CH_2$-COOEthyl substituiert sind;

$R^4$ ein Rest ausgewählt aus der Gruppe Ethyl, Propyl, Butyl, Pentyl und Hexyl, der jeweils ein- oder zweifach durch einen oder zwei der Reste -$NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind, oder
ein direkt oder über eine $C_1$-$C_4$-Alkylen-Brücke verknüpfter 5-, 6- oder 7-gliedriger, gesättigter oder ungesättigter Stickstoff-Heterocyclus, der gegebenenfalls ein oder zwei weitere Heteroatome ausgewählt aus der Gruppe Sauerstoff oder Stickstoff enthalten kann und der gegebenenfalls durch Methyl, Ethyl, Propyl oder Benzyl substituiert sein kann, oder
Cyclopentyl oder Cyclohexyl, die jeweils ein- oder zweifach durch einen oder zwei der Reste -$NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind, oder
Benzyl oder Phenylethyl, die am Phenylring jeweils ein- oder zweifach durch einen oder zwei der Reste -$NH_2$, -NHMethyl, -N(Methyl)$_2$, -NHEthyl, -N(Ethyl)$_2$, -NH(n-Propyl), -N(n-Propyl)$_2$, -NH(iso-Propyl), -N(iso-Propyl)$_2$, -C(=NH)$NH_2$ oder -NH-C(=NH)$NH_2$ substituiert sind,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**5.** Verbindungen der allgemeinen Formel (I) gemäß einem der Ansprüche 1, 2, 3 oder 4, worin

$R^1$ Methyl, Ethyl, Propyl, Butyl oder Pentyl, bevorzugt Methyl;

$R^2$ -C(=NH)$NH_2$ oder -$CH_2$-$NH_2$, bevorzugt -C(=NH)$NH_2$;

$R^3$ Phenyl oder Naphthyl, die jeweils durch einen oder zwei Reste ausgewählt aus der Gruppe Methyl, Ethyl, *tert.*-Butyl, Methoxy, Ethoxy, Fluor, Chlor, Brom, $CF_3$, -$NH_2$, -NHMethyl, -NHEthyl, -$NMethyl_2$, -$NEthyl_2$, $NO_2$, -NHCO-Methyl, -NHCO-Ethyl, -COOH, -COOMethyl, -COOEthyl, -$CONH_2$, -CONHMethyl, -CONHEthyl, -$CONMethyl_2$, -$CONEthyl_2$, -CONH-$CH_2$-COOH, -CONH-$CH_2$-COOMethyl, -C$(CH_3)_2$-Phenyl, wobei diese Phenylgruppe durch eine oder mehrere Halogenatome oder Hydroxygruppen substituiert sein kann, und -CONH-$CH_2$-COOEthyl,
substituiert sind;

$R^4$ Ethyl oder Propyl, die jeweils durch einen der Reste -$NH_2$, -N$(Methyl)_2$, -N$(Ethyl)_2$, -N$(n\text{-}Propyl)_2$ oder -C(=NH)$NH_2$ substituiert sind, oder
ein direkt oder über eine Methylen- oder Ethylen-Brücke verknüpfter 5-, 6-oder 7-gliedriger, gesättigter oder ungesättigter Stickstoff-Heterocyclus, der ein Sauerstoff als weiteres Heteroatom enthalten kann und der gegebenenfalls durch Methyl, Ethyl, Propyl oder Benzyl substituiert sein kann, oder
Cyclopentyl oder Cyclohexyl, die jeweils durch einen der Reste -$NH_2$, -N$(Methyl)_2$, -N$(Ethyl)_2$, -N$(n\text{-}Propyl)_2$ oder -C(=NH)$NH_2$ substituiert sind, oder
Benzyl, das am Phenylring durch einen der Reste -$NH_2$, -N$(Methyl)_2$, -N$(Ethyl)_2$, -N$(n\text{-}Propyl)_2$ oder -C(=NH)$NH_2$ substituiert ist,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**6.** Verbindungen der allgemeinen Formel (I) gemäß einem der Ansprüche 1 bis 5, worin

$R^1$ Methyl, Ethyl, Propyl, Butyl oder Pentyl, bevorzugt Methyl;

$R^2$ -C(=NH)$NH_2$ oder -$CH_2$-$NH_2$, bevorzugt -C(=NH)$NH_2$;

$R^3$ Phenyl das durch einen oder zwei Reste ausgewählt aus der Gruppe Methyl, Ethyl, *tert*-Butyl, Methoxy, Ethoxy, Fluor, Chlor, Brom, $CF_3$, und/oder einen Rest ausgewählt aus -$NH_2$, -NHMethyl, -NHEthyl, -$NMethyl_2$, -$NEthyl_2$, $NO_2$, -NHCO-Methyl, -NHCO-Ethyl, -COOH, -COOMethyl, -COOEthyl, -$CONH_2$, -CONHMethyl, -CONHEthyl, -$CONMethyl_2$, -$CONEthyl_2$, -CONH-$CH_2$-COOH, -CONH-$CH_2$-COOMethyl, -C$(CH_3)_2$-Phenyl, wobei diese Phenylgruppe durch eine oder mehrere Halogenatome oder Hydroxygruppen substituiert sein kann, und -CONH-$CH_2$-COOEthyl, substituiert ist, oder Naphthyl, das durch -$NMethyl_2$ oder Chlor substituiert ist;

$R^4$ Dimethylaminoethyl, Diethylaminoethyl, Dimethylaminopropyl oder Diethylaminopropyl bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**7.** Verbindungen der allgemeinen Formel (I) gemäß einem der Ansprüche 1 bis 6, worin

$R^1$ Methyl, Ethyl oder Propyl, bevorzugt Methyl;

$R^2$ -C(=NH)$NH_2$ oder -$CH_2$-$NH_2$, bevorzugt -C(=NH)$NH_2$;

$R^3$ Phenyl das durch einen oder zwei Reste ausgewählt aus der Gruppe Methyl, Ethyl, *tert*-Butyl, Methoxy, Ethoxy, Fluor, Chlor, Brom, $CF_3$, und/oder einen Rest aus der Gruppe -$NH_2$, -NHMethyl, -NHEthyl, -$NMethyl_2$, -$NEthyl_2$, $NO_2$, -NHCO-Methyl, -NHCO-Ethyl, -COOH, -COOMethyl, -COOEthyl, -$CONH_2$, -CONHMethyl, -CONHEthyl, -$CONMethyl_2$, -$CONEthyl_2$, -CONH-$CH_2$-COOH, -CONH-$CH_2$-COOMethyl, -C$(CH_3)_2$-Pheny, wobei diese Phenylgruppe durch eine oder mehrere Halogenatome oder Hydroxygruppen substituiert sein kann,l und -CONH-$CH_2$-COOEthyl,
substituiert ist, oder
Naphthyl, das durch -$NMethyl_2$ oder Chlor substituiert ist;

$R^4$ Dimethylaminoethyl oder Diethylaminoethyl bedeuten können, gegebenenfalls in Form ihrer Tautomeren, ihrer Racemate, ihrer Enantiomere, ihrer Diastereomere und ihrer Gemische, sowie gegebenenfalls ihrer

pharmakologisch unbedenklichen Säureadditionssalze.

**8.** Verbindungen der allgemeinen Formel (I) gemäß einem der Ansprüche 1 bis 7, worin

$R^1$ Methyl;

$R^2$ $-C(=NH)NH_2$ oder $-CH_2-NH_2$, bevorzugt $-C(=NH)NH_2$;

$R^3$ Phenyl das durch einen oder zwei Reste ausgewählt aus der Gruppe Methyl, *tert*-Butyl, Methoxy, Chlor, Brom, $CF_3$, und/oder einen Rest aus der Gruppe $-NH_2$, $NO_2$, -NHCO-Methyl, -COOEthyl, $-CONH-CH_2-COOH$, $-C(CH_3)_2$-Phenyl, wobei diese Phenylgruppe durch eine oder mehrere Halogenatome oder Hydroxygruppen substituiert sein kann, und $-CONH-CH_2$-COOEthyl, substituiert ist, oder Naphthyl, das durch $-NMethyl_2$ oder Chlor substituiert ist;

$R^4$ Diethylaminoethyl,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**9.** Prodrugs der allgemeinen Formel (II)

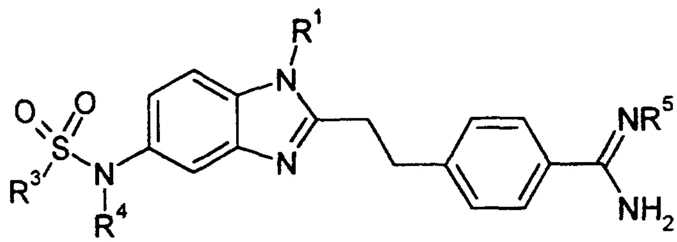
(II),

worin
$R^1$ und $R^4$ die gemäß den Ansprüchen 1-8 genannten Bedeutungen aufweisen können und

$R^3$ die in den Ansprüchen 1-8 genannten Bedeutungen aufweisen kann oder $C_1$-$C_4$-Alkyl bedeutet, welches durch einen Rest ausgewählt aus der Gruppe $-C(=NOH)NH_2$, $-C(=NCOO-C_1-C_{12}\text{-alkyl})NH_2$ oder $-C(=NCOO-C_1-C_8\text{-alkyl-Phenyl})NH_2$ substituiert ist;

$R^5$ Hydroxy, $-COO-C_1-C_{12}$-Alkyl, -CO-Phenyl, -CO-Pyridyl oder $-COO-C_1-C_8$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**10.** Prodrugs der allgemeinen Formel (II) gemäß Anspruch 9, worin

$R^3$ die in den Ansprüchen 1-8 genannten Bedeutungen aufweisen kann oder $C_1$-$C_4$-Alkyl bedeutet, welches durch einen Rest ausgewählt aus der Gruppe $-C(=NOH)NH_2$, $-C(=NCOO-C_1-C_6\text{-alkyl})NH_2$ oder $-C(=NCOO-C_1-C_6\text{-alkyl-Phenyl})NH_2$ substituiert ist;

$R^5$ Hydroxy, $-COO-C_1-C_6$-Alkyl, -CO-Phenyl, -CO-Pyridyl oder $-COO-C_1-C_6$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**11.** Prodrugs der allgemeinen Formel (II) gemäß Anspruch 9 oder 10, worin

$R^5$ Hydroxy, Methoxycarbonyl, Ethoxycarbonyl, Propyloxycarbonyl, Butyloxycarbonyl, Benzoyl, Benzyloxycarbonyl oder Nicotinoyl bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**12.** Verbindungen der allgemeinen Formel (III)

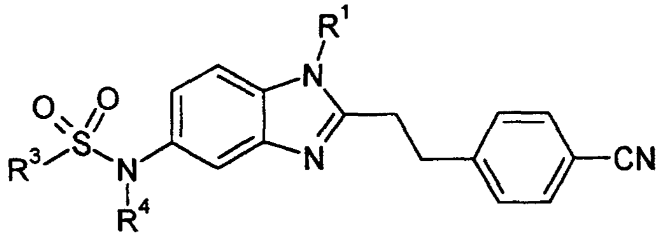

(III)

worin die Reste $R^1$, $R^3$ und $R^4$ die in den Ansprüchen 1-8 genannten Bedeutungen haben können.

**13.** Verwendung von Verbindungen der allgemeinen Formel (I) gemäß einem der Ansprüche 1-8 zur Herstellung eines Arzneimittels zur Vorbeugung und/oder Behandlung von Krankheiten, in denen Tryptase-Inhibitoren einen therapeutischen Nutzen entfalten können.

**14.** Verwendung von Prodrugs der allgemeinen Formel (II) gemäß einem der Ansprüche 9 bis 11 zur Herstellung eines Arzneimittels zur Vorbeugung und/oder Behandlung von Krankheiten, in denen Tryptase-Inhibitoren einen therapeutischen Nutzen entfalten können.

**15.** Pharmazeutische Zusammensetzung **gekennzeichnet durch** einen Gehalt einer oder mehrer Verbindungen gemäß einem der Ansprüche 1-11.

**16.** Verfahren zur Herstellung von Verbindungen der allgemeinen Formel (I)

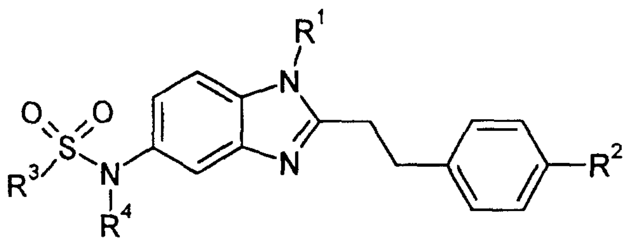

(I),

worin die Reste $R^1$, $R^2$, $R^3$ und $R^4$ die in den Ansprüchen 1-8 genannten Bedeutungen haben können, **dadurch gekennzeichnet, daß** eine Verbindung der allgemeinen Formel (III)

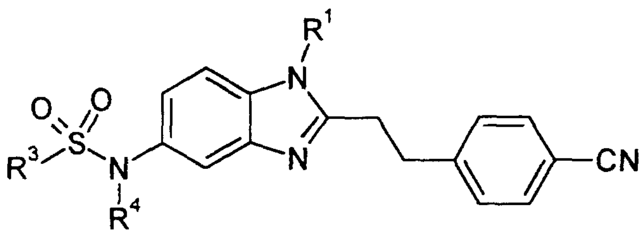

(III)

in der die Reste $R^1$, $R^3$ und $R^4$ die in den Ansprüchen 1-8 genannten Bedeutungen haben können, direkt in die Verbindungen der allgemeinen Formel (I) überführt wird.

**17.** Verfahren zur Herstellung von Verbindungen der allgemeinen Formel (I)

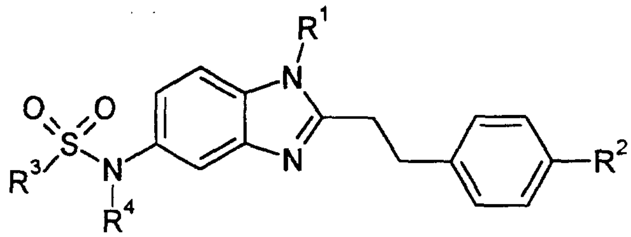

(I),

worin die Reste $R^1$, $R^2$, $R^3$ und $R^4$ die in den Ansprüchen 1-8 genannten Bedeutungen haben können, **dadurch gekennzeichnet, daß** eine Verbindung der allgemeinen Formel (II)

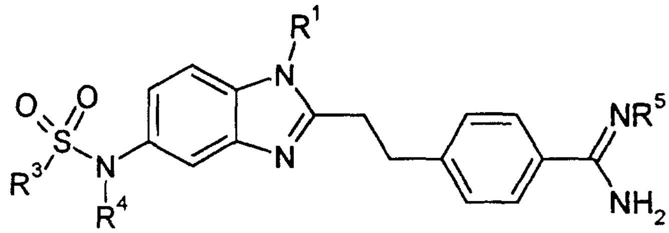

(II).

worin die Reste $R^1$, $R^3$ und $R^4$ die in den Ansprüchen 1-8 genannten Bedeutungen aufweisen können und der Rest $R^5$ die in den Ansprüchen 9-11 genannten Bedeutungen aufweisen kann, in die Verbindungen der allgemeinen Formel (I) überführt wird.

**18.** Verfahren zur Herstellung von Verbindungen der allgemeinen Formel (II)

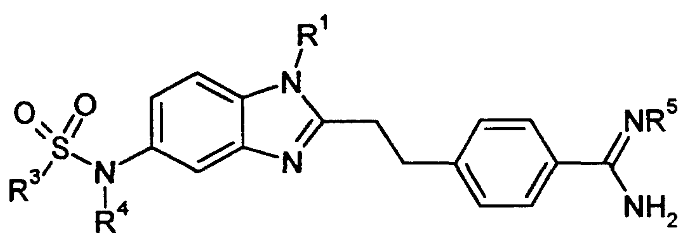

(II),

worin die Reste $R^1$, $R^3$ und $R^4$ die in den Ansprüchen 1-8 genannten Bedeutungen aufweisen können und der Rest $R^5$ die in den Ansprüchen 9-11 genannten Bedeutungen aufweisen kann, **dadurch gekennzeichnet, daß** eine Verbindung der allgemeinen Formel (III)

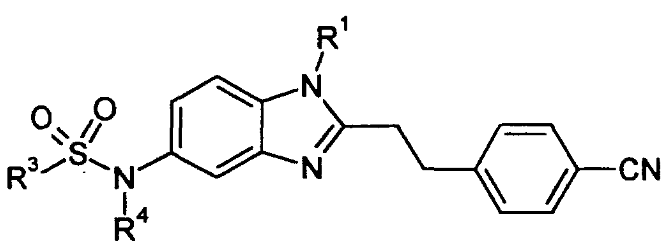

(III)

in der die Reste $R^1$, $R^3$ und $R^4$ die in den Ansprüchen 1-8 genannten Bedeutungen haben können, in eine Verbindung der allgemeinen Formel (II) überführt wird.

## Claims

**1.** Benzimidazole derivatives of general formula (I)

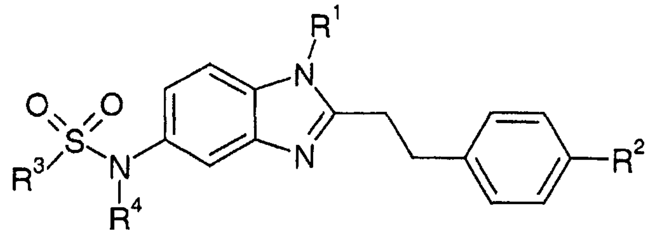

(I),

wherein

$R^1$ denotes a group selected from among $C_1$-$C_6$-alkyl, $C_2$-$C_6$-alkenyl and $C_2$-$C_6$-alkynyl, which may optionally be mono-, di- or trisubstituted by one or more of the groups hydroxy, $C_1$-$C_4$-alkoxy, $CF_3$, phenoxy, COOH, halogen, -CO($C_1$-$C_4$-alkoxy), -$CONH_2$, -CO-NH($C_1$-$C_4$-alkyl), -CO-N($C_1$-$C_4$-alkyl)$_2$, -$NH_2$, -NH($C_1$-$C_4$-alkyl), -N($C_1$-$C_4$-alkyl)$_2$ or $C_1$-$C_4$-alkoxy-phenoxy, or
a $C_3$-$C_8$-cycloalkyl optionally linked via a $C_1$-$C_4$-alkylene bridge, which may optionally be mono-, di- or trisubstituted by one or more of the groups hydroxy, $C_1$-$C_4$-alkoxy, carboxy, halogen, $C_1$-$C_4$-alkoxycarbonyl or $CF_3$, or
phenyl-$C_1$-$C_4$-alkyl, which may optionally be mono-, di- or trisubstituted by one or more of the groups hydroxy, $C_1$-$C_4$-alkoxy, carboxy, halogen, $C_1$-$C_4$-alkoxycarbonyl or $CF_3$, or
a 5- or 6-membered, saturated or unsaturated heterocyclic group linked directly or via a $C_1$-$C_4$-alkylene bridge, which may contain one or two hetero atoms selected from among oxygen, nitrogen or sulphur and which may optionally be substituted by $C_1$-$C_4$-alkyl or benzyl;

$R^2$ denotes -C(=NH)$NH_2$ or -$CH_2$-$NH_2$;

$R^3$ denotes a group selected from among phenyl, benzyl, naphthyl, quinolyl, isoquinolyl, furan, benzofuran, thiophene and benzothiophene which is mono-, di- or trisubstituted in each case by one or more of the groups selected from among $C_1$-$C_4$-alkyl, $C_1$-$C_4$-alkoxy, halogen, -$C_1$-$C_4$-alkyl-halogen, -$NH_2$, -NH($C_1$-$C_4$-alkyl), -N ($C_1$-$C_4$-alkyl)$_2$, $NO_2$, hydroxy, -$CF_3$, -NHCO-$C_1$-$C_4$-alkyl, -COOH, -COO($C_1$-$C_4$-alkyl), -$CONH_2$, -CONH ($C_1$-$C_4$-alkyl), -CON($C_1$-$C_4$-alkyl)$_2$, -CONH($C_1$-$C_4$-alkyl)-COO($C_1$-$C_4$-alkyl) and phenyl-$C_1$-$C_6$-alkyl, whilst the phenyl-$C_1$-$C_6$-alkyl substituent may in turn be substituted by a group selected from among $C_1$-$C_6$-alkoxy, hydroxy, halogen, $CF_3$ and $C_1$-$C_6$-alkyl;

$R^4$ denotes hydrogen, a $C_1$-$C_6$-alkyl group, which may be mono- or disubstituted by one or two of the groups -$NH_2$, -NH($C_1$-$C_4$-alkyl), -N($C_1$-$C_4$-alkyl)$_2$, -C(=NH)$NH_2$ or -NH-C(=NH)$NH_2$, or
a 5-, 6- or 7-membered, saturated or unsaturated heterocyclic group linked directly or via a $C_1$-$C_4$-alkylene bridge or via a $C_1$-$C_4$-alkylene-CO bridge which may optionally contain one, two or three hetero atoms selected from among oxygen, nitrogen or sulphur and which may optionally be substituted by $C_1$-$C_4$-alkyl, benzyl or pyridyl;
$C_3$-$C_8$-cycloalkyl, which may be mono- or disubstituted by one or two of the groups -$NH_2$, -NH($C_1$-$C_4$-alkyl), -N($C_1$-$C_4$-alkyl)$_2$, -C(=NH)$NH_2$ or -NH-C(=NH)$NH_2$, or
phenyl, benzyl or phenylethyl, which may be mono- or disubstituted at the phenyl ring by one or two of the groups -$NH_2$, -NH($C_1$-$C_4$-alkyl), -N($C_1$-$C_4$-alkyl)$_2$, -$C_1$-$C_4$-alkyl-$NH_2$, -C(=NH)$NH_2$ or -NH-C(=NH)$NH_2$,
optionally in the form of their tautomers, racemates, enantiomers, diastereomers and mixtures thereof, and optionally the pharmacologically acceptable acid addition salts thereof.

**2.** Compounds of general formula (I) according to Claim 1, wherein

$R^1$ denotes $C_1$-$C_6$-alkyl, which may optionally be mono-, di- or trisubstituted by one or more of the groups hydroxy, $C_1$-$C_4$-alkoxy, $CF_3$, phenoxy, COOH, halogen, -CO($C_1$-$C_4$-alkoxy), -$CONH_2$, -CO-NH($C_1$-$C_4$-alkyl), -CO-N($C_1$-$C_4$-alkyl)$_2$, -$NH_2$, -NH($C_1$-$C_4$-alkyl), -N($C_1$-$C_4$-alkyl)$_2$ or $C_1$-$C_4$-alkoxy-phenoxy, or
a $C_3$-$C_8$-cycloalkyl optionally linked via a $C_1$-$C_4$-alkylene bridge, which may optionally be mono-, di- or trisubstituted by one or more of the groups hydroxy, $C_1$-$C_4$-alkoxy, carboxy, halogen, $C_1$-$C_4$-alkoxycarbonyl or $CF_3$, or phenyl-$C_1$-$C_4$-alkyl, which may optionally be mono-, di- or trisubstituted by one or more of the groups hydroxy, $C_1$-$C_4$-alkoxy, carboxy, halogen, $C_1$-$C_4$-alkoxycarbonyl or $CF_3$, or
a 5- or 6-membered, saturated or unsaturated heterocyclic group linked directly or via a $C_1$-$C_4$-alkylene bridge, which may contain one or two hetero atoms selected from among oxygen, nitrogen or sulphur and which may optionally be substituted by $C_1$-$C_4$-alkyl or benzyl;

$R^2$ denotes $-C(=NH)NH_2$ or $-CH_2-NH_2$;

$R^3$ denotes a group selected from among phenyl, benzyl, naphthyl, quinolyl, isoquinolyl, thienyl and benzothienyl which is mono-, di- or trisubstituted in each case by one or more of the groups selected from among $C_1$-$C_4$-alkyl, $C_1$-$C_4$-alkoxy, halogen, -$C_1$-$C_4$-alkyl-halogen, $-NN_2$, -NH($C_1$-$C_4$-alkyl), -N($C_1$-$C_4$-alkyl)$_2$, $NO_2$, hydroxy, $-CF_3$, -NHCO-$C_1$-$C_4$-alkyl, -COOH, -COO($C_1$-$C_4$-alkyl), $-CONH_2$, -CONH($C_1$-$C_4$-alkyl), -CON($C_1$-$C_4$-alkyl)$_2$, -CONH($C_1$-$C_4$-alkyl)-COO($C_1$-$C_4$-alkyl) and phenyl-$C_1$-$C_4$-alkyl, wherein the substituent phenyl-$C_1$-$C_4$-alkyl may in turn be substituted by a group selected from among $C_1$-$C_4$-alkoxy, hydroxy, halogen, $CF_3$ and $C_1$-$C_4$-alkyl;

$R^4$ denotes hydrogen, a $C_1$-$C_6$-alkyl group, which may be mono- or disubstituted by one or two of the groups $-NH_2$, -NH($C_1$-$C_4$-alkyl), -N($C_1$-$C_4$-alkyl)$_2$, $-C(=NH)NH_2$ or $-NH-C(=NH)NH_2$, or
a 5-, 6- or 7-membered, saturated or unsaturated heterocyclic group linked directly or via a $C_1$-$C_4$-alkylene bridge or via a $C_1$-$C_4$-alkylene-CO bridge, which may optionally contain one, two or three hetero atoms selected from among oxygen, nitrogen or sulphur and may optionally be substituted by $C_1$-$C_4$-alkyl, benzyl or pyridyl;
$C_3$-$C_8$-cycloalkyl, which may be mono- or disubstituted by one or two of the groups $-NH_2$, -NH($C_1$-$C_4$-alkyl), -N($C_1$-$C_4$-alkyl)$_2$, $-C(=NH)NH_2$ or $-NH-C(=NH)NH_2$, or
phenyl, benzyl or phenylethyl, which may be mono- or disubstituted at the phenyl ring by one or two of the groups $-NH_2$, -NH($C_1$-$C_4$-alkyl), -N($C_1$-$C_4$-alkyl)$_2$, -$C_1$-$C_4$-alkyl-$NH_2$, $-C(=NH)NH_2$ or $-NH-C(=NH)NH_2$, optionally in the form of their tautomers, racemates, enantiomers, diastereomers and mixtures thereof, and optionally the pharmacologically acceptable acid addition salts thereof.

**3.** Compounds of general formula (I) according to Claim 1 or 2, wherein

$R^1$ denotes $C_1$-$C_5$-alkyl, which may optionally be mono-, di- or trisubstituted by one or more of the groups hydroxy, $C_1$-$C_4$-alkoxy, $CF_3$, phenoxy, COOH, -CO($C_1$-$C_4$-alkoxy), $-CONH_2$, -CO-NH($C_1$-$C_4$-alkyl), -CO-N($C_1$-$C_4$-alkyl)$_2$ or $C_1$-$C_4$-alkoxy-phenoxy, or
a $C_3$-$C_8$-cycloalkyl optionally linked via a $C_1$-$C_4$-alkylene bridge, which may optionally be mono-, di- or trisubstituted by one or more of the groups hydroxy, $C_1$-$C_4$-alkoxy, carboxy, halogen, $C_1$-$C_4$-alkoxycarbonyl or $CF_3$, or
phenyl-$C_1$-$C_4$-alkyl, which may optionally be mono-, di- or trisubstituted by one or more of the groups hydroxy, $C_1$-$C_4$-alkoxy, carboxy, $C_1$-$C_4$-alkoxycarbonyl or $CF_3$, or
a 5- or 6-membered, saturated or unsaturated heterocyclic group linked directly or via a $C_1$-$C_4$-alkylene bridge, which may contain one or two hetero atoms selected from among oxygen, nitrogen or sulphur and may optionally be substituted by $C_1$-$C_4$-alkyl or benzyl;

$R^2$ denotes $-C(=NH)NH_2$ or $-CH_2-NH_2$;

$R^3$ denotes a group selected from among phenyl, benzyl, benzothienyl, quinolyl, isoquinolyl and naphthyl, which is mono-, di- or trisubstituted in each case by one or more of the groups selected from among $C_1$-$C_4$-alkyl, $C_1$-$C_4$-alkoxy, halogen, $CF_3$, -$C_1$-$C_4$-alkyl-halogen, $-NH_2$, -NH($C_1$-$C_4$-alkyl), -N($C_1$-$C_4$-alkyl)$_2$, $NO_2$, hydroxy, $-CF_3$, -NHCO-$C_1$-$C_4$-alkyl, -COOH, -COO($C_1$-$C_4$-alkyl), $-CONH_2$, -CONH($C_1$-$C_4$-alkyl), -CON($C_1$-$C_4$-alkyl)$_2$, $-C(CH_3)_2$-phenyl (wherein the phenyl group may be substituted by one or more halogen atoms or hydroxy groups) and -CONH($C_1$-$C_4$-alkyl)-COO($C_1$-$C_4$-alkyl);

$R^4$ denotes a $C_1$-$C_6$-alkyl group, which may be mono- or disubstituted by one or two of the groups $-NH_2$, -NH-methyl, -N(methyl)$_2$, -NHethyl, -N(ethyl)$_2$, -NH(n-propyl), -N(n-propyl)$_2$, -NH(iso-propyl), -N(iso-propyl)$_2$, $-C(=NH)NH_2$ or $-NH-C(=NH)NH_2$, or
a 5-, 6- or 7-membered, saturated or unsaturated nitrogen heterocyclic group linked directly or via a $C_1$-$C_4$-alkylene bridge or via a $C_1$-$C_4$-alkylene-CO bridge, which may optionally contain one or two other hetero

atoms selected from among oxygen, nitrogen or sulphur and may optionally be substituted by $C_1$-$C_4$-alkyl, benzyl or pyridyl;
cyclopropyl, cyclopentyl or cyclohexyl, each of which is mono- or disubstituted by one or two of the groups $-NH_2$, -NHmethyl, -N(methyl)$_2$, -NHethyl, -N(ethyl)$_2$, -NH(n-propyl), -N(n-propyl)$_2$, -NH(iso-propyl), -N(iso-propyl)$_2$, -C(=NH)$NH_2$ or -NH-C(=NH)$NH_2$, or
phenyl, benzyl or phenylethyl, which are mono- or disubstituted at the phenyl ring by one or two of the groups $-NH_2$, -NHmethyl, -N(methyl)$_2$, -NHethyl, -N(ethyl)$_2$, -NH(n-propyl), -N(n-propyl)$_2$, -NH(iso-propyl), -N(iso-propyl)$_2$, -C(=NH)$NH_2$ or -NH-C(=NH)$NH_2$,
optionally in the form of their tautomers, racemates, enantiomers, diastereomers and mixtures thereof, and optionally the pharmacologically acceptable acid addition salts thereof.

**4.** Compounds of general formula (I) according to one of claims 1, 2 or 3, wherein

$R^1$ denotes methyl, ethyl, propyl, butyl or pentyl, each of which may be substituted by one of the groups hydroxy, methoxy, ethoxy, propoxy, $CF_3$, phenoxy, COOH, $CONH_2$, CONHMe or methoxy-phenoxy, or
benzyl, phenylethyl or phenylpropyl, which may be mono- or disubstituted at the phenyl ring by one or two of the groups hydroxy, methoxy, ethoxy, carboxy, methoxycarbonyl, ethoxycarbonyl or $CF_3$, or
a 5- or 6-membered, saturated or unsaturated heterocyclic group linked directly or via a $C_1$-$C_4$-alkylene bridge, which may contain one or two hetero atoms selected from among oxygen or nitrogen and may optionally be substituted by methyl, ethyl, propyl or benzyl;

$R^2$ denotes -C(=NH)$NH_2$ or $-CH_2-NH_2$;

$R^3$ denotes phenyl, benzyl or naphthyl, each of which is mono- or disubstituted by one or two groups selected from among methyl, ethyl, propyl, butyl, methoxy, ethoxy, propoxy, butoxy, fluorine, chlorine, bromine, iodine, $-CF_3$, $-NH_2$, -NHmethyl, -NHethyl, -Nmethyl$_2$, -Nethyl$_2$, $NO_2$, -NHCO-methyl, -NHCO-ethyl, -COOH, -COOmethyl, -COOethyl, $-CONH_2$, -CONHmethyl, -CONHethyl, -CONmethyl$_2$, -CONethyl$_2$, -CONH-$CH_2$-COOH, -CONH-$CH_2$-COOmethyl, -CONH-$CH_2$-COOethyl, -CONH-$CH_2CH_2$-COOH, -CONH-$CH_2CH_2$-COOmethyl, -C($CH_3$)$_2$-phenyl (wherein the phenyl group may be substituted by one or more halogen atoms or hydroxy groups) and -CONH-$CH_2CH_2$-COOethyl;

$R^4$ denotes a group selected from among ethyl, propyl, butyl, pentyl and hexyl, each of which is mono- or disubstituted by one or two of the groups $-NH_2$, -NHmethyl, -N(methyl)$_2$, -NHethyl, -N(ethyl)$_2$, -NH(n-propyl), -N(n-propyl)$_2$, -NH(iso-propyl), -N(iso-propyl)$_2$, -C(=NH)$NH_2$ or -NH-C(=NH)$NH_2$, or
a 5-, 6- or 7-membered, saturated or unsaturated nitrogen-heterocyclic group linked directly or via a $C_1$-$C_4$-alkylene bridge, which may optionally contain one or two other hetero atoms selected from among oxygen or nitrogen and may optionally be substituted by methyl, ethyl, propyl or benzyl, or
cyclopentyl or cyclohexyl, each of which may be mono- or disubstituted by one or two of the groups $-NH_2$, -NHmethyl, -N(methyl)$_2$, -NHethyl, -N(ethyl)$_2$, -NH(n-propyl), -N(n-propyl)$_2$, -NH(iso-propyl), -N(iso-propyl)$_2$, -C(=NH)$NH_2$ or -NH-C(=NH)$NH_2$, or
benzyl or phenylethyl, which are mono- or disubstituted at the phenyl ring by one or two of the groups $-NH_2$, -NHmethyl, -N(methyl)$_2$, -NHethyl, -N(ethyl)$_2$, -NH(n-propyl), -N(n-propyl)$_2$, -NH(iso-propyl), -N(iso-propyl)$_2$, -C(=NH)$NH_2$ or -NH-C(=NH)$NH_2$,
optionally in the form of their tautomers, racemates, enantiomers, diastereomers and mixtures thereof, and optionally the pharmacologically acceptable acid addition salts thereof.

**5.** Compounds of general formula (I) according to one of claims 1, 2, 3 or 4, wherein

$R^1$ denotes methyl, ethyl, propyl, butyl or pentyl, preferably methyl;

$R^2$ denotes -C(=NH)$NH_2$ or $-CH_2-NH_2$, preferably -C(=NH)$NH_2$;

$R^3$ denotes phenyl or naphthyl, each of which is substituted by one or two groups selected from among methyl, ethyl, *tert*.-butyl, methoxy, ethoxy, fluorine, chlorine, bromine, $CF_3$, $-NH_2$, -NHmethyl, -NHethyl, -Nmethyl$_2$, -Nethyl$_2$, $NO_2$, -NHCO-methyl, -NHCO-ethyl, -COOH, -COOmethyl, -COOethyl, $-CONH_2$, -CONHmethyl, -CONHethyl, -CONmethyl$_2$, -CONethyl$_2$, -CONH-$CH_2$-COOH, -CONH-$CH_2$-COOmethyl, -C($CH_3$)$_2$-phenyl (wherein the phenyl group may be substituted by one or more halogen atoms or hydroxy groups) and -CONH-$CH_2$-COOethyl;

$R^4$ denotes ethyl or propyl, each of which is substituted by one of the groups $-NH_2$, -N(methyl)$_2$, -N(ethyl)$_2$, -N (n-propyl)$_2$ or -C(=NH)$NH_2$, or
a 5-, 6- or 7-membered, saturated or unsaturated nitrogen-heterocyclic group linked directly or via a methylene or ethylene bridge, which may contain an oxygen as a further heteroatom and may optionally be substituted by methyl, ethyl, propyl or benzyl, or
cyclopentyl or cyclohexyl, each of which is substituted by one of the groups $-NH_2$, -N(methyl)$_2$, -N(ethyl)$_2$, -N(n-propyl)$_2$ or -C(=NH)$NH_2$, or
benzyl, substituted at the phenyl ring by one of the groups $-NH_2$, -N(methyl)$_2$, -N(ethyl)$_2$, -N(n-propyl)$_2$ or -C (=NH)$NH_2$,
optionally in the form of their tautomers, racemates, enantiomers, diastereomers and mixtures thereof, and optionally the pharmacologically acceptable acid addition salts thereof.

**6.** Compounds of general formula (I) according to one of claims 1 to 5, wherein

$R^1$ denotes methyl, ethyl, propyl, butyl or pentyl, preferably methyl;

$R^2$ denotes -C(=NH)$NH_2$ or $-CH_2-NH_2$, preferably -C(=NH)$NH_2$;

$R^3$ denotes phenyl substituted by one or two groups selected from among methyl, ethyl, *tert*-butyl, methoxy, ethoxy, fluorine, chlorine, bromine, $CF_3$, and/or a group selected from among $-NH_2$, -NHmethyl, -NHethyl, -Nmethyl$_2$, -Nethyl$_2$, $NO_2$, -NHCO-methyl, -NHCO-ethyl, -COOH, -COOmethyl, -COOethyl, $-CONH_2$, -CONHmethyl, -CONHethyl, -CONmethyl$_2$, -CONethyl$_2$, $-CONH-CH_2-COOH$, $-CONH-CH_2-$COOmethyl, -C $(CH_3)_2$-phenyl, (wherein the phenyl group may be substituted by one or more halogen atoms or hydroxy groups), and $-CONH-CH_2-$COOethyl,
or naphthyl which is substituted by -Nmethyl$_2$ or chlorine;

$R^4$ denotes dimethylaminoethyl, diethylaminoethyl, dimethylaminopropyl or diethylaminopropyl,
optionally in the form of their tautomers, racemates, enantiomers, diastereomers and mixtures thereof, and optionally the pharmacologically acceptable acid addition salts thereof.

**7.** Compounds of general formula (I) according to one of claims 1 to 6, wherein

$R^1$ denotes methyl, ethyl or propyl, preferably methyl;

$R^2$ denotes -C(=NH)$NH_2$ or $-CH_2-NH_2$, preferably -C(=NH)$NH_2$;

$R^3$ denotes phenyl substituted by one or two groups selected from among methyl, ethyl, *tert*-butyl, methoxy, ethoxy, fluorine, chlorine, bromine, $CF_3$, and/or a group selected from among $-NH_2$, -NHmethyl, -NHethyl, -Nmethyl$_2$, -Nethyl$_2$, $NO_2$, -NHCO-methyl, -NHCO-ethyl, -COOH, -COOmethyl, -COOethyl, $-CONH_2$, -CONHmethyl, -CONHethyl, -CONmethyl$_2$, -CONethyl$_2$, $-CONH-CH_2-COOH$, $-CONH-CH_2-$COOmethyl, -C $(CH_3)_2$-phenyl, (wherein the phenyl group may be substituted by one or more halogen atoms or hydroxy groups), and $-CONH-CH_2-$COOethyl,
or naphthyl which is substituted by -Nmethyl$_2$ or chlorine;

$R^4$ denotes dimethylaminoethyl or diethylaminoethyl,
optionally in the form of their tautomers, racemates, enantiomers, diastereomers and mixtures thereof, and optionally the pharmacologically acceptable acid addition salts thereof.

**8.** Compounds of general formula (I) according to one of claims 1 to 7, wherein

$R^1$ denotes methyl;

$R^2$ denotes -C(=NH)$NH_2$ or $-CH_2-NH_2$, preferably -C(=NH)$NH_2$;

$R^3$ denotes phenyl substituted by one or two groups selected from among methyl, *tert*-butyl, methoxy, chlorine, bromine, $CF_3$, and/or a group selected from among $-NH_2$, $NO_2$, -NHCO-methyl, -COOethyl, $-CONH-CH_2-COOH$, $-C(CH_3)_2$-phenyl (wherein the phenyl group may be substituted by one or more halogen atoms or hydroxy groups), and $-CONH-CH_2-$COOethyl, or naphthyl which is substituted by -Nmethyl$_2$ or

chlorine;

$R^4$ denotes diethylaminoethyl, optionally in the form of their tautomers, racemates, enantiomers, diastereomers and mixtures thereof, and optionally the pharmacologically acceptable acid addition salts thereof.

**9.** Prodrugs of general formula (II)

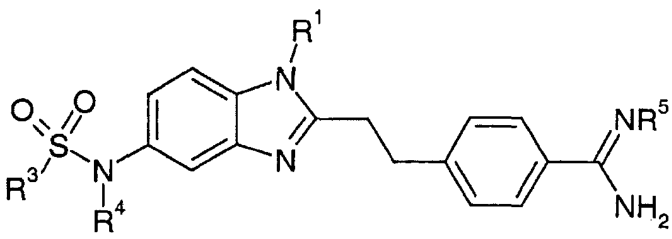
(II),

wherein

$R^1$ and $R^4$ may have the meanings given according to claims 1-8 and

$R^3$ may have the meanings given according to claims 1-8 or denotes $C_1$-$C_4$-alkyl which is substituted by a group selected from among -C(=NOH)$NH_2$, -C(=NCOO-$C_1$-$C_{12}$-alkyl)$NH_2$ or -C(=NCOO-$C_1$-$C_8$-alkyl-phenyl)$NH_2$;

$R^5$ denotes hydroxy, -COO-$C_1$-$C_{12}$-alkyl, -CO-phenyl, -CO-pyridyl or-COO-$C_1$-$C_8$-alkyl-phenyl, whilst in the abovementioned group the phenyl ring may be substituted in each case by $C_1$-$C_4$-alkyl, $C_1$-$C_4$-alkoxy, OH, halogen or $CF_3$, optionally in the form of their tautomers, racemates, enantiomers, diastereomers and mixtures thereof, and optionally the pharmacologically acceptable acid addition salts thereof.

**10.** Prodrugs of general formula (II) according to Claim 9, wherein

$R^3$ may have the meanings given in claims 1-8 or denotes $C_1$-$C_4$-alkyl, which is substituted by a group selected from among -C(=NOH)$NH_2$, -C(=NCOO-$C_1$-$C_6$-alkyl)$NH_2$ or -C(=NCOO-$C_1$-$C_6$-alkyl-phenyl)$NH_2$;

$R^5$ denotes hydroxy, -COO-$C_1$-$C_6$-alkyl, -CO-phenyl, -CO-pyridyl or -COO-$C_1$-$C_6$-alkyl-phenyl, whilst in the abovementioned group the phenyl ring may be substituted in each case by $C_1$-$C_4$-alkyl, $C_1$-$C_4$-alkoxy, OH, halogen or $CF_3$, optionally in the form of their tautomers, racemates, enantiomers, diastereomers and mixtures thereof, and optionally the pharmacologically acceptable acid addition salts thereof.

**11.** Prodrugs of general formula (II) according to Claim 9 or 10, wherein

$R^5$ may denote hydroxy, methoxycarbonyl, ethoxycarbonyl, propyloxycarbonyl, butyloxycarbonyl, benzoyl, benzyloxycarbonyl or nicotinoyl, optionally in the form of their tautomers, racemates, enantiomers, diastereomers and mixtures thereof, and optionally the pharmacologically acceptable acid addition salts thereof.

**12.** Compounds of general formula (III)

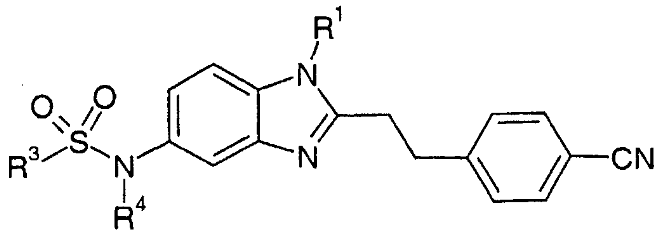

(III)

wherein the groups $R^1$, $R^3$ and $R^4$ may have the meanings given in claims 1-8.

**13.** Use of compounds of general formula (I) according to one of claims 1-8 for preparing a pharmaceutical composition for the prevention and/or treatment of diseases in which tryptase inhibitors may have a therapeutic benefit.

**14.** Use of prodrugs of general formula (II) according to one of claims 9 to 11 for preparing a pharmaceutical composition for the prevention and/or treatment of diseases in which tryptase inhibitors may have a therapeutic benefit.

**15.** Pharmaceutical composition, **characterised in that** it contains one or more compounds according to one of claims 1-11.

**16.** Process for preparing compounds of general formula (I)

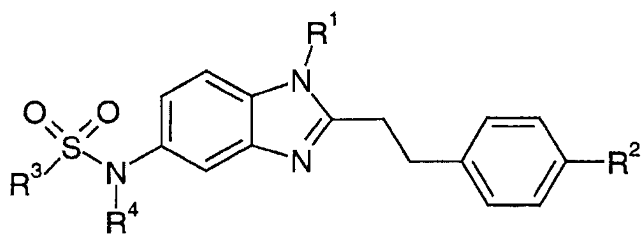

(I),

wherein the groups $R^1$, $R^2$, $R^3$ and $R^4$ may have the meanings given in claims 1 to 8, **characterised in that** a compound of general formula (III)

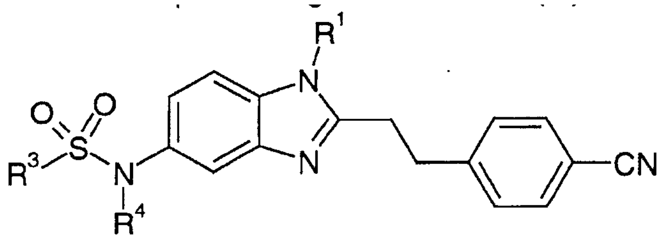

(III)

wherein the groups $R^1$, $R^3$ and $R^4$ may have the meanings given in claims 1 to 8, is converted directly into the compounds of general formula (I).

**17.** Process for preparing compounds of general formula (I)

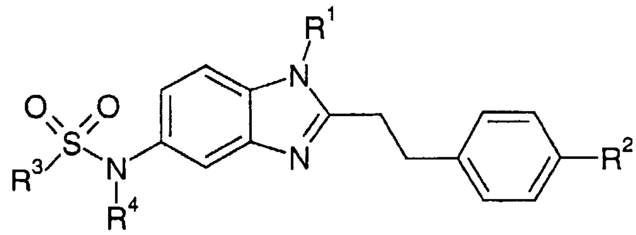

(I),

wherein the groups $R^1$, $R^2$, $R^3$ and $R^4$ may have the meanings given in claims 1 to 8, **characterised in that** a compound of general formula (II)

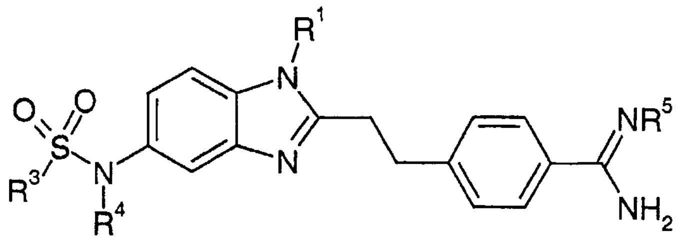

(II),

wherein the groups $R^1$, $R^3$ and $R^4$ may have the meanings given in claims 1 to 8 and the group $R^5$ may have the meanings given in claims 9 to 11, is converted into a compound of general formula (I).

**18.** Process for preparing compounds of general formula (II)

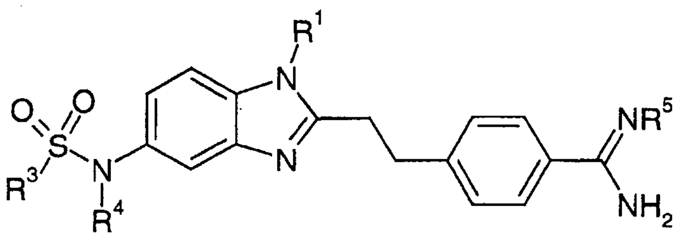

(II),

wherein the groups $R^1$, $R^3$ and $R^4$ may have the meanings given in claims 1-8 and the group $R^5$ may have the meanings given in claims 9-11, **characterised in that** a compound of general formula (III)

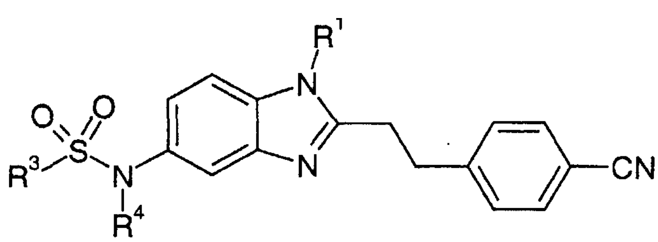

(III)

wherein the groups $R^1$, $R^3$ and $R^4$ may have the meanings given in claims 1-8, is converted into a compound of general formula (II).

**Revendications**

**1.** Dérivés de benzimidazole de formule générale (I)

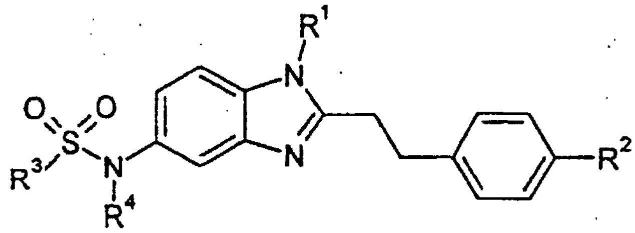

(I),

où

$R^1$ peut représenter un reste choisi dans le groupe alkyle en $C_1$-$C_6$, alcényle en $C_2$-$C_6$ et alcynyle en $C_2$-$C_6$, qui peut éventuellement être substitué une, deux ou trois fois par un ou plusieurs des restes hydroxyle, alcoxy en $C_1$-$C_4$, $CF_3$, phénoxy, COOH, halogène, -CO(alcoxy en $C_1$-$C_4$), -$CONH_2$, -CO-NH(alkyle en $C_1$-$C_4$), -CO-N (alkyle en $C_1$-$C_4$)$_2$, -$NH_2$, -NH(alkyle en $C_1$-$C_4$), -N(alkyle en $C_1$-$C_4$)$_2$ ou alcoxy en $C_1$-$C_4$-phénoxy, ou
un cycloalkyle en $C_3$-$C_8$ éventuellement lié par le biais d'un pont alkylène en $C_1$-$C_4$, qui peut éventuellement être substitué une, deux ou trois fois par un ou plusieurs des restes hydroxyle, alcoxy en $C_1$-$C_4$, carboxyle, halogène, alcoxy en $C_1$-$C_4$-carbonyle ou $CF_3$, ou
phényl-alkyle en $C_1$-$C_4$ qui peut éventuellement être substitué une, deux ou trois fois par un ou plusieurs des restes hydroxyle, alcoxy en $C_1$-$C_4$, carboxyle, halogène, alcoxy en $C_1$-$C_4$-carbonyle ou $CF_3$, ou
un hétérocycle saturé ou insaturé, à 5 ou 6 chaînons, lié directement ou par le biais d'un pont alkylène en $C_1$-$C_4$, qui peut contenir un ou deux hétéroatomes choisis dans le groupe oxygène, azote et soufre et qui peut éventuellement être substitué par alkyle en $C_1$-$C_4$ ou benzyle ;
$R^2$ peut représenter -C(=NH)$NH_2$ ou -$CH_2$-$NH_2$ ;
$R^3$ peut représenter un reste choisi dans le groupe phényle, benzyle, naphtyle, quinolyle, isoquinolyle, furane, benzofurane, thiophène et benzothiophène qui est substitué dans chaque cas une, deux ou trois fois par un ou plusieurs des restes choisis dans le groupe alkyle en $C_1$-$C_4$, alcoxy en $C_1$-$C_4$, halogène, alkyle en $C_1$-$C_4$-halogène, -$NH_2$, -NH(alkyle en $C_1$-$C_4$), -N(alkyle en $C_1$-$C_4$)$_2$, $NO_2$, hydroxyle, -$CF_3$, -NHCO-alkyle en $C_1$-$C_4$, -COOH, -COO(alkyle en $C_1$-$C_4$), -$CONH_2$, -CONH-(alkyle en $C_1$-$C_4$), -CON(alkyle en $C_1$-$C_4$)$_2$, -CONH-(alkyle en $C_1$-$C_4$)-COO(alkyle en $C_1$-$C_4$) et phényl-alkyle en $C_1$-$C_6$, où le substituant phényl-alkyle en $C_1$-$C_6$ peut être substitué pour sa part par un reste choisi dans le groupe alcoxy en $C_1$-$C_6$, hydroxyle, halogène, $CF_3$ et alkyle en $C_1$-$C_6$ ;
$R^4$ peut représenter l'hydrogène, un reste alkyle en $C_1$-$C_6$ qui peut être substitué une ou deux fois par un ou deux des restes -$NH_2$, -NH(alkyle en $C_1$-$C_4$), -N(alkyle en $C_1$-$C_4$)$_2$, -C(=NH)$NH_2$ ou -NH-C(=NH)$NH_2$, ou
un hétérocycle saturé ou insaturé, à 5, 6 ou 7 chaînons, lié directement ou par le biais d'un pont alkylène en $C_1$-$C_4$ ou par le biais d'un pont alkylène en $C_1$-$C_4$-CO, qui peut contenir éventuellement un, deux ou trois hétéroatomes choisis dans le groupe oxygène, azote et soufre et qui peut éventuellement être substitué par alkyle en $C_1$-$C_4$, benzyle ou pyridyle ;
cycloalkyle en $C_3$-$C_8$ qui peut être substitué une ou deux fois par un ou deux des restes -$NH_2$, -NH(alkyle en $C_1$-$C_4$), -N(alkyle en $C_1$-$C_4$)$_2$, -C(=NH)$NH_2$ ou -NH-C(=NH)$NH_2$, ou
phényle, benzyle ou phényléthyle, qui peuvent être substitués dans chaque cas une ou deux fois sur le cycle phényle par un ou deux des restes -$NH_2$, -NH(alkyle en $C_1$-$C_4$), -N(alkyle en $C_1$-$C_4$)$_2$, alkyle en $C_1$-$C_4$-$NH_2$, -C(=NH)$NH_2$ ou -NH-C(=NH)$NH_2$,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**2.** Composés de formule générale (I) selon la revendication 1 où

$R^1$ peut représenter alkyle en $C_1$-$C_6$, qui peut éventuellement être substitué une, deux ou trois fois par un ou plusieurs des restes hydroxyle, alcoxy en $C_1$-$C_4$, $CF_3$, phénoxy, COOH, halogène, -CO(alcoxy en $C_1$-$C_4$), -$CONH_2$, -CO-NH(alkyle en $C_1$-$C_4$), -CO-N(alkyle en $C_1$-$C_4$)$_2$, -$NH_2$, -NH(alkyle en $C_1$-$C_4$), -N(alkyle en $C_1$-$C_4$)$_2$ ou alcoxy en $C_1$-$C_4$-phénoxy, ou
un cycloalkyle en $C_3$-$C_8$ éventuellement lié par le biais d'un pont alkylène en $C_1$-$C_4$, qui peut éventuellement être substitué une, deux ou trois fois par un ou plusieurs des restes hydroxyle, alcoxy en $C_1$-$C_4$, carboxyle, halogène, alcoxy en $C_1$-$C_4$-carbonyle ou $CF_3$, ou

phényl-alkyle en $C_1$-$C_4$ qui peut éventuellement être substitué une, deux ou trois fois par un ou plusieurs des restes hydroxyle, alcoxy en $C_1$-$C_4$, carboxyle, halogène, alcoxy en $C_1$-$C_4$-carbonyle ou $CF_3$, ou
un hétérocycle saturé ou insaturé, à 5 ou 6 chaînons, lié directement ou par le biais d'un pont alkylène en $C_1$-$C_4$, qui peut contenir un ou deux hétéroatomes choisis dans le groupe oxygène, azote et soufre et qui peut éventuellement être substitué par alkyle en $C_1$-$C_4$ ou benzyle ;
$R^2$ peut représenter -C(=NH)$NH_2$ ou -$CH_2$-$NH_2$ ;
$R^3$ peut représenter un reste choisi dans le groupe phényle, benzyle, naphtyle, quinolyle, isoquinolyle, thiényle et benzothiényle qui est substitué dans chaque cas une, deux ou trois fois par un ou plusieurs des restes choisis dans le groupe alkyle en $C_1$-$C_4$, alcoxy en $C_1$-$C_4$, halogène, alkyle en $C_1$-$C_4$-halogène, -$NH_2$, -NH (alkyle en $C_1$-$C_4$), -N(alkyle en $C_1$-$C_4$)$_2$, $NO_2$, hydroxyle, -$CF_3$, -NHCO-alkyle en $C_1$-$C_4$, -COOH, -COO(alkyle en $C_1$-$C_4$), -$CONH_2$, -CONH(alkyle en $C_1$-$C_4$), -CON(alkyle en $C_1$-$C_4$)$_2$, -CONH(alkyle en $C_1$-$C_4$)-COO(alkyle en $C_1$-$C_4$) et phényl-alkyle en $C_1$-$C_4$, où le substituant phényl-alkyle en $C_1$-$C_4$ peut être substitué pour sa part par un reste choisi dans le groupe alcoxy en $C_1$-$C_4$, hydroxyle, halogène, $CF_3$ et alkyle en $C_1$-$C_4$ ;
$R^4$ peut représenter l'hydrogène, un reste alkyle en $C_1$-$C_6$ qui peut être substitué une ou deux fois par un ou deux des restes -$NH_2$, -NH(alkyle en $C_1$-$C_4$), -N(alkyle en $C_1$-$C_4$)$_2$, -C(=NH)$NH_2$ ou -NH-C(=NH)$NH_2$, ou
un hétérocycle saturé ou insaturé, à 5, 6 ou 7 chaînons, lié directement ou par le biais d'un pont alkylène en $C_1$-$C_4$, ou par le biais d'un pont alkylène en $C_1$-$C_4$-CO, qui peut contenir éventuellement un, deux ou trois hétéroatomes choisis dans le groupe oxygène, azote et soufre et qui peut éventuellement être substitué par alkyle en $C_1$-$C_4$, benzyle ou pyridyle ;
cycloalkyle en $C_3$-$C_8$ qui peut être substitué une ou deux fois par un ou deux des restes $NH_2$, -NH(alkyle en $C_1$-$C_4$), -N(alkyle en $C_1$-$C_4$)$_2$, -C(=NH)$NH_2$ ou -NH-C(=NH)$NH_2$, ou
phényle, benzyle ou phényléthyle, qui peuvent être substitués dans chaque cas une ou deux fois sur le cycle phényle par un ou deux des restes -$NH_2$, -NH(alkyle en $C_1$-$C_4$), -N(alkyle en $C_1$-$C_4$)$_2$, alkyle en $C_1$-$C_4$-$NH_2$, -C(=NH)$NH_2$ ou -NH-C(=NH)$NH_2$,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**3.** Composés de formule générale (I) selon la revendication 1 ou 2 où

$R^1$ peut représenter alkyle en $C_1$-$C_5$, qui peut éventuellement être substitué une, deux ou trois fois par un ou plusieurs des restes hydroxyle, alcoxy en $C_1$-$C_4$, $CF_3$, phénoxy, COOH, -CO(alcoxy en $C_1$-$C_4$), -$CONH_2$, -CO-NH(alkyle en $C_1$-$C_4$), -CO-N(alkyle en $C_1$-$C_4$)$_2$ ou alcoxy en $C_1$-$C_4$-phénoxy, ou
un cycloalkyle en $C_3$-$C_8$ éventuellement lié par le biais d'un pont alkylène en $C_1$-$C_4$, qui peut éventuellement être substitué une, deux ou trois fois par un ou plusieurs des restes hydroxyle, alcoxy en $C_1$-$C_4$, carboxyle, halogène, alcoxy en $C_1$-$C_4$-carbonyle ou $CF_3$, ou
phényl-alkyle en $C_1$-$C_4$ qui peut éventuellement être substitué une, deux ou trois fois par un ou plusieurs des restes hydroxyle, alcoxy en $C_1$-$C_4$, carboxyle, alcoxy en $C_1$-$C_4$-carbonyle ou $CF_3$, ou
un hétérocycle saturé ou insaturé, à 5 ou 6 chaînons, lié directement ou par le biais d'un pont alkylène en $C_1$-$C_4$, qui peut contenir un ou deux hétéroatomes choisis dans le groupe oxygène, azote et soufre et qui peut éventuellement être substitué par alkyle en $C_1$-$C_4$ ou benzyle ;
$R^2$ peut représenter -C(=NH)$NH_2$ ou -$CH_2$-$NH_2$ ;
$R^3$ peut représenter un reste choisi dans le groupe phényle, benzyle, benzothiényle, quinolyle, isoquinolyle et naphtyle qui est substitué dans chaque cas une, deux ou trois fois par un ou plusieurs des restes choisis dans le groupe alkyle en $C_1$-$C_4$, alcoxy en $C_1$-$C_4$, halogène, -$CF_3$, alkyle en $C_1$-$C_4$-halogène, -$NH_2$, -NH(alkyle en $C_1$-$C_4$), -N(alkyle en $C_1$-$C_4$)$_2$, $NO_2$, hydroxyle, -$CF_3$, -NHCO-alkyle en $C_1$-$C_4$, -COOH, -COO(alkyle en $C_1$-$C_4$), -$CONH_2$, -CONH(alkyle en $C_1$-$C_4$), -CON(alkyle en $C_1$-$C_4$)$_2$, -C$(CH_3)_2$-phényle, où ce groupe phényle peut être substitué par un ou plusieurs atomes d'halogène ou groupes hydroxyle, et -CONH(alkyle en $C_1$-$C_4$) -COO(alkyle en $C_1$-$C_4$) ;
$R^4$ peut représenter un reste alkyle en $C_1$-$C_6$ qui est substitué une ou deux fois par un ou deux des restes -$NH_2$, -NHméthyle, -N(méthyle)$_2$, -NHéthyle, -N(éthyle)$_2$, -NH(n-propyle), -N(n-propyle)$_2$, -NH(isopropyle), -N (isopropyle)$_2$, -C(=NH)$NH_2$ ou -NH-C(=NH)$NH_2$, ou
un hétérocycle azoté saturé ou insaturé, à 5, 6 ou 7 chaînons, lié directement ou par le biais d'un pont alkylène en $C_1$-$C_4$ ou par le biais d'un pont alkylène en $C_1$-$C_4$-CO, qui peut contenir éventuellement un ou deux autres hétéroatomes choisis dans le groupe oxygène, azote et soufre et qui peut éventuellement être substitué par alkyle en $C_1$-$C_4$, benzyle ou pyridyle;
cyclopropyle, cyclopentyle ou cyclohexyle qui sont substitués dans chaque cas une ou deux fois par un ou deux des restes -$NH_2$, -NHméthyle, -N(méthyle)$_2$, -NHéthyle, -N(éthyle)$_2$, -NH(n-propyle), -N(n-propyle)$_2$, -NH

(isopropyle), -N(isopropyle)$_2$, -C(=NH)NH$_2$ ou -NH-C(=NH)NH$_2$, ou
phényle, benzyle ou phényléthyle, qui sont substitués dans chaque cas une ou deux fois sur le cycle phényle par un ou deux des restes NH$_2$, -NHméthyle, -N(méthyle)$_2$, -NHéthyle, -N(éthyle)$_2$, -NH(n-propyle), -N(n-propyle)$_2$, -NH(isopropyle), -N(isopropyle)$_2$, -C(=NH)NH$_2$ ou -NH-C(=NH)NH$_2$,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**4.** Composés de formule générale (I) selon l'une des revendications 1, 2 et 3 où

$R^1$ peut représenter méthyle, éthyle, propyle, butyle ou pentyle, qui peuvent être substitués dans chaque cas par l'un des restes hydroxyle, méthoxy, éthoxy, propoxy, $CF_3$, phénoxy, COOH, -CONH$_2$, -CONHMe ou méthoxy-phénoxy, ou
benzyle, phényléthyle ou phénylpropyle, qui peuvent être substitués dans chaque cas une ou deux fois sur le cycle phényle par un ou deux des restes hydroxyle, méthoxy, éthoxy, carboxyle, méthoxycarbonyle, éthoxycarbonyle ou $CF_3$,
ou
un hétérocycle saturé ou insaturé, à 5 ou 6 chaînons, lié directement ou par le biais d'un pont alkylène en $C_1$-$C_4$, qui peut contenir un ou deux hétéroatomes choisis dans le groupe oxygène et azote et qui peut éventuellement être substitué par méthyle, éthyle, propyle ou benzyle ;
$R^2$ peut représenter -C(=NH)NH$_2$ ou -CH$_2$-NH$_2$ ;
$R^3$ peut représenter phényle, benzyle ou naphtyle qui sont substitués dans chaque cas une ou deux fois par un ou deux restes choisis dans le groupe méthyle, éthyle, propyle, butyle, méthoxy, éthoxy, propoxy, butoxy, fluor, chlore, brome, iode, -CF$_3$, -NH$_2$, -NHméthyle, -NHéthyle, -Nméthyle$_2$, -Néthyle$_2$, -NO$_2$, -NHCO-méthyle, -NHCO-éthyle, -COOH, -COOméthyle, -COOéthyle, -CONH$_2$, -CONHméthyle, -CONHéthyle, -CONméthyle$_2$, -CONéthyle$_2$, -CONH-CH$_2$-COOH, -CONH-CH$_2$-COOméthyle, -CONH-CH$_2$-COOéthyle, -CONH-CH$_2$CH$_2$-COOH, -CONH-CH$_2$CH$_2$-COOméthyle, -C(CH$_3$)$_2$-phényle, où ce groupe phényle peut être substitué par un ou plusieurs atomes d'halogène ou groupes hydroxyle, et -CONH-CH$_2$CH$_2$-COOéthyle ;
$R^4$ peut représenter un reste choisi dans le groupe éthyle, propyle, butyle, pentyle et hexyle, qui sont substitués dans chaque cas une ou deux fois par un ou deux des restes -NH$_2$, -NHméthyle, -N(méthyle)$_2$, -NHéthyle, -N(éthyle)$_2$, -NH(n-propyle), -N(n-propyle)$_2$, -NH(isopropyle), -N(isopropyle)$_2$, -C(=NH)NH$_2$ ou -NH-C(=NH)NH$_2$, ou
un hétérocycle azoté saturé ou insaturé, à 5, 6 ou 7 chaînons, lié directement ou par le biais d'un pont alkylène en $C_1$-$C_4$, qui peut contenir éventuellement un ou deux autres hétéroatomes choisis dans le groupe oxygène et azote et qui peut éventuellement être substitué par méthyle, éthyle, propyle ou benzyle, ou cyclopentyle ou cyclohexyle qui sont substitués dans chaque cas une ou deux fois par un ou deux des restes NH$_2$, -NHméthyle, -N(méthyle)$_2$, -NHéthyle, -N(éthyle)$_2$, -NH(n-propyle), -N(n-propyle)$_2$, -NH(isopropyle), -N(isopropyle)$_2$, -C(=NH)NH$_2$ ou -NH-C(=NH)NH$_2$, ou benzyle ou phényléthyle, qui sont substitués dans chaque cas une ou deux fois sur le cycle phényle par un ou deux des restes NH$_2$, -NHméthyle, -N(méthyle)$_2$, -NHéthyle, -N(éthyle)$_2$, -NH(n-propyle), -N(n-propyle)$_2$, -NH(isopropyle), -N(isopropyle)$_2$, -C(=NH)NH$_2$ ou -NH-C(=NH)NH$_2$,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**5.** Composés de formule générale (I) selon l'une des revendications 1, 2, 3 et 4 où

$R^1$ peut représenter méthyle, éthyle, propyle, butyle ou pentyle, de préférence méthyle ;
$R^2$ peut représenter -C(=NH)NH$_2$ ou -CH$_2$-NH$_2$, de préférence -C(=NH)NH$_2$ ;
$R^3$ peut représenter phényle ou naphtyle qui sont substitués dans chaque cas par un ou deux restes choisis dans le groupe méthyle, éthyle, tert.butyle, méthoxy, éthoxy, fluor, chlore, brome, -CF$_3$, -NH$_2$, -NHméthyle, -NHéthyle, -Nméthyle$_2$, -Néthyle$_2$, -NO$_2$, -NHCO-méthyle, -NHCO-éthyle, -COOH, -COOméthyle, -COOéthyle, -CONH$_2$, -CONHméthyle, -CONHéthyle, -CONméthyle$_2$, -CONéthyle$_2$, -CONH-CH$_2$-COOH, -CONH-CH$_2$-COOméthyle, -C(CH$_3$)$_2$-phényle, où ce groupe phényle peut être substitué par un ou plusieurs atomes d'halogène ou groupes hydroxyle, et -CONH-CH$_2$-COOéthyle ;
$R^4$ peut représenter éthyle ou propyle, qui sont substitués dans chaque cas par l'un des restes -NH$_2$, -N(méthyle)$_2$, -N(éthyle)$_2$, -N(n-propyle)$_2$, ou -C(=NH)NH$_2$, ou
un hétérocycle azoté saturé ou insaturé, à 5, 6 ou 7 chaînons, lié directement ou par le biais d'un pont méthylène ou éthylène, qui peut contenir un oxygène comme autre hétéroatome et qui peut éventuellement être

substitué par méthyle, éthyle, propyle ou benzyle, ou cyclopentyle ou cyclohexyle qui sont substitués dans chaque cas par l'un des restes $-NH_2$, -N(méthyle)$_2$, -N(éthyle)$_2$, -N(n-propyle)$_2$ et $-C(=NH)NH_2$, ou benzyle, qui est substitué sur le cycle phényle par l'un des restes $-NH_2$, -N(méthyle)$_2$, -N(éthyle)$_2$, -N(n-propyle)$_2$ ou $-C(=NH)NH_2$,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**6.** Composés de formule générale (I) selon l'une des revendications 1 à 5 où

$R^1$ peut représenter méthyle, éthyle, propyle, butyle ou pentyle, de préférence méthyle ;
$R^2$ peut représenter $-C(=NH)NH_2$ ou $-CH_2-NH_2$, de préférence $-C(=NH)NH_2$ ;
$R^3$ peut représenter phényle qui est substitué par un ou deux restes choisis dans le groupe méthyle, éthyle, tert.butyle, méthoxy, éthoxy, fluor, chlore, brome, $-CF_3$, et/ou un reste choisi parmi $-NH_2$, -NHméthyle, -NHéthyle, -Nméthyle$_2$, -Néthyle$_2$, $-NO_2$, -NHCO-méthyle, -NHCO-éthyle, -COOH, -COOméthyle, -COOéthyle, $-CONH_2$,-CONHméthyle,-CONHéthyle,-CONméthyle$_2$,-CONéthyle$_2$,$-CONH-CH_2-COOH$,$-CONH-CH_2-$COOméthyle, -C $(CH_3)_2$-phényle, où ce groupe phényle peut être substitué par un ou plusieurs atomes d'halogène ou groupes hydroxyle, et $-CONH-CH_2-$COOéthyle, ou naphtyle qui est substitué par -Nméthyle$_2$ ou par le chlore ;
$R^4$ peut représenter diméthylaminoéthyle, diéthylaminoéthyle, diméthylaminopropyle ou diéthylaminopropyle,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**7.** Composés de formule générale (I) selon l'une des revendications 1 à 6 où

$R^1$ peut représenter méthyle, éthyle ou propyle, de préférence méthyle ;
$R^2$ peut représenter $-C(=NH)NH_2$ ou $-CH_2-NH_2$, de préférence $-C(=NH)NH_2$ ;
$R^3$ peut représenter phényle qui est substitué par un ou deux restes choisis dans le groupe méthyle, éthyle, tert.butyle, méthoxy, éthoxy, fluor, chlore, brome, $-CF_3$, et/ou un reste du groupe $-NH_2$, -NHméthyle, -NHéthyle, -Nméthyle$_2$, -Néthyle$_2$, $-NO_2$, -NHCO-méthyle, -NHCO-éthyle, -COOH, -COOméthyle, -COOéthyle, $-CONH_2$, -CONHméthyle, -CONHéthyle, -CONméthyle$_2$, -CONéthyle$_2$, $-CONH-CH_2-COOH$, $-CONH-CH_2-$COOméthyle, $-C(CH_3)_2$-phényle, où ce groupe phényle peut être substitué par un ou plusieurs atomes d'halogène ou groupes hydroxyle, et $-CONH-CH_2-$COOéthyle, ou
naphtyle qui est substitué par -Nméthyle$_2$ ou par le chlore ;
$R^4$ peut représenter diméthylaminoéthyle ou diéthylaminoéthyle,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**8.** Composés de formule générale (I) selon l'une des revendications 1 à 7 où

$R^1$ peut représenter méthyle ;
$R^2$ peut représenter $-C(=NH)NH_2$ ou $-CH_2-NH_2$, de préférence $-C(=NH)NH_2$ ;
$R^3$ peut représenter phényle qui est substitué par un ou deux restes choisis dans le groupe méthyle, tert. butyle, méthoxy, chlore, brome, $-CF_3$, et/ou un reste du groupe $-NH_2$, $-NO_2$, -NHCO-méthyle, -COOéthyle, $-CONH-CH_2-COOH$, $-C(CH_3)_2$-phényle, où ce groupe phényle peut être substitué par un ou plusieurs atomes d'halogène ou groupes hydroxyle, et $-CONH-CH_2-$COOéthyle, ou
naphtyle qui est substitué par -Nméthyle$_2$ ou par le chlore ;
$R^4$ peut représenter diéthylaminoéthyle,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**9.** Promédicaments de formule générale (II)

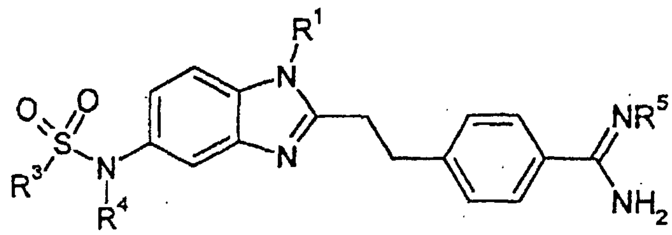

(II),

où

$R^1$ et $R^4$ peuvent présenter les significations citées selon les revendications 1-8 et
$R^3$ peut présenter les significations citées dans les revendications 1-8 ou représente alkyle en $C_1$-$C_4$ qui est substitué par un reste choisi dans le groupe -C(=NOH)$NH_2$, -C(=NCOO-alkyle en $C_1$-$C_{12}$)$NH_2$ et -C(=NCOO-alkyle en $C_1$-$C_8$-phényl)$NH_2$ ;
$R^5$ peut représenter hydroxyle, -COO-alkyle en $C_1$-$C_{12}$, -CO-phényle, -CO-pyridyle ou -COO-alkyle en $C_1$-$C_8$-phényle, où, dans le groupe cité précédemment, le cycle phényle peut être substitué dans chaque cas par alkyle en $C_1$-$C_4$, alcoxy en $C_1$-$C_4$, OH, halogène ou $CF_3$,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**10.** Promédicaments de formule générale (II) selon la revendication 9 où

$R^3$ peut présenter les significations citées dans les revendications 1-8 ou représente alkyle en $C_1$-$C_4$ qui est substitué par un reste choisi dans le groupe -C(=NOH)$NH_2$, -C(=NCOO-alkyle en $C_1$-$C_6$)$NH_2$ ou -C(=NCOO-alkyle en $C_1$-$C_6$-phényl)$NH_2$ ;
$R^5$ peut représenter hydroxyle, -COO-alkyle en $C_1$-$C_6$, -CO-phényle, -CO-pyridyle ou -COO-alkyle en $C_1$-$C_6$-phényle, où, dans le groupe cité précédemment, le cycle phényle peut être substitué dans chaque cas par alkyle en $C_1$-$C_4$, alcoxy en $C_1$-$C_4$, OH, halogène ou $CF_3$,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**11.** Promédicaments de formule générale (II) selon la revendication 9 ou 10 où

$R^5$ peut représenter hydroxyle, méthoxycarbonyle, éthoxycarbonyle, propyloxycarbonyle, butyloxycarbonyle, benzoyle, benzyloxycarbonyle ou nicotinoyle,
éventuellement sous forme de leurs tautomères, de leurs racémates, de leurs énantiomères, de leurs diastéréo-isomères et de leurs mélanges, et éventuellement de leurs sels d'addition d'acide pharmacologiquement acceptables.

**12.** Composés de formule générale (III)

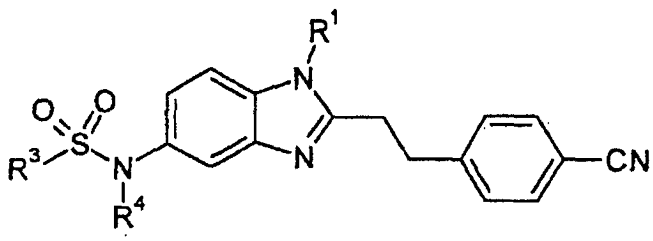

(III)

où les restes $R^1$, $R^3$ et $R^4$ peuvent avoir les significations citées dans les revendications 1-8.

**13.** Utilisation de composés de formule générale (I) selon l'une des revendications 1-8 pour la production d'un médicament pour la prévention et/ou le traitement de maladies dans lesquelles les inhibiteurs de tryptase peuvent présenter une utilité thérapeutique.

**14.** Utilisation de promédicaments de formule générale (II) selon l'une des revendications 9 à 11 pour la production d'un médicament pour la prévention et/ou le traitement de maladies dans lesquelles les inhibiteurs de tryptase peuvent présenter une utilité thérapeutique.

**15.** Composition pharmaceutique **caractérisée par** une teneur en un ou plusieurs composés selon l'une des revendications 1-11.

**16.** Procédé de préparation de composés de formule générale (I)

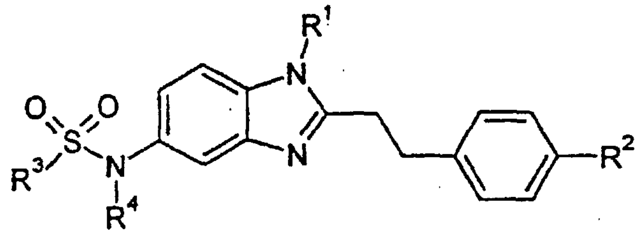

(I),

où les restes $R^1$, $R^2$, $R^3$ et $R^4$ peuvent avoir les significations citées dans les revendications 1-8, **caractérisé en ce qu'**un composé de formule générale (III)

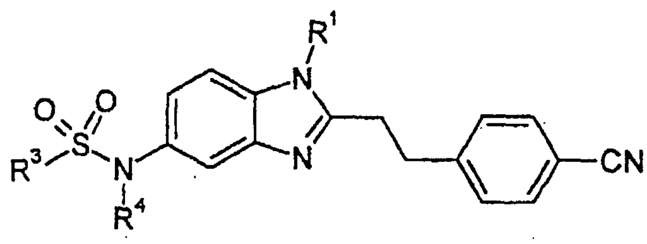

(III)

où les restes $R^1$, $R^3$ et $R^4$ peuvent avoir les significations citées dans les revendications 1-8 est converti directement en les composés de formule générale (I).

**17.** Procédé de préparation de composés de formule générale (I)

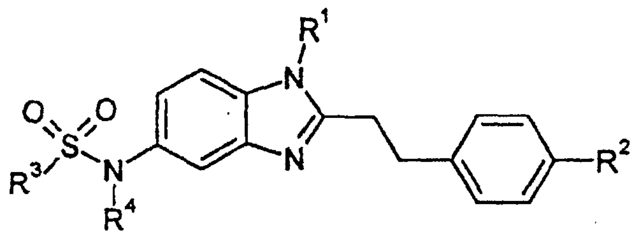

(I),

où les restes $R^1$, $R^2$, $R^3$ et $R^4$ peuvent avoir les significations citées dans les revendications 1-8, **caractérisé en ce qu'**un composé de formule générale (II)

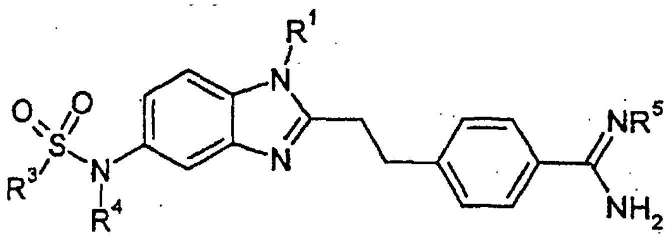

(II),

où les restes $R^1$, $R^3$ et $R^4$ peuvent présenter les significations citées dans les revendications 1-8 et le reste $R^5$ peut présenter les significations citées dans les revendications 9-11 est converti en les composés de formule générale (I).

**18.** Procédé de préparation de composés de formule générale (II)

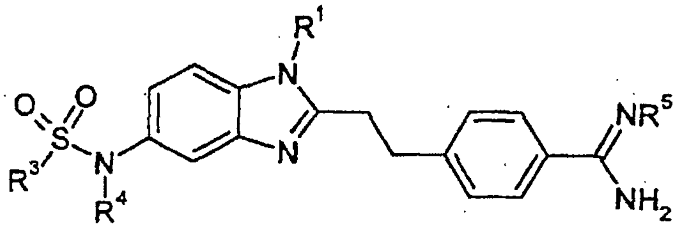

(II),

où les restes $R^1$, $R^3$ et $R^4$ peuvent présenter les significations citées dans les revendications 1-8 et le reste $R^5$ peut présenter les significations citées dans les revendications 9-11, **caractérisé en ce qu'**un composé de formule générale (III)

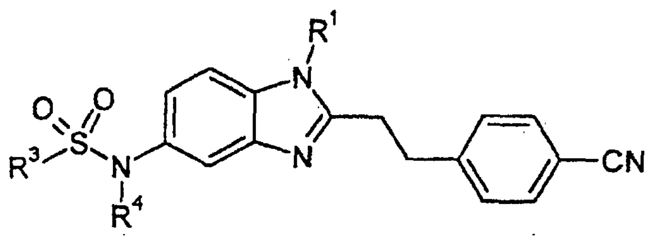

(III)

où les restes $R^1$, $R^3$ et $R^4$ peuvent avoir les significations citées dans les revendications 1-8 est converti en un composé de formule générale (II).